# EUROPEAN PATENT SPECIFICATION

(11) **EP 1 610 779 B1**
(45) Date of publication and mention of the grant of the patent: **09.09.2009**
(21) Application number: 04723448.9
(22) Date of filing: 25.03.2004
(51) Int. Cl.: A61K 31/416, C07D 231/56, C07C 275/26, A61P 3/06

(54) **3-OXO-1,3-DIHYDRO-INDAZOLE-2-CARBOXYLIC ACID AMIDE DERIVATIVES AS PHOSPHOLIPASE INHIBITORS**
3-OXO-1,3-DIHYDRO-INDAZOL-2-CARBONSÄUREAMID DERIVATE ALS PHOSPHOLIPASE INHIBITOREN
UTILISATION DE DERIVES AMIDES D'ACIDE 3-OXO-1,3-DIHYDRO-INDAZOLE-2-CARBOXYLIQUE COMME INHIBITEURS DE LA PHOSPHOLIPASE

(30) Priority: 31.03.2003 US 459362 P
(43) Date of publication of application: 04.01.2006
(73) Proprietor: ELI LILLY AND COMPANY, Indianapolis IN 46285 (US)
(72) Inventor: EACHO, Patrick, Irving, Indianapolis, IN 46236 (US); FOXWORTHY-MASON, Patricia, Sue, Indianapolis, IN 46250 (US); LIN, Ho-Shen, Indianapolis, IN 46217 (US); LOPEZ, Jose, Eduardo, Fishers, IN 46038 (US); MOSIOR, Marian, Kazimierz, Carmel, IN 46032 (US); RICHETT, Michael, Enrico, Indianapolis, IN 46250 (US)
(74) Representative: Commander, Paul Martin Brial
(86) International application number: PCT/US2004/006092
(87) International publication number: WO 2004/093872

(56) References cited:
- WO-A-00/07590
- JP-A- 11 044 927
- US-A- 4 340 662
- DATABASE CHEMABS [Online] CHEMICAL ABSTRACTS SERVICE, COLUMBUS, OHIO, US; 2002, STADLBAUER, W.: "Product class 2: 1H- and 2H-indazoles" XP002290147 retrieved from STN Database accession no. 2002:855866 & SCIENCE OF SYNTHESIS , 12, 227-324 CODEN: SSCYJ9, 2002,

## Description

### Field of the Invention

This invention relates to novel dihydro-1*H-*indazole-5-carboxylic acid derivative compounds useful for the treatment and/or prevention of diseases mediated by phospholipases including hepatic lipase and endothelial lipase.

### Backgound of the Invention

Hepatic lipase plays an important role in lipid metabolism. Hepatic lipase is a glycoprotein that functions as a ligand or as an enzyme of approximately 65Kda, which has been shown to catalyze the hydrolysis of lipids including triglycerides, diglycerides and phospholipids in native lipoproteins. It has also been shown to facilitate the selective uptake of cholesterol from high-density lipoproteins and the removal of remnant particles by the liver (Jonathan C. Cohen, et al Biochemistry 1992, 31: 8544-8551 and Neve et al Biochemistry J. 1998, 330:741-706).

Other studies showing the inverse relationship of HDL and hepatic lipase activity include for example, Haffner S.M. et al., "Studies on the metabolic mechanism of reduced high density lipoproteins during anabolic steroid therapy," Metabolism 1983; 32:413-420; Applebauril-Bowden D, et al., "The Dyslipoproteinemia of Anabolic steroid therapy: increase in hepatic triglyceride lipase precedes the decrease in high density lipoprotein-2 cholesterol," Metabolism 1987; 36:949-952; and Kantor M.A. et al., "Androgens reduce HDL-2 cholesterol and increase hepatic triglyceride lipase activity," Med. Sci. Sport exercise 1985; 17:462-465.

The inverse relationship between hepatic lipase activity and the level of HDL-cholesterol, particularly type-2 HDL-cholesterol, can be used to advantage in up-regulating the Level of HDL cholesterol-the good cholesterol.

Endothelial lipase (EL) is a newly described member of the lipase gene family. Like hepatic lipase, endothelial lipase has been implicated in the hydrolysis of HDL phospholipids and in the reduction of HDL-cholesterol in vivo. In experiments using hepatic lipase knockout mice the infusion of a polygonal antibody inhibitory to endothelial lipase resulted in a marked increase in HDL-cholesterol levels (Rader, D. J., et al Journal of Clinical Investigation (2003), 111(3) 357-362.

Chan, et al, Proceedings of the National Academy of Sciences U.S.A. (2003), 100(5), 2748-2753, has also reported the inverse relation between endothelial lipase and HDL-cholesterol.

WO 00/7590 discloses indole sPLA2 inhibitors for inhibiting sPLA2-mediated release of fatty acids for treatment of inflammatory diseases such as septic shock.

JP 11044927 discloses compounds useful in a heat developing recording material.

US 4,340,662 discloses compounds useful as improved masked reducing agents for imaging compositions.

Stadlbauer, Science of Synthesis (2002), 12, p.227-324 is a review of methods for preparation of 1H- and 2H-indazoles.

Given the preceding information, it is desirable to discover and develop compounds that increase HDL levels by methods that may include inhibiting the activity of hepatic lipase and/or endothelial lipase in order to treat, prevent and/or ameliorate the effects of hepatic lipase and/or endothelial lipase mediated diseases. Few therapeutically desirable agents are available to accomplish the task of increasing HDL levels hence the need for and utility of the present invention.

### Summary of the Invention

The present invention provides a compound of formula I 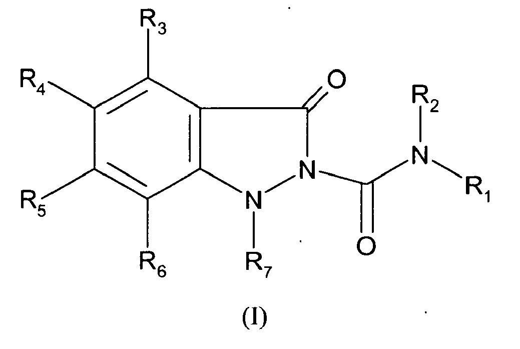 wherein;
R₁ is selected from C₅-C₁₃alkyl, C₁-C₁₂haloalkyl, C₄-C₁₂alkenyl, C₄-C₁₂alkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloatkyl, C₂-C₈alkenyl, C₂-C₈alkynyl, phenyl, benzyl, hydroxy, C₁-C₅alkoxy, -(CH₂)ₘCOOC₁-C₅alkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₂-C₁₂alkynyl, C₂-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, with the proviso that the compound of formula (I) is not: 4,6-dichloro-3-oxo-1,3-dihydro-indazole-2-carboxylic acid phenylamide; 3-oxo-1,3-dihydro-indazole-2,4-dicarboxylic acid-2-butylamide-4-octylamide; 2-octylcarbamoyl-3-oxo-2,3-dihydro-1H-indazole-4-carboxylic acid; 3-oxo-1,3-dihydro-indazole-2-carboxylic acid dodecylamide; 3-oxo-1,3-dihydro-indazole-2-carboxylic acid octylamide; or 3-oxo-1-phenyl-1,3-dihydro-indazole-2-carboxylic acid phenylamide; and with the further proviso that when R₃, R₄, R₅, R₆ and R₇ are each hydrogen, R₁ is not phenyl, an alkyl group having up to seven carbons or an alkylphenyl group where the alkyl group has up to seven carbons.

The present invention provides a pharmaceutical composition containing a dihydro-1H-indarzole-5-carboxylic acid derivative compound according to any one of claims 1 to 9 together with a pharmaceutically acceptable carrier or diluent.

The present invention relates to the use of a compound of claim 9 for the manufacture of a medicament for the treatment and /or prevention of hepatic lipase and/or endothelial lipase-mediated disease.

The present invention provides a use of a dihydro-1*H*-indolazole-5-carboxylic acid derivative compound according to claim 9 for the manufacture of a medicament for the treatment of a mammal to alleviate the pathological effects of low HDL.

The present invention also provides a compound according to claim 9 for use as a medicament.

The present invention further provides a dihydro-1H-indazole-5-carboxylic acid derivative compound of formula (I) 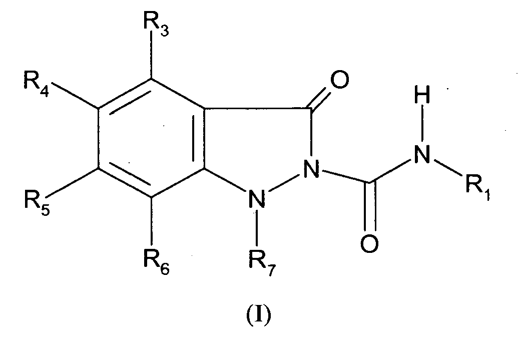 wherein;
R₁ is selected from the C₅-C₁₃alkyl, C₁-C₁₂haloalkyl, C₄-C₁₂alkenyl, C₄-C₁₂alkynyl, C₁-C₅alkylC₃-C₅cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloalkyl, C₂-C₈alkenyl, C₂-C₈alkynyl, phenyl, benzyl, hydroxy, C₁-C₅ alkoxy, -(CH₂)ₘCOOC₁-C₅alkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₂-C₁₂alkynyl, C₂-C₁₂alkylaryl, C₁-C₁₂alkylcyclohexyl, C₁-C₁₂alkylcyclopentyl, C₁-C₁₂alkytheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for use as a medicament.

The present invention also relates to a use of a dihydro-1H-indazole-5-carboxylic acid derivative compound of formula (I) 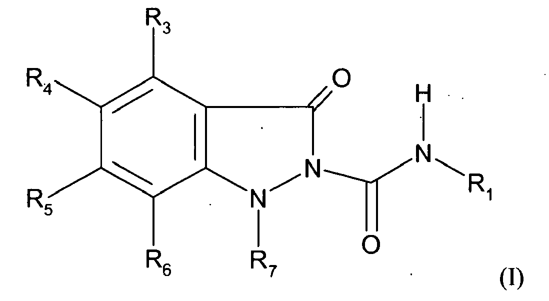 wherein; R₁ is selected from C₅-C₁₃alkyl, C₁-C₁₂haloalkyl, C₄-C₁₂alkenyl, C₄-C₁₂alkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₂-C₁₂alkynyl, C₂-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for the manufacture of a medicament for the treatment of a mammal to alleviate the pathological effects of low HDL.

The present invention further relates to a use of a dihydro-1H-indazole-5-carboxylic acid derivative compound of formula (I) 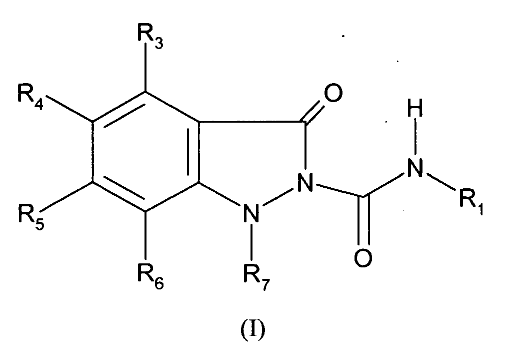 wherein;
R₁ is selected from C₅-C₁₃alkyl, C₁-C₁₂haloalkyl, C₄-C₁₂alkenyl, C₄-C₁₂alkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₂-C₁₂alkynyl, C₂-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b} phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for the manufacture of a medicament for the treatment or prevention of hepatic lipase and/or endothelial lipase-mediated disease.

### Definitions:

The phrase, "hepatic lipase and/or endothelial lipase mediated-diseases" refers to diseases symptomatic of low HDL levels, caused by, modulated by, exacerbated by or induced directly or indirectly by elevated hepatic lipase and/or endothelial lipase activity, and include for example, hypercholesterolemia, hyperlipidemia, stroke, hypertriglyceridemia, atherosclerosis and related diseases. Treatment and/or prevention of such diseases comprises administering to a mammal in need of such treatment a therapeutically effective amount of the compound of formula I in an amount sufficient to inhibit, ameliorate and/or prevent hepatic lipase and/or endothelial lipase activity and to thereby inhibit or prevent the deleterious effects of hepatic lipase and/or endothelial lipase activity.

The term "Active Ingredient" as used herein refers to a compound(s) of Formula (I) or a pharmaceutically acceptable salt, solvate, prodrug, racemate or enantiomer thereof either as the pure compound or delivered as a pharmaceutical formulation or a pharmaceutical composition. The pharmaceutical composition or formulation containing a compound of the invention and other compound(s) or treatment regimens useful for the treatment and/or prevention of diseases associated with or exacerbated by hepatic lipase and/or endothelial lipase activity (combination drugs) are contemplated to be within the meaning of the term "Active Ingredient(s)."

The term, "indazole nucleus", or "dihydro-1*H-*indazole nucleus" as used herein refers to a nucleus (having numbered positions) with the structural formula (X):

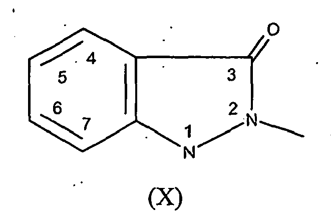

The dihydro-1*H-*indazole derivative compounds of the invention employ certain defining terms as follows:
' The term, "alkyl" by itself or as part of another substituent means, unless otherwise defined, a straight or branched chain monovalent hydrocarbon radical such as methyl, ethyl, n-propyl, isopropyl, n-butyl, tertiary butyl, sec-butyl, n-pentyl, and n-hexyl.
The term, "alkenyl" employed alone or in combination with other terms means a straight chain or branched monovalent hydrocarbon group having the stated number ranges of carbon atoms, and typified by groups such as vinyl, propenyl, crotonyl, isopentenyl, and various butenyl isomers.
The term, "hydrocarbyl" means an organic group containing only carbon and hydrogen.
The term, "halo" means fluoro, chloro, bromo, or iodo.
The term, "heterocyclic radical or heterocyclic group", refers to radicals or group derived from monocyclic or polycyclic, saturated or unsaturated, substituted or unsubstituted heterocyclic nuclei having 5 to 14 ring atoms and containing from 1 to 3 hetero atoms selected from the group consisting of nitrogen, oxygen or sulfur. Typical heterocyclic, radicals are pyrrolyl, pyrrolodinyl, piperidinyl, furanyl, thiophenyl, pyrazolyl, imidazolyl, phenylimidazolyl, triazolyl, isoxazolyl, oxazolyl, thiazolyl, thiadiazolyl, benzo(b)thiophenyl, carbazolyl, norharmanyl, azabenzo(b)thiophenyl, benzofuranyl, dibenzofuranyl, dibenzothiophenyl, indazolyl, imidazo(1.2-A)pyridinyl, benzotriazolyl, anthranilyl, 1,2-benzisoxazolyl, benzoxazolyl, benzothiazolyl, purinyl, pyridinyl, dipyridylyl. phenylpyridinyl, benzylpyridinyl, pyrimidinyl, phenylpyrimidinyl, pyrazinyl, 1,3,5-triazinyl, quinolinyl, phthalazinyl, quinazolinyl, morpholino, thiomorpholino, homopiperazinyl, tetrahydrofuranyl, tetrahydropyranyl, oxacanyl, 1,3-dioxolanyl, 1,3-dioxanyl, 1,4-dioxanyl, tetrahydrothiophenyl, pentamethylenesulfadyl, 1,3-dithianyl, 1,4-dithianyl, 1,4-thioxanyl, azetidinyl, hexamethyleneiminium, heptamethyleneiminium, piperazinyl and quinoxalinyl.
The terms "C₁-C₁₂alkylcyclopentyl," "C₁-C₁₂alkylcyclohexyl," or "C₁-C₁₂alkylheterocyclic" represent respectively a C₁-C₁₂alkyl, C₁-C₁₂alkyl, or C₁-C₁₂alkyl attached to a cylopentyl, cyclohexyl, and heterocyclic group respectively, wherein the entire group is attached to the dihydro-1*H-*indazole nucleus (X) or other substrate via the alkyl terminus at indicated or designated positions. The term "cycloalkyl" without more implies a cycloalkyl group having from 3 to 8 carbon atoms.
The term "substituted group" is an organic group substituted with one or more suitable substituents. For example, substituted phenyl as used herein refers to a phenyl group having one to three substituents selected from C₁-C₁₂alkyl, C₂-C₁₂haloalkyl, C₂-C₁₂alkenyl, C₂-C₁₂alkynyl, C₂-C₁₂alkylaryl, C₁-C₁₂alkylcydohexyl, C₁-C₁₂alkylcyclopentyl, C₁-C₁₂alkylheterocyclic, (CH₂)ₘCOOH, (CH₂)ₘCO(C₁-C₁₀)alkyl, (CH₂)ₘ COO(C₁-C₁₀)alkyl, (CH₂)ₘ COO(C₁-C₁₀)alkylaryl, C₁-C₁₀alkylamino, halo, (CH₂)ₘ CONH₂, (CH₂)ₘCON((C1-C6)alkyl)₂, phenyl, substituted phenyl, or aryl, wherein m = 0, 1, 2, or 3. Similarly, the term substituted benzyl means a benzyl group (CH₂Phenyl) having substitution on the phenyl ring as described above. Analogously, the term aryl as used herein has its usual meaning and especially refers to the benzyl group.

As used herein the terms "group", "radical" or "fragment" are synonymous and are intended to indicate functional groups or fragments of molecules attachable to a bond or other fragments of molecules. For example acetamide group represent the acetamide fragment or radical. Structures of groups, radicals or fragments unattached to the dihydro-1*H-*indazole-5-carboxylic acid derivative nucleus have been drawn to show the first line as a connecting bond only. Thus, the group 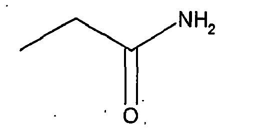 represents the acetamide radical or group, not the propanamide radical unless otherwise indicated.

### Preferred Subgroups of Compounds of Formula (I):

Preferred R₁ substituents:
The preferred group for R₁ is a substituted or unsubstituted group selected from the group consisting of C₅-C₁₃alkyl, C₁-C₅alkylcycloalkyl, C₄-C₁₂cycloalkenyl, cycohexylmethyl, cyclopentylmethyl, cyclohexylethyl, substituted or unsubstituted benzyl.

A most preferred R₁ is selected from the group consisting of 2, 4 or 5-substituted benzyl, 2,5 disubstituted benzyl, 2, 4-disubstituted benzyl, or 3,5-disubstitued benzyl. Preferred substituents on the benzyl group include methyl, ethyl, propyl, butyl, pentyl, hexyl, cyclohexylmethyl, phenyl, and cycloheptylmethyl. Most preferred substituents on the benzyl group include methyl, ethyl, propyl, isopropyl, isobutyl, tert-butyl, and pentane.

### Preferred R₃, R₄, R₅, and R₆ substituents:

R₃, R₄, R₅, and R₆ are preferably independently selected from the group consisting of hydrogen, C₁-C₆alkyl, C₂-C₆alkenyl, -O-(C₁-C₆)alkyl, -S-(C₁-C₆)alkyl, -C₅-C₁₂cycloalkyl, (CH₂)ₘCOOH, (CH₂)CO(C₁-C₁₀)alkyl, (CH₂)ₘ COO(C₁-C₁₀)alkyl, (CH₂)ₘCOO(C₁-C₁₀)alkylaryl, C₁-C₁₀alkylamino, halo, (CH₂)ₘCONH₂, (CH₂)ₘCONR^{a}R^{b}, phenyl, substituted phenyl, or aryl;. Particularly preferred R₃, R₄, R₅, and R₆ groups are selected from hydrogen, methyl, ethyl, propyl, chloro, fluoro, -COOH, CO(C₁-C₁₀)alkyl, COO(C₁-C₁₀)alkyl, and CONH₂ and salts thereof. More particularly hydrogen, chloro, and sodium, potassium and lithium salts of -COOH are preferred as independently selected R₃, R₄, R₅, and R₆ groups.

A preferred compound of the invention is a compound selected from the group consisting of:
3-Oxo-1,3-dihydro-indazole-2-carboxylic acid (2-ethyl-hexyl)-amide,
3-Oxo-1,3-dihydro-indazole-2-carboxylic acid (3,7-dimethyl-octa-2,6-dienyl)-amide,
3-Oxo-1,3-dihydro-indazole-2-carboxylic acid cyclohexylmethyl-amide,
1-Methyl-3-oxo-1,3-dihydro-indazole-2-carboxylic acid pentylamide,
1-Butyl-3-oxo-1,3-dihydro-indazole-2-carboxylic acid pentylamide,
1-Benzyl-3-oxo-1,3-dihydro-indazole-2-carboxylic acid pentylamide,
3-Oxo-1-phenethyl-1,3-dihydro-indazole-2-carboxylic acid pentylamide,
1-(2-Methoxy-ethyl)-3-oxo-1,3-dihydro-indazole-2-carboxylic acid hexylamide,
(2-Hexylcarbamoyl-3-oxo-2,3-dihydro-indazol-1-yl)-acetic acid methyl ester,
3-Oxo-1,3-dihydro-indazole-2-carboxylic acid 2-methyl-benzylamide,
3-Oxo-1,3-dihydro-indazole-2-carboxylic acid 3-methyl-benzylamide,
3-Oxo-1,3-dihydro-indazole-2-carboxylic acid 4-methyl-benzylamide,
3-Oxo-1,3-dihydro-indazole-2-carboxylic acid 2-trifluoromethyl-benzylamide,
3-oxo-1,3-dihydro-indazole-2-carboxylic acid 2-ethyl-benzylamide,
3-Oxo-1,3-dihydro-indazole-2-carboxylic acid 3-ethyl-benzylamide,
3-Oxo-1,3-dihydro-indazole-2-carboxylic acid 3-fluoro-benzylamide,
3-Oxo-1,3-dihydro-indazole-2-carboxylic acid 3-chloro-benzylamide,
3-Oxo-1,3-dihydro-indazole-2-carboxylic acid 3-methoxy-benzylamide,
3-Oxo-1,3-dihydro-indazole-2-carboxylic acid 3-trifluoromethoxy-benzylamide,
3-Oxo-1,3-dihydro-indazole-2-carboxylic acid 2-methylsulfanyl-benzylamide,
3-Oxo-1,3-dihydro-indazole-2-carboxylic acid 4-amino-benzylamide,
3-Oxo-1,3-dihydro-indazole-2-carboxylic acid 4-dimethylamino-benzylamide,
3-Oxo-1,3-dihydro-indazole-2-carboxylic acid (biphenyl-3-ylmethyl)-amide,
3-Oxo-1,3-dihydro-indazole-2-carboxylic acid (naphthalen-1-ylmethyl)-amide,
3-Oxo-1,3-dihydro-indazole-2-carboxylic acid (4-methyl-naphthalen-1-ylmethyl)-amide,
3-Oxo-1,3-dihydro-indazole-2-carboxylic acid 5-chloro-2-methyl-benzylamide,
3-Oxo-1,3-dihydro-indazole-2-carboxylic acid 2-isopropyl-6-methyl-benzylamide,
3-Oxo-1,3-dihydro-indazole-2-carboxylic acid 2,4-dichloro-benzylamide,
3-Oxo-1,3-dihydro-indazole-2-carboxylic acid 5-chloro-2-methoxy-benzylamide
3-Oxo-1,3-dihydro-indazole-2-carboxylic acid 2,4-dimethoxy-benzylamide,
3-Oxo-1,3-dihydro-indazole-2-carboxylic acid 3,4-dihydroxy-benzylamide,
3-Oxo-1,3-dihydro-indazole-2-carboxylic acid (2,3-dihydro-benzo[1,4]dioxin-6-ylmethyl)-amide,
3-Oxo-1,3-dihydro-indazole-2-carboxylic acid 2,4,6-trimethyl-benzylamide,
3-Oxo-1,3-dihydro-indazole-2-carboxylic acid 4,6-dichloro-2-methyl-benzylamide,
3-Oxo-1,3-dihydro-indazole-2-carboxylic acid 3,4,5-trimethoxy-benzylamide,
3-Oxo-1,3-dihydro-indazole-2-carboxylic acid (4-phenyl-but-3-enyl)-amide,
4-methyl-3-oxo-1,3-dihydro-indazole-2-carboxylic acid 2-methyl-benzylamide,
6-methyl-3-oxo-1,3-dihydro-indazole-2-carboxylic acid 2-methyl-benzylamide,
4-chloro-3-oxo-1,3-dihydro-indazole-2-carboxylic acid 2-methyl-benzylamide,
5-Chloro-3-oxo-1,3-dihydro-indazole-2-carboxylic acid 2-methyl-benzylamide,
6-Chloro-3-oxo-1,3-dihydro-indazole-2-carboxylic acid 2-methyl-benzylamide,
7-methoxy-3-oxo-1,3-dihydro-indazole-2-carboxylic acid 2-methyl-benzylamide,
5-hydroxy-3-oxo-1,3-dihydro-indazole-2-carboxylic acid 2-methyl-benzylamide,
5-Nitro-3-oxo-1,3-dihydro-indazole-2-carboxylic acid 2-methyl-benzylamide,
2-(2-methyl-benzylcarbamoyl)-3-oxo-2,3-dihydro-1*H* indazole-4-carboxylic acid,
2-(2-methyl-benzylcarbamoyl)-3-oxo-2,3-dihydro-1*H*-indazole-5-carboxylic acid,
2-(2-Methyl-benzylcarbamoyl)-3-oxo-2,3-dihydro-1*H*-indazole-6-carboxylic acid,
2-(2-methyl-benzylcarbamoyl)-3-oxo-2,3-dihydro-1*H*-indazole-7-carboxylic acid,
3-Oxo-2-(2-trifluoromethyl-benzylcarbamoyl)-2,3-dihydro-1*H*-indazole-7-carboxylic acid,
2-(5-fluoro-2-methyl-benzylcarbamoyl)-3-oxo-2,3-dihydro-1*H*-indazole-4-carboxylic acid,
2-(5-Fluoro-2-methyl-benzylcarbamoyl)-3-oxo-2,3-dihydro-1*H*-indazole-7-carboxylic acid,
2-(2,6-dimethyl-benzylcarbamoyl)-3-oxo-2,3-dihydro-1*H-*indazole-4-carboxylic acid,
2-(2,6-Dimethyl-benzylcarbamoyl)-3-oxo-2,3-dihydro-1*H*-indazole-7-carboxylic acid,
2-(2-Ethyl-6-methyl-benzylcarbamoyl)-3-oxo-2,3-dihydro-1*H*-indazole-4-carboxylic acid,
2-(2-Ethyl-6-methyl-benzylcarbamoyl)-3-oxo-2,3-dihydro-1*H*-indazole-5-carboxylic acid,
2-(2-Ethyl-6-methyl-benzylcarbamoyl)-3-oxo-2,3-dihydro-1*H*-indazole-6-carboxylic acid,
2-(2-Ethyl-6-methyl-benzylcarbamoyl)-3-oxo-2,3-dihydro-1*H*-indazole-7-carboxylic acid,
2-(2-Isopropyl-6-methyl-benzylcarbamoyl)-3-oxo-2,3-dihydro-1*H-*indazole-4-carboxylic acid,
2-(2-Isopropyl-6-methyl-benzylcarbamoyl)-3-oxo-2,3-dihydro-1*H*-indazole-7-carboxylic acid,
2-(2-*tert*-Butyl-6-methyl-benzylcarbamoyl)-3-oxo-2,3-dihydro-1*H-*indazole-7-carboxylic acid,
2-(4-butyl-2-methyl-benzylcarbamoyl)-3-oxo-2,3-dihydro-1*H-*indazole-4-carboxylic acid,
2-(2,6-Diethyl-benzylcarbamoyl)-3-oxo-2,3-dihydro-1*H-*indazole-4-carboxylic acid,
2-(2,6-Diethyl-benzylcarbamoyl)-3-oxo-2,3-dihydro-1*H*-indazole-7-carboxylic acid,
2-(2,6-diisopropyl-benzylcarbamoyl)-3-oxo-2,3-dihydro-1*H-*indazole-4-carboxylic acid,
2-(2,6-Diisopropyl-benzylcarbamoyl)-3-oxo-2,3-dihydro-1*H*-indazole-7-carboxylic acid,
2-(2,4,6-trimethyl-benzylcarbamoyl)-3-oxo-2,3-dihydro-1*H*-indazole-4-carboxylic acid,
2-[(4-methyl-naphthalen-1-ylmethyl)-carbamoyl]-3-oxo-2,3-dihydro-1*H*-indazole-4-carboxylic acid,
3-Oxo-2-(4-phenyl-butylcarbamoyl)-2,3-dihydro-1*H*-indazole-4-carboxylic acid,
2-(4-Cyclohexyl-butylcarbamoyl)-3-oxo-2,3-dihydro-1*H*-indazole-4-carboxylic acid,
3-Oxo-2-(3-phenoxy-propylcarbamoyl)-2,3-dihydro-1*H*-indazole-4-carboxylic acid,
2-Hexylcarbamoyl-3-oxo-2,3-dihydro-1*H*-indazole-4-carboxylic acid,
2-(2-Ethyl-6-methyl-benzylcarbamoyl)-1-methyl-3-oxo-2,3-dihydro-1*H*-indazole-7-carboxylic acid,
1-Ethyl-2-(2-ethyl-6-methyl-benzylcarbamoyl)-3-oxo-2,3-dihydro-1*H*-indazole-7-carboxylic acid,
2-(2-Ethyl-6-methyl-benzylcarbamoyl)-3-oxo-1-propyl-2,3-dihydro-1*H*-indazole-7-carboxylic acid,
1-Carboxymethyl-2-(2-ethyl-6-methyl-benzylcarbamoyl)-3-oxo-2,3-dihydro-1*H-*indazole-7-carboxylic acid,
2-(2-Ethyl-6-methyl-benzylcarbamoyl)-1-methoxycarbonylmethyl-3-oxo-2,3-dihydro-1*H*-indazole-7-carboxylic acid,
2-(2-Isopropyl-6-methyl-benzylcarbamoyl)-1-methyl-3-oxo-2,3-dihydro-1*H*-indazole-7-carboxylic acid,
1-Ethyl-2-(2-isopropyl-6-methyl-benzylcarbamoyl)-3-oxo-2,3-dihydro-1*H*-indazole-7-carboxylic acid,
2-(2-Isopropyl-6-methyl-benzylcarbamoyl)-3-oxo-1-propyl-2,3-dihydro-1*H*-indazole-7-carboxylic acid,
1-(3-Chloro-propyl)-2-(2-isopropyl-6-methyl-benzylcarbamoyl)-3-oxo-2,3-dihydro-1*H-*indazole-7-carboxylic acid,
2-(2-Isopropyl-6-methyl-benzylcarbamoyl)-3-oxo-1-(prop-2-ynyl)-2,3-dihydro-1*H-*indazole-7-carboxylic acid,
1-Allyl-2-(2-isopropyl-6-methyl-benzylcarbamoyl)-3-oxo-2,3-dihydro-1*H*-indazole-7-carboxylic acid,
I -Butyl-2-(2-isopropyl-6-methyl-benzylcarbamoyl)-3-oxo-2,3-dihydro-1*H*-indazole-7-carboxylic acid,
1-Cyanomethyl-2-(2-isopropyl-6-methyl-benzylcarbamoyl)-3-oxo-2,3-dihydro-1*H-*indazole-7-carboxylic acid,
1-Cyclopropylmethyl-2-(2-isopropyl-6-methyl-benzylcarbamoyl)-3-oxo-2,3-dihydro-1*H-*indazole-7-carboxylic acid,
1-Carboxymethyl-2-(2-isopropyl-6-methyl-benzylcarbamoyl)-3-oxo-2,3-dlhydro-1*H-*indazole-7-carboxylic acid,
2-(2-Isopropyl-6-methyl-benzylcarbamoyl)-1-methoxycarbonylmethyl-3-oxo-2,3-dihydro-1*H*-indazole-7-carboxylic acid,
1-Carbamoylmethyl-2-(2-isopropyl-6-methyl-benzylcarbamoyl)-3-oxo-2,3-dihydro-1*H-*indazole-7-carboxylic acid,
1-Benzyl-2-(2-isopropyl-6-methyl-benzylcarbamoyl)-3-oxo-2,3-dihydro-1*H*-indazole-7-carboxylic acid,
2-(2-*tert*-Butyl-6-methyl-benzylcarbamoyl)-1-methyl-3-oxo-2,3-dihydro-1*H*-indazole-7-carboxylic acid,
2-(2,6-Diethyl-benzylcarbamoyl)-1-methyl-3-oxo-2,3-dihydro-1*H*-indazole-7-carboxylic acid,
2-hexylcarbamoyl-3-oxo-2,3-dihydro-1*H*-indazole-4-carboxylic acid methyl ester,
2-Hexylcarbamoyl-3-oxo-2,3-dihydro-1*H*-indazole-4-carboxylic acid allyl ester,
2-hexylcarbamoyl-3-oxo-2,3-dihydro-1*H*-indazole-7-carboxylic acid allyl ester,
2-hexylcarbamoyl-3-oxo-2,3-dihydro-1*H*-indazole-7-carboxylic acid methyl ester,
2-(2-Isopropyl-6-methyl-benzylcarbamoyl)-3-oxo-2,3-dihydro0-1*H*-indazole-4-carboxylic acid methyl ester, and
2-(2-Isopropyl-6-methyl-benzylcarbamoyl)-3-oxo-2,3-dihydro-1*H*-indazole-7-carboxylic acid methyl ester.

More preferred compounds of the invention are represented by the formulae (C1), (C2), (C3), (C4) and (C5): 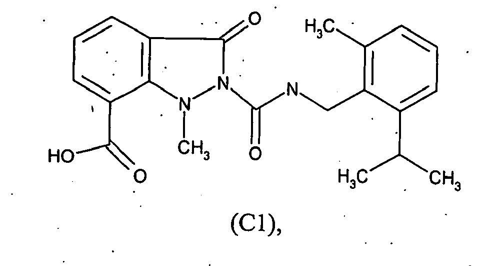 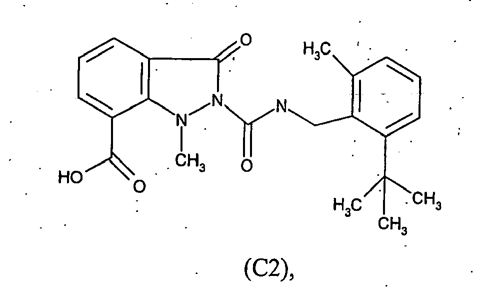 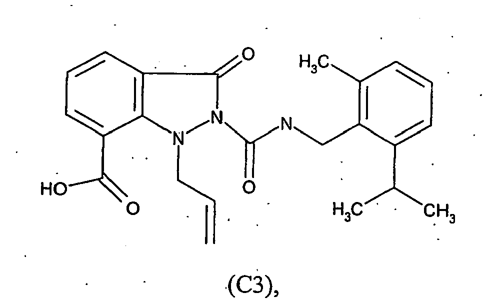 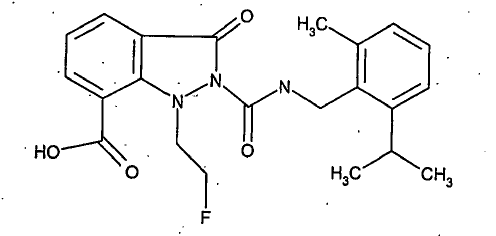 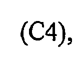 or

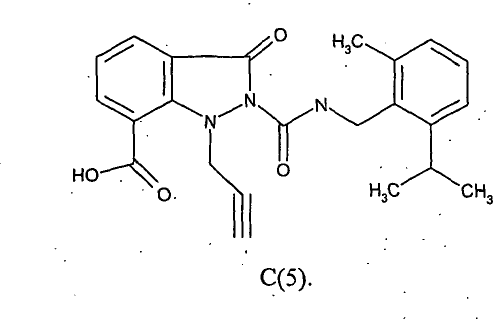

The salts of the 1*H*-indazole compounds represented by formula (I), are an additional aspect of the invention.

In those instances when the compound of the invention possesses acidic or basic functional groups, various salts may be formed which are more water soluble and more physiologically suitable than the parent compound. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an ion-exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, et al., "Pharmaceutical Salts," J. Phar. Sci., 66: 1-19 (1977)). Moreover, the basic group(s) of the compound of the invention may be reacted with suitable organic or inorganic acids to form salts such as acetate, benzenesulfonate, benzoate, bicarbonate, bisulfate, bitartrate, borate, hydrobromide, camsylate, carbonate, chloride, clavulanate, citrate, chloride, edetate, edisylate, estolate, esylate, fluoride, fumarate, gluceptate, gluconate, glutamate, glycolylarsanilate, hexylresorcinate, hydrochloride, hydroxynaphthoate, hydroiodide, isothionate, lactate, lactobionate, laurate, maleate, mandelate, mesylate, methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate.

Certain compounds of the invention may possess one or more chiral centers, and thus, may exist in optically active forms. Likewise, when the compounds contain an alkenyl or alkenyl group, there exist the possibility of cis- and trans- isomeric forms of the compounds. The R- and S- isomers and mixtures thereof, including racemic mixtures as well as mixtures of cis- and trans- isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods. For example, a racemic mixture may be reacted with a single enantiomer of some other compound. This changes the racemic form into a mixture of stereoisomers and diastereomers, because they have different melting points, different boiling points, and different solubilities and can be separated by conventional means, such as crystallization.

The following paragraph is provided for reference purposes only and does not form part of the invention. Prodrugs are derivatives of the compounds of the invention which have chemically or metabolically cleavable groups and become by solvolysis or under physiological conditions the compounds of the invention which are pharmaceutically active *in vivo*. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acidic compound with a suitable alcohol, or amides prepared by reaction of the parent acid compound with a suitable amine. Simple aliphatic or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters. Particularly preferred esters as prodrugs are methyl, ethyl, propyl, isopropyl, n-butyl, isobutyl, tert-butyl, morpholinoethyl, and N,N-diethylglycolamido.

### Methods for Preparing the Compounds of the Invention

Compounds useful for the invention are prepared following the general procedures outlined below. One of skill in the art is aware that minimal experimentation would be needed to modify the schemes herein provided for the preparation of other compounds of the invention. Scheme 1 provides a general method starting from available dihydroindazole-2-one compound.

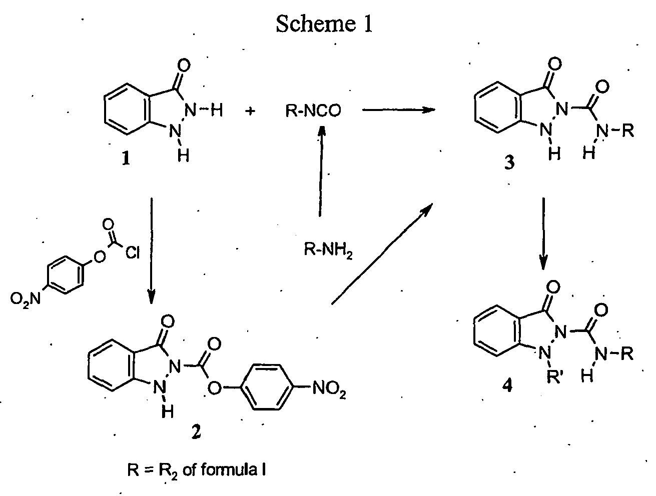

According to Scheme 1, 2,3-dihydro-indazole **1**may be reacted with p-nitophenylchloroformate or other suitable leaving group synthon in the presence of a suitable base to afford the compound **2**. The resulting compound **2** is readily reacted with an amine to effect displacement of the *p*-nitrophenol group affording the carbamoyl compound **3**. The carbamoyl compound **3** may be further alkylated or otherwise substituted with a nucleophile at the free ring nitrogen atom to afford an N-substituted dihydroindazole carboxylic acid derivative **4,** a compound of the invention.

Alternatively, the dihydro-indazole **1** may be reacted with an isocynate having the desired alkyl, aryl, or other substituent to afford directly the compound **3**. The compound **3** is a compound of the invention, which may itself be converted to an N-substituted derivative compound of the formula **4** as discussed above. The isocyanate reagent may be prepared as shown in Scheme 1a below wherein R is represented by the group 5-methylhexyl: 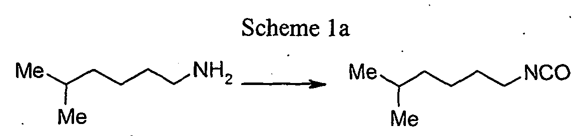 Preparation of the isocyanate may be accomplished by adding a solution of the amine e.g. 5-methylhexylamine, and a proton sponge e.g. triethylamine, dropwise to a cold solution of triphosgene in anhydrous dichloromethane. A detailed procedure for the formation of the isocyanate derivative is provided in the experimental section, or may be found in general organic texts and references therein or is known to one of skill in the art. The resulting isocyanate is typically obtained after acidic workup and purification by chromatography or crystallization.

Benzyl derivatives of compounds of formula I may be prepared as shown in Scheme 2. 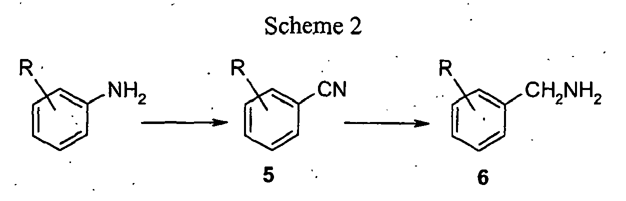 The amide component of the compound of formula I is introduced via an isocyanate or amine prepared from the corresponding or nitrile. As shown above, an appropriately substituted aniline is converted to the nitrile **5** following procedures described in the experimental section and/or known to one of skill in the art. For example, the aniline is reacted with tert-butoxynitrite in the presence of copper cyanide in DMSO or other suitable solvent. The initial reaction results in a diazonium intermediate that is displaced by cyanide ion to form the nitrile **5.** The nitrile **5** is then reduced to the benzyl amine derivative **6** using for example lithium aluminum hydride in anhydrous ethyl alcohol as a reducing agent. The benzyl amine derivative **6** is then reacted with an activated dihydroindazole **2** having the *p*-*nitrophenoxy* leaving group as shown in Scheme **1** and discussed previously.

Alternatively, the amine **6** may be converted to the isocyanate **7** via reaction of the amine with triphosgene in the presence of a proton sponge. The resulting isocyanate **7** is then reacted with an appropriately substituted dihydro-indazole to afford the compound 8, a compound of the invention.

In another procedure, the benzyl amine compound **6** may be reacted directly with a nucleophile source such as compound 2 to afford the desired compound **8** as shown in Scheme 3

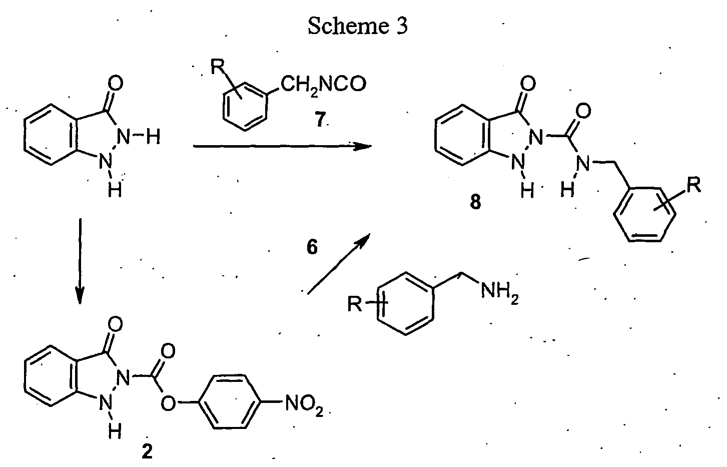

Compounds of formula I wherein the starting material dihydro-indazole is not readily available may be prepared starting with an appropriately substituted 2-aminobenzoic acid as shown in Scheme 4

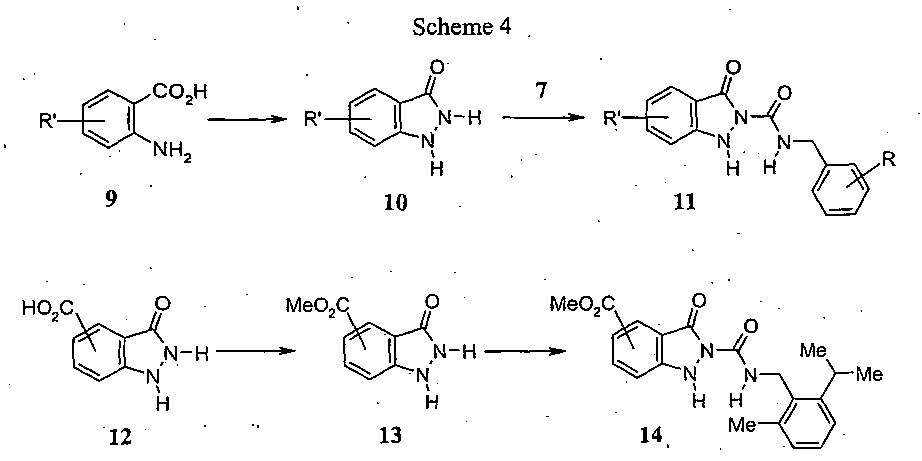

The reaction to form the dihydro-indazole group starting with an aminobenzoic acid **9** is believed to proceed via a diazonium salt intermediate (Sandmeyer reaction, see March, J., Advanced Organic Chemistry, 3rd Edition, Wiley Interscience, New York, New York). The diazonium intermediate may be formed by adding sodium nitrite to a solution of **9.** The diazonium salt intermediate is reduced by stannuous chloride to form a phenylhydrazine intermidate which ring closes *in-situ* to afford the dihydro-indazole compound **10.** Detailed procedures are provided in the experimental section, are known to one of skill in the art, or are readily accessed by one of skill in the art via available literature and reference sources.

The dihydro-indazole compound **10** may then be reacted with isocyanate **7** (from Scheme 3) to afford compound **11,** a compound of the invention. The carboxylic acid side chain on the dihydro-indazole **12** may be converted to the methyl ester **13** prior to reaction with an isocyanate derivative to afford the compound **14.** The compound **14** is a compound of the invention.

Protection of side chain substitutents may also be necessary for the purpose of alkylating or substituting on the secondary amine position of the dihydro-indazole as shown in Scheme 5:

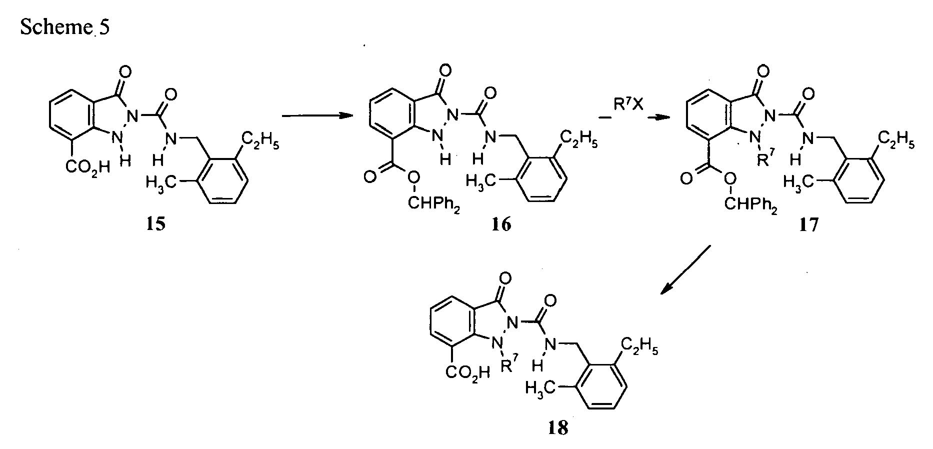

As shown in scheme 5, the carboxylic acid side chain of compound **15** may be protected by reaction with diphenylmethyl diazomethane to afford the corresponding ester **16** (diphenylmethylester). Protection of the acid allows for a facile nucleophilic reaction of an alkyl halide (R⁷X) or other nucleophile source on the free dihydro-indazole ring NH proton of compound **16.** The resulting N alkyl or N-substituted compound **17** may be hydrolyzed under acidic conditions to afford the deprotected, N-substituted compound **18.**

Compounds of formula I wherein all of R₃, R₄, R₅, and R₆ are other than hydrogen are made starting with purchased starting materials having the requisite substituents or by methods known to one of skill in the art.

### Methods of Using the Compounds of the Invention:

The dihydro-1*H*-indazole-5-carboxylic acid derivative compounds described herein are believed to achieve their beneficial therapeutic action principally by direct inhibition of hepatic lipase and/or endothelial lipase activity.

Another aspect of this invention relates to inhibition or prevention of "Hepatic Lipase-Mediated Diseases" such as hypercholesterolemia, stroke, atherosclerosis and related diseases as described earlier.

As previously noted the compounds of this invention are useful for inhibiting hepatic lipase and/or endothelial lipase activity. By the term, "inhibiting" is meant the prevention or therapeutically significant reduction in hepatic lipase and/or endothelial lipase activity by the compounds of the invention. By "pharmaceutically acceptable" it is meant the carrier, diluent or excipient must be compatible with the other ingredients of the formulation and not deleterious to the recipient thereof.

The specific dose of a compound administered according to this invention to obtain therapeutic or ameliorative or prophylactic effect will, of course, be determined by the particular circumstances surrounding the case, including, for example, the compound administered, the route of administration and the condition being treated. Typical daily doses will contain a non-toxic dosage level of from about 0.01 mg/kg to about 50 mg/kg of body weight of an active compound of this invention.

Preferably compounds of the invention per Formula (I) or pharmaceutical formulations containing these compounds are in unit dosage form for administration to a mammal. The unit dosage form can be a capsule or tablet itself, or the appropriate number of any of these. The quantity of Active Ingredient in a unit dose of composition may be varied or adjusted from about 0.1 to about 500 milligrams or more according to the particular treatment involved. It may be appreciated that it may be necessary to make routine variations to the dosage depending on the age and condition of the patient. The dosage will also depend on the route of administration.

The compound of formula I may be administered by a variety of routes including oral, aerosol, transdermal, subcutaneous, intravenous, intramuscular, or intranasal.

Pharmaceutical formulations of the invention are prepared by combining (e.g., mixing) a therapeutically effective amount of the dihydro-1*H*-indazole-5-carboxylic acid derivative compound of the invention together with a pharmaceutically acceptable carrier or diluent. The present pharmaceutical formulations are prepared by known procedures using well-known and readily available ingredients.

In making the compositions of the present invention, the Active Ingredient will usually be admixed with a carrier, or diluted by a carrier, or enclosed within a carrier, which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. For example, for intravenous injection the compounds of the invention may be dissolved in at a concentration of 2 mg/ml in a 4% dextrose/0.5% Na citrate aqueous solution. Solid form formulations include powders, tablets and capsules. A solid carrier can be one or more substance, which may also act as flavoring agents, lubricants, solubilizers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc. A preferred tablet formulation for oral administration is one that affords rapid dissolution in the mouth of a patient in need thereof.

In powders the carrier is a finely divided solid, which is in admixture with the finely divided Active Ingredient. In tablets the Active Ingredient is mixed with a carrier having the necessary binding properties in suitable proportions and compacted in the shape and size desired. The powders and tablets preferably contain from about 1 to about 99 weight percent of the Active Ingredient, which is the novel compound of this invention. Suitable solid carriers are magnesium carbonate, magnesium stearate, talc, sugar lactose, pectin, dextrin, starch, gelatin, tragacanth, methylcellulose, sodium carboxymethyl cellulose, low melting waxes, and cocoa butter.

Sterile liquid form formulations include suspensions, emulsions, syrups and elixirs.

The Active Ingredient can be dissolved or suspended in a pharmaceutically acceptable carrier, such as sterile water, sterile organic solvent or a mixture of both. The Active Ingredient can often be dissolved in a suitable organic solvent, for instance aqueous propylene glycol. Other compositions can be made by dispersing the finely divided Active Ingredient in aqueous starch or sodium carboxymethyl cellulose solution or in a suitable oil.

"Active Ingredient", refers to a compound according to Formula (I) or a pharmaceutically acceptable salt, solvate, racemate or enantiomer thereof.

### Formulation 1

Hard gelatin capsules are prepared using the following ingredients:

| | Quantity |
|---|---|
| | (mg/capsule) |
| Active ingredient | 250 |
| Starch, dried | 200 |
| Magnesium stearate | 10 |
| Total | 460 mg |

### Formulation 2

A tablet is prepared using the ingredients below:

| | Quantity |
|---|---|
| | (mg/tablet) |
| Active Ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

### Formulation 3

An aerosol solution is prepared containing the following components:

| | Weight |
|---|---|
| Active Ingredient | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

### Formulation 4

Tablets, each containing 60 mg of Active Ingredient, are made as follows:

| | |
|---|---|
| Active Ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The Active Ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 5

Capsules, each containing 80 mg of Active Ingredient, are made as follows:

| | |
|---|---|
| Active Ingredient | 80 mg |
| Starch | 59 mg |
| Microcrystalline cellulose | 59 mg |
| Magnesium stearate | 2 mg |
| Total | 200 mg |

The Active Ingredient, cellulose, starch, and magnesium stearate are blended, passed through a No. 45 mesh U.S. sieve, and filled into hard gelatin capsules in 200 mg quantities.

### Formulation 6

Suppositories, each containing 225 mg of Active Ingredient, are made as follows:

| | |
|---|---|
| Active Ingredient | 225 mg |
| Saturated fatty acid glycerides | 2,000 mg |
| Total | 2,225 mg |

The Active Ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### Formulation 7

Suspensions, each containing 50 mg of Active Ingredient per 5 ml dose, are made

| | |
|---|---|
| as follows: | |
| Active Ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The Active Ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation 8

An intravenous formulation may be prepared as follows:

| | |
|---|---|
| Active Ingredient | 100 mg |
| Isotonic saline | 1,000 ml |

The solution of the above ingredients generally is administered intravenously to a subject at a rate of 1 ml per minute.

The abbreviations, symbols and terms used in the examples have the following meanings.
Ac = acetyl
Anal. = elemental analysis
calcd = calculated
Cpd. = compound
DMF = dimethylformamide
DMSO = dimethylsulfoxide
Et = ethyl
EtOAc = ethyl acetate
EtOH = ethanol
EtSH = ethanethiol
ESIMS = Electospray Ionization Mass Spectrometry
FAB = Fast Atom Bombardment (Mass Spectroscopy)
FDMS = field desorption mass spectrum
Hex = hexanes
HL = Hepatic Lipase
HPLC = High Performance Liquid Chromatograph
HRMS = high resolution mass spectrum
IR = Infrared Spectrum
Me = methyl
MeI = methyl iodide
MeOH = methanol
MPLC = Medium Pressure Liquid Chromatography
NMR = Nuclear Magnetic Resonance
PPA = polyphosphoric acid
Rochelle's Salt = potassium sodium tartrate
RPHPLC = Reversed Phase High Performance Liquid Chromatography
SiO₂ = silica gel
SM = starting material
Temp. = temperature
TFA = trifluoroacetic acid
THF = tetrahydrofuran
TLC = thin layer chromatography

### Hepatic Lipase Phospholipase Assay

Compounds of the present invention were found to be efficacious *in-vitro* in inhibiting hepatic lipase. Efficacy was determined by testing various compounds of the invention in a hepatic lipase assay discussed below, and disclosed in U.S patent application No. 09/609, 871 filed July 3, 2000.

### Reagents

Substrate Buffer A: 100 mM Hepes, pH 8.3 at 37 °C
Substrate Buffer B: 100 mM Hepes, pH 8.3 at 37 °C with 6.83 mM Triton X100
ThioPEG: Molecular wt. of 540
Recombinant Hepatic Lipase
Thiophospholipid: about 0.42 mM thiophospholipid in chloroform
DTNB Solution: about 50 nM DTNB in DMSO (dimethyl sulfoxide)

### Hepes Buffer A

For Hepes Buffer A, there is 2.4 g Hepes/100 mL water. Therefore 36 grams of Hepes is dissolved in 1500 mL of water. The mix solution's pH is adjusted to pH 8.3 at 37 °C and brought up to 1500 mL with water. 500 mL of Buffer A is retained for the Protein Buffer.

### Hepes Buffer B

To the remaining 1000 mL of Hepes Buffer A, 4.49 g of Triton X-100 is added and then the combination mixed on a stir plate. It is optimal that stock Buffer A not be too cold or Triton X-100 will take a long time to go into solution

### ThioPEG Substrate Solution

For 0.42 mM substrate stock, use 0.227 mg of thioPEG/mL of Substrate Buffer B. Approximately 20 mg of sn-1 thiol substituted Phosphatidyl Ethylene Glycol (see Examples for preparation method) is weighed into a vial, such as a scintillation vial. Enough chloroform should be added to make a 2.043 mg/mL solution. Sonicate the solution briefly until well dissolved. Next, pipette 1 mL of chloroform/substrate solution into each scintillation vial. This should give enough substrate for one full 96 well plate. Each vial is dried with nitrogen until solvent removed, swirling each vial simultaneously such that a thin film of substrate will be easily reconstituted in each buffer. Each vial is then frozen.

Daily stock preparation is performed for 9 mL of substrate (one microtiter plate). On the day of the assay, the substrate vial is removed from the freezer and combined with 9 mL of pre-warmed (37 °C) substrate buffer (the final concentration is 0.227 mg/mL). Place the buffer in a 37 °C water bath. Sonicate for 5 minutes or vortex until solution is clear before use.

### Enzyme Solution

The enzyme is stored at -80 °C in 100 or 50 µL portions. A 0.406 mg/mL recombinant hepatic lipase stock requires a 50-fold dilution. Therefore, to a 50 µL or 100 µL enzyme aliquot, 2450 µL or 4900 µL, respectively, of substrate Buffer A (protein buffer) should be added. The enzyme should then be stored on ice until ready to use. The protein concentration of enzyme is about 0.406 mg/mL.

### DTNB Solution

To make a 20 mg/mL stock solution, 2-3 mg of DTNB is weighed and then mixed with an appropriate amount of 100% DMSO (dimethyl sulfoxide) to make the desired concentration. This mixture is sonicated for five minutes.

The above solution should be diluted 10 fold with substrate Buffer B (concentration now 2 mg/mL). Then to the thioPEG substrate solution, add 60 µl of dilute DTNB per mL of thioPEG substrate solution. Thus for 9 mL of substrate, 540 microliters of dilute DTNB (final concentration in substrate solution = 0.11 mg/mL).

Table 1 below shows final assay volumes and concentrations of various components used following the above procedure.

**Table 1: Final Assay Volumes and Concentrations Hepatic Lipase Phospholipase Assay**

| Component | Assay Volume | Final Concentration |
|---|---|---|
| HL | 10 µl | 12.5 nM |
| Test Cpd. | 10 µl | Varies |
| ThioPEG Substrate (stock = | 80 µl | 90 mM Hepes |
| 0.42mM + substrate buffer) | | 5.8 mM TX100 |
| | | 0.336 mM ThioPEG (0.06 |
| | | mol fraction) 0.088 mM |
| | | DTNB/mL |

### Sample Preparation

The test compound is dissolved in pure DMSO at 1 µM (1000 nM). As shown below in Table 2, assay concentrations are 10, 1, 0.1, 0.33, 0.011, 0.0037, 0.0012 and 0.00041 µM. Table 2 shows the assay concentrations and the corresponding volume of stock and 10% DMSO for each concentration.

**Table 2: Assay concentrations for compound preparation**

| **Concentration (**µ**M)** | **Assay Conc. (**µ**M)** | **Microliters of stock solution** | **Diluents** |
|---|---|---|---|
| 100 | 10 | 50 of 1mM in straight DMSO | 450 µl of ***WATER*** |
| 10 | 1 | 5 µl of 100 µM | 450 µl 10% DMSO |
| 1 | 0.1 | 50 µl of 10 µM | 450 µl 10% DMSO |
| 0.33 | 0.033 | 200 µl of 1 µM | 400 µl 10% DMSO |
| 0.11 | 0.011 | 200 µl of 0.33 µM | 400 µl 10% DMSO |
| 0.037 | 0.0037 | 200 µl of 0.11 µM | 400 µl of 10% DMSO |
| 0.012 | 0.0012 | 200 µl of 0.037 µM | 400 µl of 10% DMSO |
| 0.0041 | 0.00041 | 200 µl of 0.012 µM | 400 µl of 10% DMSO |

### Assay Procedure

Using a spectrometer, DTNB is used as a thiol coloring reagent with an incubator temperature of 37 °C. Substrate Buffer B is placed in a 37 °C water bath to pre-warm. The substrate is removed from the freezer and 9 mL of substrate Buffer B, 100 mM Hepes, 6.83 mM Tx-100) is added, sonicated for 5 min. and then kept in a 37 °C water bath. Dilutions of the test compound are next made in preparation for assay.

10 µl of the diluted test compound are transferred via pipette into the wells. Control wells receive 10 µl each of 10% DMSO and enzyme solution, while blank wells receive 10 microliters of 10% DMSO and 10 microliters of saline (no enzyme).

Next, DTNB is weighed and diluted to 20 mg/mL with DMSO. The DTNB is then diluted 10 fold with the substrate Buffer B. 540 µl of diluted DTNB is added to 9 ml of ThioPEG and mixed well.

The stock enzyme is diluted with Buffer A. Next, 10 microliters of protein solution is added to each well except the blank, and the wells mixed. The stock solution and test compounds are incubated at 37 °C for 10 min. At 10 minutes, 80 microliters of substrate are added to each well. The plate is then placed in the spectrometer and read at 412 nM every 2 minutes for 30 minutes.

### Results for Hepatic Lipase Inhibition

| Compounds | IC₅₀ (nM) |
|---|---|
| 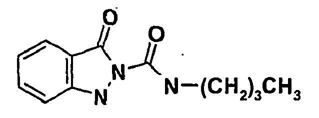 | 368 ± 15 |
| 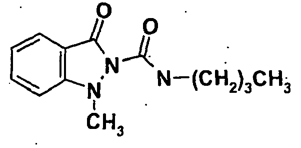 | 19 ± 0.4 |
| 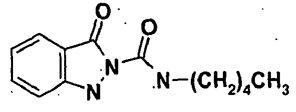 | 67.2 ± 3.2 |
| 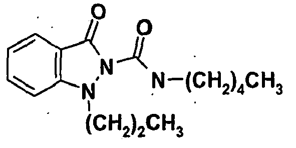 | 59.2 ± 1.1 |
| 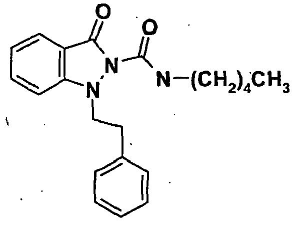 | 97.1 ± 1.0 |

### Methods for characterization of Endothelial Lipase activity

Hepatic lipase (HL) and endothelial lipase (EL) were expressed from AV12 cells. Aliquots from one day's collection of media were stored at-70 °C. Activity was measured for both enzymes in conditioned media, (non-purified ) where they were tested on the same plate with Thio PEG substrate (0.06 mol fraction, 7.24 mM total lipid), at 37 °C for 30 minutes. The HL, at 1x, had an OD of 14.7. The OD for EL at 1x was 6.029. Therefore, when HL was used.in studies where it was compared to EL, the HL was at 0.25x and EL was used at 1x. All experiments were done in triplicate with enzyme from conditioned media.

Kinetic experiments for EL were done varying the total lipid with a constant 0.044 mol fraction determining that a 10mM total lipid was optimal. In addition, kinetic experiments varying the mol fractions with a constant total lipid showed that 0.03 mol fraction was optimal. Each experiment was run three times.

Experiments to determine proper pH of the substrate to be used with EL were performed at 37 °C with the above-mentioned conditions. The enzyme was tested at pH 7.0, 7.4 and 8.3. The order of addition of reagents/enzyme was as follows: 10µL of 10% DMSO, 80 µL of substrate and 10 µL of enzyme. Data represents an average of three experiments.

Temperature of the assay was varied from 26.9 °C to 37 °C with the above-mentioned conditions. This was the temperature of the incubation during the 30-minute read. The pH of the substrate was 8.3. The order of addition of reagents/enzyme was as follows: 10µL of 10% DMSO, 80 µL of substrate and 10 µL of enzyme. Each experiment was run three times. Data is an average of these experiments.

Substrate specificity was determined by testing the activity of HL and EL with Thio Phosphatidylethylene glycol (PEG) and Thio-phosphatidylethanolamine (PE).

Assay conditions of assay were as follows. Both substrates for EL were run at 0.03 mol fraction, 10mM total lipid. They were dissolved in 100mM Hepes with 9.95mM TX100. Both substrates for HL were run at 0.06 mol fraction and 7.25 mM total lipid. They were dissolved in 100mM Hepes with 6.83mM Triton X100. The EL enzyme was used at 1x and the HL enzyme was used at 0.25x. The order of addition was as follows: 10µL of 10% DMSO, 80 µL of substrate and 10 µL of enzyme. The DMSO and substrate were incubated for 10 minutes at 37°C before the addition of the enzyme. DTNB was added to the substrate prior to addition to the well at 0.096 mg/mL final plate concentration. The experiments were performed 3 times. Data represents an average of these.

Several compounds were tested with HL and EL to determine their ability to inhibit these enzymes. Conditions of the assay were as mentioned in the previous paragraph except for the order of addition. In these experiments, the order of addition was 10µL of drug/control, 10 µL of enzyme followed by a 10 minute incubation at 37 °C. This was followed by the addition of the 80 µL of Thio PEG substrate containing DTNB. Each experiment was performed 3 times. Data represents an average.

### Results for Endothelial Lipase Inhibition

| Compounds | IC₅₀ (nM) |
|---|---|
| 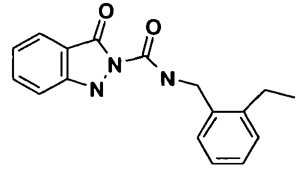 | 11.39 ± 5.17 |
| 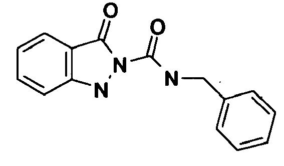 | 73.83 ± 26.2 |
| 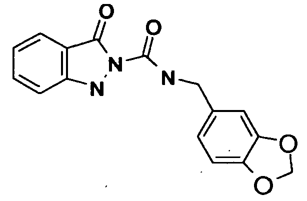 | 45.14 ± 27.53 |
| 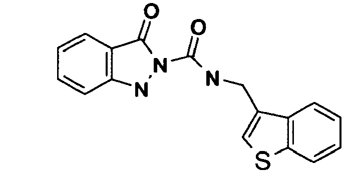 | 13.20 ± 2.83 |

### Experimental

All of the products of the Examples described below as well as intermediates used in the following procedures showed satisfactory NMR and IR spectra. They also had the correct mass spectral values.

### Reference Example 1

### 3-Oxo-1,3-dihydro-indazole-2-carboxylic acid propylamide

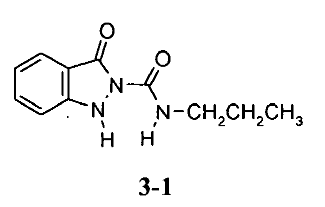

Propyl isocyanate (88.1 µL, 0.940 mmol) is added dropwise to a stirred suspension of 1,2-dihydro-indazol-3-one (105 mg, 0.783 mmol) in anhydrous THF (3 mL) at ambient temperature under nitrogen. The resultant clear solution turns into a heavy suspension in a minute and the suspension is allowed to stir for 2 hours. After concentration and subsequent chromatography on silica (gradient 0-0.5% CH₃OH in CH₂Cl₂), the title compound **3-1** is obtained as a white solid (156 mg, 91% yield). mp 153.0-155.0 °C; ESIMS *m*/*e* 220 (M+H)⁺. Analysis for C₁₁H₁₃N₃O₂: calcd: C, 60.26; H, 5.98; N, 19.17; found: C, 60.13; H, 5.91; N, 19.01.

### Reference Example 2

### 3-Oxo-1,3-dihydro-indazole-2-carboxylic acid pentylamide

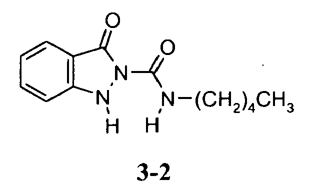

By following similar procedure as described in Example 1, the title compound **3-2** (92% yield) is obtained as a white solid. mp 119.0-120.0 °C; ESIMS *m*/*e* 248 (M+H)⁺. Analysis for C₁₃H₁₇N₃O₂: calcd: C, 63.14; H, 6.93; N, 16.99; found: C, 62.96; H, 6.84; N, 16.91.

### Reference Example 3

### 3-Oxo-1,3-dihydro-indazole-2-carboxylic acid hexylamide

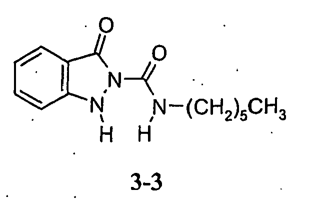

By following similar procedure as described in Example 1, the title compound **3-3** (93% yield) is obtained as a white solid. ESIMS *m*/*e* 262 (M+H)⁺. Analysis for C₁₄H₁₉N₃O₂: calcd: C, 64.35; H, 7.33; N, 16.08; found: C, 64.06; H, 7.43; N, 15.84.

### Example 4

### 3-Oxo-1,3-dihydro-indazole-2-carboxylic acid (2-ethyl-hexyl)-amide

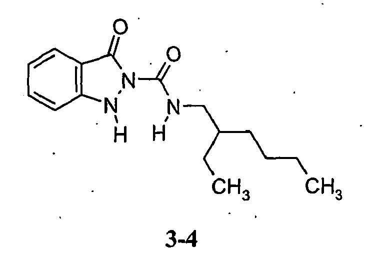

### A. The preparation of 3-oxo-1,3-dihydro-indazole-2-carboxylic acid 4-nitro-phenyl ester

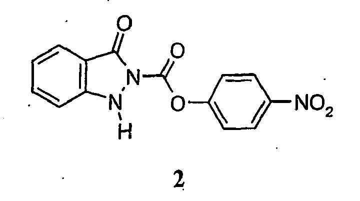

A solution of 4-nitrophenyl chloroformate (3.17 g, 15.7 mmol) in anhydrous THF (15 mL) is added to a stirred suspension of 3-oxo-1,3-dihydro-indazole (2.01 g, 15.0 mmol) in anhydrous THF (30 mL) at ambient temperature under nitrogen. The resultant mixture is stirred for two hours. Then half of THF is evaporated off at ambient temperature under vacuum. The concentrated suspension is treated with Et₂O (25 mL), sonicated and filtered to give the title compound **2** as a white solid (3.26 g, 73% yield). ¹H-NMR (DMSO-d₆) δ7.18 (t, *J* = 7.8 Hz, 1H), 7.30 (d, *J* = 7.8 Hz, 1H), 7.66 (d, *J* = 8.8
Hz, 2H), 7.67 (t, *J* = 7.8 Hz, 1H), 7.77 (d, *J* = 7.8 Hz, 1H), 8.36 (d, *J =* 8.8 Hz, 2H), 10.87 (s, 1H).

### B. The preparation of 3-oxo-1,3-dihydro-indazole-2-carboxylic acid (2-ethylhexyl)-amide

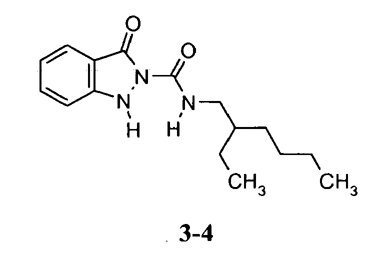

2-Ethylhexyl amine hydrobromide (211 mg, 1.00 mmol) is added to a stirred suspension of 3-oxo-1,3-dihydro-indazole-2-carboxylic acid 4-nitro-phenyl ester **2** (300 mg, 1.00 mmol) in anhydrous THF (10 mL) at ambient temperature under nitrogen. Then triethyl amine (0.140 mL, 1.00 mmol) is added to the mixture and the resultant mixture is allowed to stir for 18 hours. The mixture is concentrated and the crude product is chromatographed on silica (gradient 0-1% EtOAc in CHCl₃) to give the title compound **3-4** (227 mg, 78% yield) as a white solid. ESIMS *m*/*e* 290 (M+H)⁺.

### Reference Example 5

### 3-Oxo-1,3-dihydro-indazole-2-carboxylic acid (5-methyl-hexyl)-amide

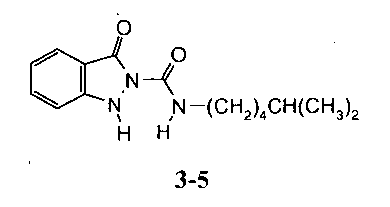

### A. Preparation of (1-isocyanato-5-methyl)-hexane

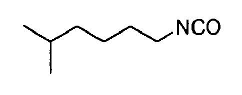

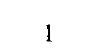

A solution of 5-(methyl)hexylamine (407 mg, 3.53 mmol) and proton sponge (1.51 g, 7.06 mmol) in anhydrous CH₂Cl₂ (6 mL) is added dropwise to a stirred solution of triphosgene (419 mg, 1.41 mmol) in anhydrous CH₂Cl₂ (6 mL) at 0 °C. The resultant solution is allowed to stir at ambient temperature for 15 minutes. After dilution with CH₂Cl₂ (40 mL), the mixture is washed with 1N HCl (15x2 mL) and water (15 mL). The organic layer is dried over Na₂SO₄, filtered and concentrated to give the desired isocyanate **1** (365 mg, 73% yield) as oil. ¹H-NMR (CDCl₃) δ 0.88 (d, *J* = 6.6 Hz, 6H), 1.16-1.23 (m, 2H), 1.32-1.42 (m, 2H), 1.50-1.62 (m, 3H), 3.29 (t, *J* = 6.6 Hz, 2H).

### B. Preparation of 3-oxo-1,3-dihydro-indazole-2-carboxylic acid (5-methyl-hexyl)-amide

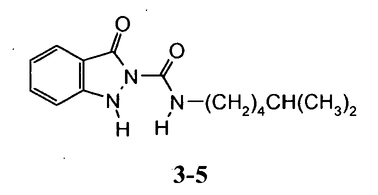

By following similar procedure as described in Example 1, the title compound **3-5** is obtained as a white solid. mp 134.0-136.0 °C; ESIMS *m*/*e* 276 (M+H)⁺.

### Reference Example 6

### 3-Oxo-1,3-dihydro-indazole-2-carboxylic acid octylamide

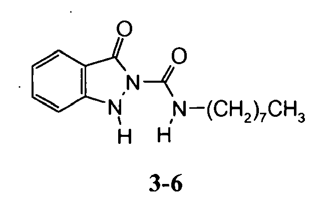

By following similar procedure as described in Example 1, the title compound **3-6** (99% yield) is obtained as a white solid. ESIMS *m*/*e* 290 (M+H)⁺. Analysis for C₁₆H₂₃N₃O₂: calcd: C, 66.41; H, 8.01; N, 14.52; found: C, 66.25; H, 7.90; N, 14.75.

### Example 7

### 3-Oxo-1,3-dihydro-indazole-2-carboxylic acid (3,7-dimethyl-octa-2,6-dienyl)-amide

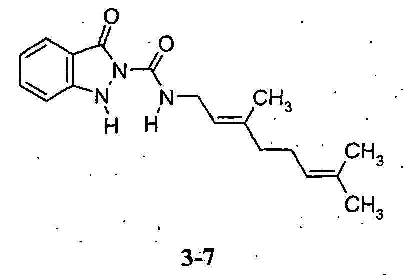

By following similar procedure as described in Example 4, the title compound **3-7** (45% yield) is obtained as a white solid. ESIMS *m*/*e* 314 (M+H)⁺. Analysis for C₁₈H₂₃N₃O₂: calcd: C, 68.98; H, 7.40; N, 13.41; found: C, 68.99; H, 7.30; N, 13.42.

### Example 8

### 3-Oxo-1,3-dihydro-indazole-2-carboxylic acid cyclohexylmethyl-amide

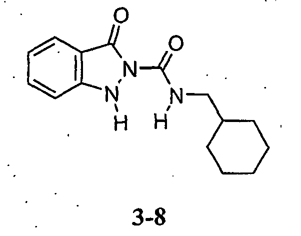

By following similar procedure as described in Example 4, the title compound **3-8** (71% yield) is obtained as a white solid. ESIMS *m*/*e* 274 (M+H)⁺. Analysis for C₁₅H₁₉N₃O₂: calcd: C, 65.91; H, 7.01; N, 15.37; found: C, 65.88; H, 6.96; N, 15.05.

### Example 9

### 1-Methyl-3-oxo-1,3-dihydro-indazole-2-carboxylic acid pentylamide

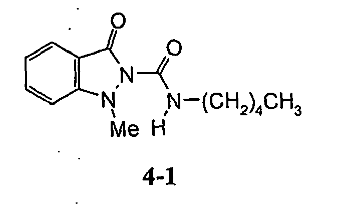

Methyl iodide (0.426 mL, 7.15 mmol) is added to a stirred suspension of 3-oxo-1,3-dihydro-indazole-2-carboxylic acid pentylamide **3-2** (70.9 mg, 0.287 mmol) and anhydrous K₂CO₃ (39.6 mg, 0.287 mmol) in anhydrous THF (3 mL) at ambient temperature under nitrogen. The resultant mixture is stirred for 24 hours. Ethyl acetate (30 mL) and half-saturated aquous NaCl solution (10 mL) are added to the mixture. The organic layer is separated, dried over MgSO4, filtered and concentrated. The crude product is chromatographed on silica (gradient 0-3% CH₃OH in CH₂Cl₂) to give the title compound **4-1** as oil (55.0 mg, 73% yield). ESIMS *m*/*e* 262 (M+H)⁺. Analysis for C₁₄H₁₉N₃O₂: calcd: C, 64.35; H, 7.33; N, 16.08; found: C, 64.17; H, 7.33; N, 16.04.

### Example 10

### 1-Butyl-3-oxo-1,3-dihydro-indazole-2-carboxylic acid pentylamide

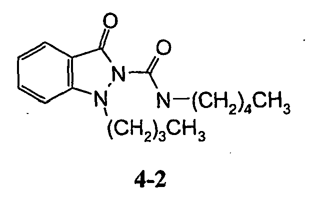

By following similar procedure as described in Example 8, the title compound **4-2** is obtained as oil. ESIMS *m*/*e* 304 (M+H)⁺. Analysis for C₁₇H₂₅N₃O₂: calcd: C, 67.30; H, 8.31; N, 13.85; found: C, 67.30; H, 8.40; N, 13.98.

### Example 11

### 1-Benzyl-3-oxo-1,3-dihydro-indazole-2-carboxylic acid pentylamide

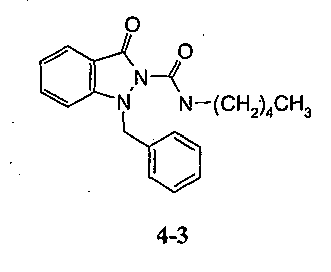

By following similar procedure as described in Example 8, the title compound **4-3** is obtained as oil (90% yield). ESIMS *m*/*e* 338 (M+H)⁺. Analysis for C₂₀H₂₃N₃O₂: calcd: C, 71.19; H, 6.87; N, 12.45; found: C, 71.30; H, 6.79; N, 12.59.

### Example 12

### 3-Oxo-1-phenethyl-1,3-dihydro-indazole-2-carboxylic acid pentylamide

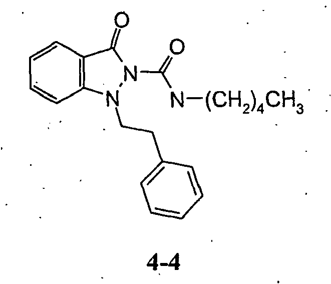

By following similar procedure as described in Example 8, the title compound **4-4** is obtained as oil. ESIMS *m*/*e* 352 (M+H)⁺.

### Example 13

### 1-(2-Methoxy-ethyl)-3-oxo-1,3-dihydro-indazole-2-carboxylic acid hexylamide

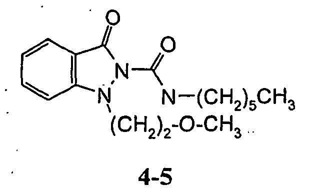

By following similar procedure as described in Example 8, the title compound **4-5** is obtained as oil. FDMS *m*/*e* 319 (M)⁺.

### Example 14

### (2-Hexylcarbamoyl-3-oxo-2,3-dihydro-indazol-1-yl)-acetic acid methyl ester

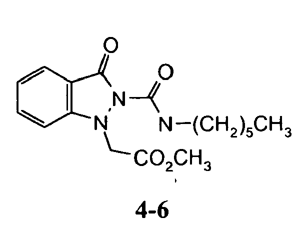

Methyl bromoacetate (0.724 mL, 7.65 mmol) is added to a stirred suspension of 3-oxo-1,3-dihydro-indazole-2-carboxylic acid hexylamide **3-3** (200 mg, 0.765 mmol) and anhydrous K₂CO₃ (159 mg, 1.15 mmol) in anhydrous THF (3 mL) at ambient temperature under nitrogen. The resultant mixture is stirred for 24 hours. Ethyl acetate (30 mL) and half-saturated aqueous NaCl solution (10 mL) are added to the mixture. The organic layer is separated, dried over MgSO4, filtered and concentrated. The crude product is chromatographed on silica (gradient 0-0.25% CH₃OH in CH₂Cl₂) to give the title compound **4-6** as oil (188 mg, 74% yield). ESIMS *m*/*e* 334 (M+H)⁺. Analysis for C₁₇H₂₃N₃O₄: calcd: C, 61.25; H, 6.95; N, 12.60; found: C, 61.12; H, 6.93; N, 12.64.

### Reference Example 15

### 3-Oxo-1,3-dihydro-indazole-2-carboxylic acid benzylamide

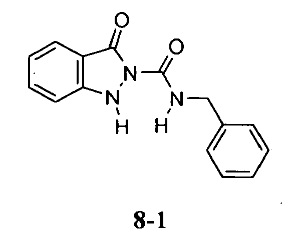

By following similar procedure as described in Example 1, the title compound **8-1** (91% yield) is obtained as a white solid. ESIMS *m*/*e* 268 (M+H)⁺. Analysis for C₁₅H₁₃N₃O₂: calcd: C, 67.41; H, 4.90; N, 15.72; found: C, 67.18; H, 5.05; N, 15.62.

### Example 16

### 3-Oxo-1,3-dihydro-indazole-2-carboxylic acid 2-methyl-benzylamide

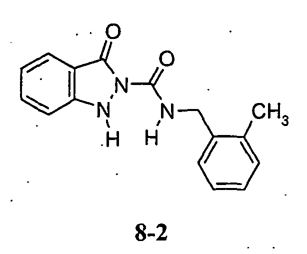

By following similar procedure as described in Example 1, the title compound **8-2** (97% yield) is obtained as a white solid. mp 166.0-168.0 °C; ESIMS *m*/*e* 282 (M+H)⁺. Analysis for C₁₆H₁₅N₃O₂: calcd: C, 68.31; H, 5.37; N, 14.94; found: C, 67.96; H, 5.22; N, 14.77.

### Example 17

### 3-Oxo-1,3-dihydro-indazole-2-carboxylic acid 3-methyl-benzylamide

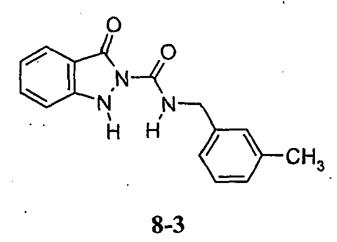

By following similar procedure as described in Example 1, the title compound **8-3** (89% yield) is obtained as a white solid. mp 179.0-180.0 °C; ESIMS *m*/*e* 282 (M+H)⁺. Analysis for C₁₆H₁₅N₃O₂: calcd: C, 68.31; H, 5.37; N, 14.94; found: C, 68.36; H, 5.48; N, 14.85.

### Example 18

### 3-Oxo-1,3-dihydro-indazole-2-carboxylic acid 4-methyl-benzylamide

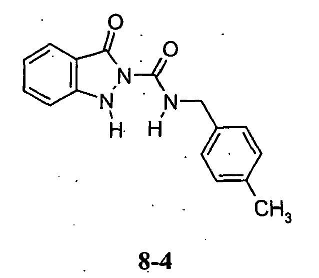

4-Methylbenzyl amine (125 mg, 1.03 mmol) is added dropwise to a stirred suspension of 3-oxo-1,3-dihydro-indazole-2-carboxylic acid 4-nitro-phenyl ester **2** (308 mg, 1.03 mmol) in anhydrous THF (10 mL) at ambient temperature under nitrogen. The resultant mixture is allowed to stir for 18 hours. After concentration, the crude product is chromatographed on silica (gradient 0-0.5% CH₃OH in CH₂Cl₂) to give the title compound **8-4** (210 mg, 72% yield) as a white solid. ESIMS *m*/*e* 282 (M+H)⁺. Analysis for C₁₆H₁₅N₃O₂: calcd: C, 68.31; H, 5.37; N, 14.94; found: C, 68.04; H, 5.38; N, 14.86.

### Example 19

### 3-Oxo-1,3-dihydro-indazole-2-carboxylic acid 2-trifluoromethyl-benzylamide

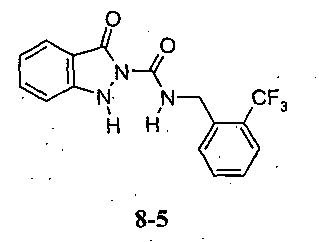

By following similar procedure as described in Example 18, the title compound **8-5** is obtained as a white solid. ESIMS *m*/*e* 336 (M+H⁺).

### Example 20

### 3-Oxo-1,3-dihydro-indazole-2-carboxylic acid 2-ethyl-benzylamide

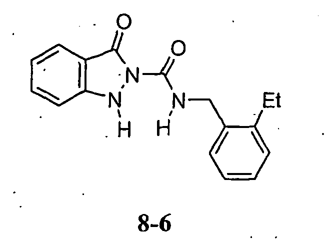

### A. Preparation of 2-ethyl-benzylamine

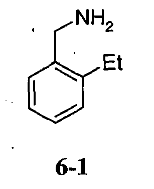

To the ice-cold 2-ethyl-benzonitrile **5-1** (2.01 g, 15.3 mmol) in anhydrous THF (20 mL) is slowly added lithium aluminum hydride (IN in THF, 19.2 mL) under nitrogen. The resultant mixture is allowed to stir at ambient temperature for 16 hr. The reaction mixture is cooled at 0 °C and treated dropwise with MeOH (6 mL), then EtOAc (60 mL) and saturated aqueous Rochelle's salt (60 mL) and water (40 mL) are added to the mixture .The resultant two-layered mixture is stirred vigorously under nitrogen for 1 hour. The organic layer is separated, dried over MgSO₄, filtered and concentrated to give the title amine **6-1** (2.01 g, crude yield 98%) as light oil. ¹H-NMR (CDCl₃) δ1.24 (t, *J* = 7.4 Hz, 3H), 1.54 (br s, 2H), 2.69 (q, *J* = 7.4 Hz, 2H), 3.89 (s, 2H), 7.19-7.24 (m, 3H), 7.31-7:35 (m, 1H); ESIMS *mle* 136 (M+H)⁺.

### B. Preparation of 3-oxo-1,3-dihydro-indazole-2-carboxylic acid 2-ethyl-benzylamide

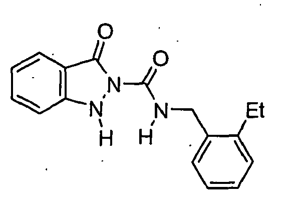

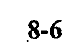

By following similar procedure as described in Example 18, the title compound **8-6** is obtained as a white solid. ESIMS *m*/*e* 294 (M-H)⁻. Analysis for C₁₇H₁₇N₃O₂: calcd: C, 69.14; H, 5.80; N, 14.23; found: C, 68.86; H, 5.70; N, 14.25.

### Example 21

### 3-Oxo-1,3-dihydro-indazole-2-carboxylic acid 3-ethyl-benzylamide

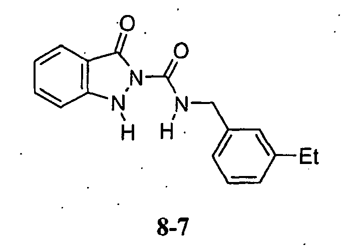

By following similar procedure as described in Example 20, the title compound **8-7** is obtained as a white solid. ESIMS *m*/*e* 296 (M+H)⁺. Analysis for C₁₇H₁₇N₃O₂: calcd: C, 69.14; H, 5.80; N, 14.23; found: C, 68.99; H, 5.82; N, 14.21.

### Example 22

### 3-Oxo-1,3-dihydro-indazole-2-carboxylic acid 3-fluoro-benzylamide

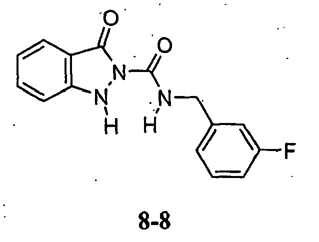

By following similar procedure as described in Example 18, the title compound **8-8** is obtained as a white solid. ESIMS *m*/*e* 286 (M+H)⁺.

### Example 23

### 3-Oxo-1,3-dihydro-indazole-2-carboxylic acid 3-chloro-benzylamide

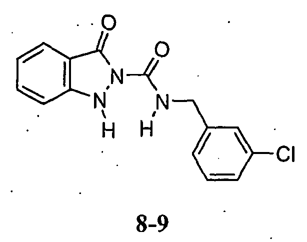

By following similar procedure as described in Example 18, the title compound **8-9** is obtained as a white solid. ESIMS *m*/*e* 302 (M+H, ³⁵Cl)⁺. Analysis for C₁₅H₁₂ClN₃O₂: calcd: C, 59.71; H, 4.01; N, 13.93; found: C, 59.40; H, 3.83; N, 13.75.

### Example 24

### 3-Oxo-1,3-dihydro-indazole-2-carboxylic acid 3-methoxy-benzylamide

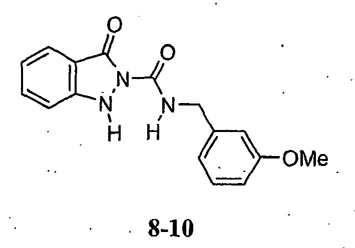

By following similar procedure as described in Example 18, the title compound **8-10** is obtained as a white solid. mp 186.0-188.0 °C; ESIMS *m*/*e* 298 (M+H)⁺. Analysis for C₁₆H₁₅N₃O₃: calcd: C, 64.64; H, 5.09; N, 14.13; found: C, 64.35; H, 4.90; N, 14.13.

### Example 25

### 3-Oxo-1,3-dihydro-indazole-2-carboxylic acid 3-trifluoromethoxy-benzylamide

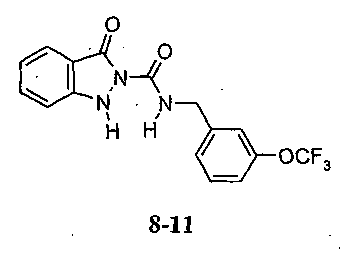

By following similar procedure as described in Example 18, the title compound **8-11** is obtained as a white solid. ESIMS *m*/*e* 350 (M-H)⁻. Analysis for C₁₆H₁₂F₃N₃O₃: calcd: C, 54.71; H, 3.44; N, 11.96; found: C, 55.00; H, 3.51; N, 11.88.

### Example 26

### 3-Oxo-1,3-dihydro-indazole-2-carboxylic acid 2-methylsulfanyl-benzylamide

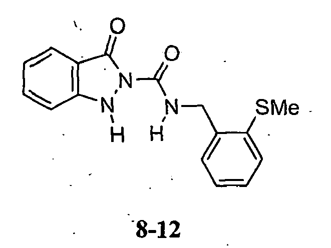

By following similar procedure as described in Example 18, the title compound **8-12** is.obtained as a white solid. ESIMS *m*/*e* 314 (M+H)⁺. Analysis for C₁₆H₁₅N₃O₂S: calcd: C, 61.32; H, 4.82; N, 13.41; found: C, 61.02; H, 4.75; N, 13.31.

### Example 27

### 3-Oxo-1,3-dihydro-indazole-2-carboxylic acid 4-amino-benzylamide

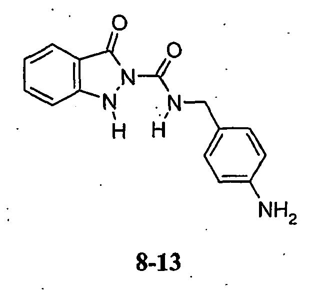

By following similar procedure as described in Example 18, the title compound **8-13** is obtained as a white solid. ESIMS *m*/*e* 281 (M-H)⁻. Analysis for C₁₅H₁₄N₄O₂: calcd: C, 63.82; H, 5.00; N, 19.85; found: C, 63.47; H, 5.02; N, 19.58.

### Example 28

### 3-Oxo-1,3-dihydro-indazole-2-carboxylic acid 4-dimethylamino-benzylamide

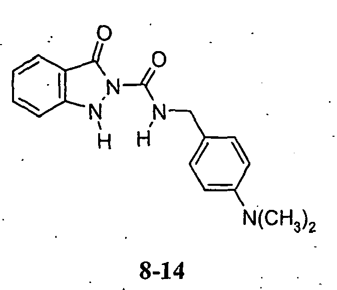

By following similar procedure as described in Example 18, the title compound **8-14** is obtained as a white solid. ESIMS *m*/*e* 309 (M-H)⁻. Analysis for C₁₇H₁₈N₄O₂: calcd: C, 65.79; H, 5.85; N, 18.05; found: C, 65.50; H, 5.85; N, 18.01.

### Example 29

### 3-Oxo-1,3-dihydro-indazole-2-carboxylic acid (biphenyl-3-ylmethyl)-amide

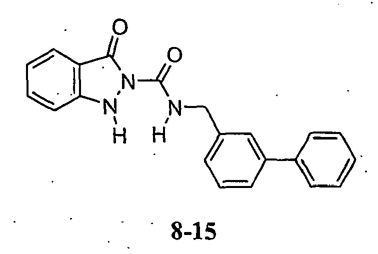

By following similar procedure as described in Example 18, the title compound **8-15** is obtained as a white solid. ESIMS *m*/*e* 342 (M-H)⁻. Analysis for C₂₁H₁₇N₃O₂: calcd: C, 73.45; H, 4.99; N, 12.24; found: C, 73.07; H, 4.82; N, 12.09.

### Example 30

### 3-Oxo-1,3-dihydro-indazole-2-carboxylic acid (naphthalen-1-ylmethyl)-amide

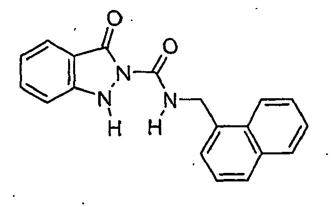

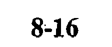

By following similar procedure as described in Example 18, title compound **8-16** is obtained as a white solid. ESIMS *m*/*e* 316 (M-H)⁻.

### Example 31

### 3-Oxo-1,3-dihydro-indazole-2-carboxylic acid (4-methyl-naphthalen-1-ylmethyl)-amide

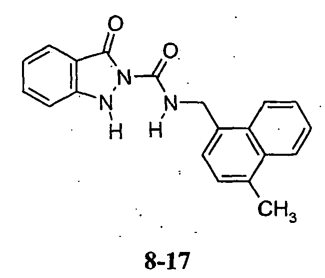

By following similar procedure as described in Example 18, the title compound **8-17** is obtained as a white solid. ESIMS *m*/*e* 330 (M-H)⁻. Analysis for C₂₀H₁₇N₃O₂: calcd: C, 72.49; H, 5.17; N, 12.68; found: C, 72.23; H, 5.10; N, 12.33.

### Example 32

### 3-Oxo-1,3-dihydro-indazole-2-carboxylic acid 5-chloro-2-methyl-benzylamide

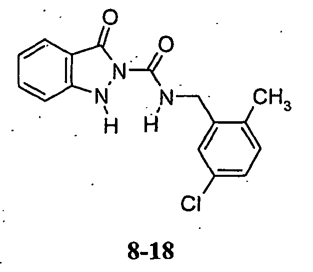

By following similar procedure as described in Example 20, the title compound **8-18** is obtained as a white solid. ESIMS *m*/*e* 316 [(M+H)⁺, ³⁵Cl], 318 [(M+H)⁺, ³⁷Cl]. Analysis for C₁₆H₁₄ClN₃O₂: calcd: C, 60.86; H, 4.47; N, 13.31; found: C, 60.73; H, 4.49; N,13.21.

### Example 33

### 3-Oxo-1,3-dihydro-indazole-2-carboxylic acid 2-isopropyl-6-methyl-benzylamide

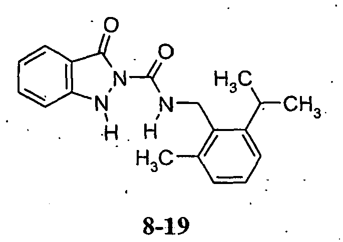

### A. Preparation of 2-isopropyl-6-methyl-benzonitrile

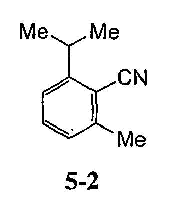

CuCN (7.80 g, 87.2 mmol) is added to a stirred anhydrous DMSO (70 mL) at 60 °C to form a clear solution, and then followed by the addition of *t*-BuNO₂ (24.0 mL, 202 mmol) all at once. A solution of 2-isopropyl-6-methylaniline (10.0 g, 67.0 mmol) in anhydrous DMSO (30 mL) is added dropwise, via an addition funnel, to the mixture. After the addition is complete, the reaction mixture is allowed to stir for 1 hour. After being cooled to 45 °C, the mixture is slowly treated with 5N HCl (100 mL). Five minutes later, the reaction mixture is cooled to ambient temperature before it is extracted with EtOAc/hexane (1:1; 500x2 mL). The combined organic layers are washed with water (100 mL) and brine (100 mL), dried, concentrated *in vacuo*, then chromatographed on silica (0-5% EtOAc in hexane) to give 8.43 g of the crude nitrile **5-2**. IR(CHCl₃) 2220 cm⁻¹; ¹H-NMR (CDCl₃) δ1.30 (d, *J* = 6.9 Hz, 6H), 2.54 (s, 3H), 3.38 (h, *J* = 6.9 Hz, 1H), 7.13 (d, *J* = 7.8 Hz, 1H), 7.20 (d, *J* = 7.8 Hz, 1H), 7.41 (t, *J* = 7.8 Hz, 1H); ESIMS *m*/*e* 160 (M+H)⁺.

### B. Preparation of 2-isopropyl-6-methyl-benzylamine

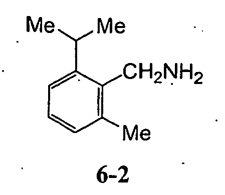

To the crude ice-cold nitrile **5-2** (7.74 g, 48.6 mmol) in anhydrous Et₂O (70 mL) is slowly added lithium aluminum hydride (IN in Et₂O, 97.2 mL) under nitrogen. The resultant mixture is allowed to stir at ambient temperature for 16 hours. Then the reaction mixture is cooled at 0 °C and quenched with MeOH until the gas evolution stops. EtOAc (500 mL) and saturated aqueous Rochelle's salt are added and the two-layered mixture is stirred vigorously under nitrogen for 1 hour to give two relatively clear layers. The organic layer is separated, dried over MgSO₄, filtered and concentrated, the crude oil is chromatographed on silica [20% EtOAc in hexane, then 1-2% (4.2 M Me₃N in EtOH) in CHCl₃]. Amine **6-2** (3.78 g, yield 48%) is obtained as a brown oil. IR(CHCl₃) 3300(br) cm⁻¹; ¹H-NMR (CDCl₃) δ 1.16 (d, *J* = 6.8 Hz, 6H), 1.55 (br s, 2H), 2.33 (s, 3H), 3.28 (h, *J* = 6.8 Hz, 1H), 3.71 (s, 2H), 6.92-6.95 (m, 1H), 7.03-7.10 (m, 2H); ESIMS *m*/*e* 164 (M+H)⁺.

### C. Preparation of 3-oxo-1,3-dihydro-indazole-2-carboxylic acid 2-isopropyl-6-methyl-benzylamide

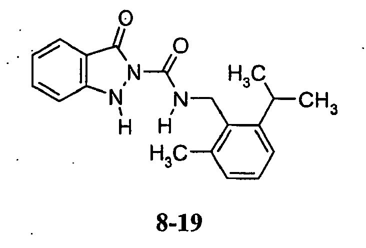

By following similar procedure as described in Example 18, the title compound **8-19** is obtained as a white solid (64% yield). ESIMS *m*/*e* 324 (M+H)⁺. Analysis for C₁₉H₂₁N₃O₂: calcd: C, 70.57; H, 6.55; N, 12.99; found: C, 70.72; H, 6.55; N, 12.90.

### Example 34

### 3-Oxo-1,3-dihydro-indazole-2-carboxylic acid 2,4-dichloro-benzylamide

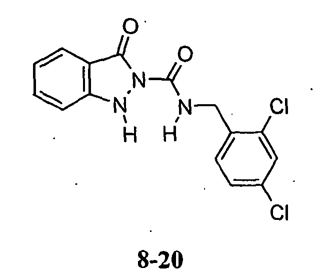

By following similar procedure as described in Example 18, the title compound **8-20** is obtained as a white solid. ESIMS *m*/*e* 336 [(M+H)⁺, ³⁵Cl, ³⁵Cl], 338 [(M+H)⁺, ³⁵Cl, ³⁷Cl], 340 [(M+H)⁺; ³⁷Cl, ³⁷Cl].

### Example 35

### 3-Oxo-1,3-dihydro-indazole-2-carboxylic acid 5-chloro-2-methoxy-benzylamide

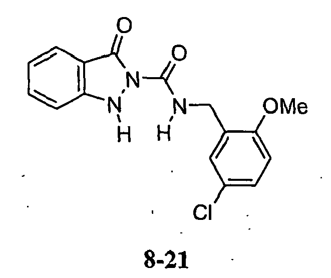

By following similar procedure as described in Example 20, the title compound **8-21** is obtained as a white solid. ESIMS *m*/*e* 332 [(M+H)⁺, ³⁵Cl], 334 [(M+H)⁺, ³⁷Cl]. Analysis for C₁₆H₁₄ClN₃O₃: calcd: C, 57.93; H, 4.25; N, 12.67; found: C, 58.29; H, 4.05; N, 12.52.

### Example 36

### 3-Oxo-1,3-dihydro-indazole-2-carboxylic acid 2,4-dimethoxy-benzylamide

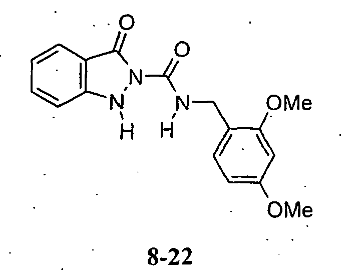

By following similar procedure as described in Example 18, the title compound **8-22** is obtained as a white solid (75% yield). ESIMS nile 326 (M-H)⁻. Analysis for C₁₇H₁₇N₃O₄: calcd: C, 62.38; H, 5.23; N, 12.84; found: C, 62.64; H, 5.20; N, 12.86.

### Example 37

### 3-Oxo-1,3-dihydro-indazole-2-carboxylic acid 3,4-dihydroxy-benzylamide

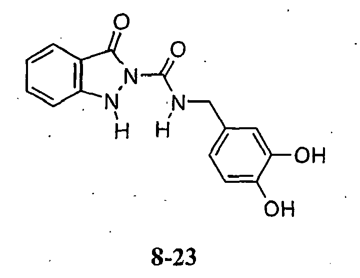

By following similar procedure as described in Example 18, the title compound **8-23** is obtained as a white solid. ESIMS *m*/*e* 300 (M+H)⁺.

### Example 38

### 3-Oxo-1,3-dihydro-indazole-2-carboxylic acid (2,3-dihydro-benzo[1,4]dioxin-6-ylmethyl)-amide

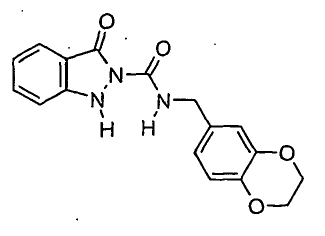

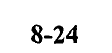

By following similar procedure as described in Example 18, the title compound **8-24** is obtained as a white solid (73% yield). ESIMS *m*/*e* 326 (M+H)⁺. Analysis for C₁₇H₁₅N₃O₄: calcd: C, 62.76; H, 4.65; N, 12.92; found: C, 62.79; H, 4.66; N, 12.86.

### Example 39

### 3-Oxo-1,3-dihydro-indazole-2-carboxylic acid 2,4,6-trimethyl-benzylamide

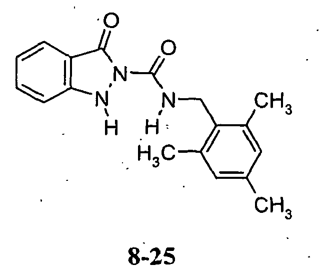

By following similar procedure as described in Example 20, the title compound **8-25** is obtained as a white solid. ESIMS *m*/*e* 310 (M+H)⁺. Analysis for C₁₈H₁₉N₃O₂: calcd: C, 69.88; H, 6.19; N, 13.58; found: C, 69.67; H, 6.13; N, 13.53.

### Example 40

### 3-Oxo-1,3-dihydro-indazole-2-carlioxylic acid 4,6-dichloro-2-methyl-benzylamide

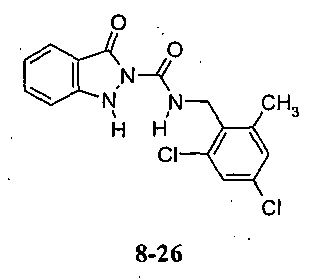

By following similar procedure as described in Example 18, the title compound **8-26** is obtained as a white solid. FDMS *m*/*e* 349 (M⁺, ³⁵Cl, ³⁵Cl), 351 (M⁺, ³⁵Cl, ³⁷Cl), 353 (M⁺, ³⁷Cl, ³⁷Cl). Analysis for C₁₆H₁₃Cl₂N₃O₂: calcd: C, 54.88; H, 3.74; N, 12.00; found: C, 54.50; H, 3.75; N, 11.72.

### Example 41

### 3-Oxo-1,3-dihydro-indazole-2-carboxylic acid 3,4,5-trimethoxy-benzylamide

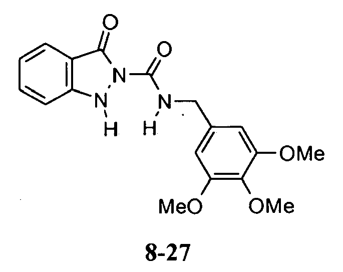

By following similar procedure as described in Example 18, the title compound **8-27** is obtained as a white solid (78% yield). ESIMS *m*/*e* 356 (M-H)⁻. Analysis for C₁₈H₁₉N₃O₅: calcd: C, 60.50; H, 5.36; N, 11.76; found: C, 60.48; H, 5.35; N, 11.73.

### Reference Example 42

### 3-Oxo-1,3-dihydro-indazole-2-carboxylic acid (4-phenyl-butyl)-amide

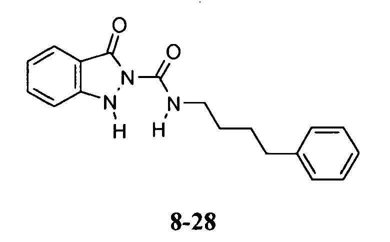

By following similar procedure as described in Example 18, the title compound **8-28** is obtained as a white solid (76% yield). ESIMS *m*/*e* 310 (M+H)⁺. Analysis for C₁₈H₁₉N₃O₂: calcd: C, 69.88; H, 6.19; N, 13.58; found: C, 69.93; H, 6.11; N, 13.58.

### Example 43

### 33-Oxo-1,3-dihydro-indazole-2-carboxylic acid (4-phenyl-but-3-enyl)-amide

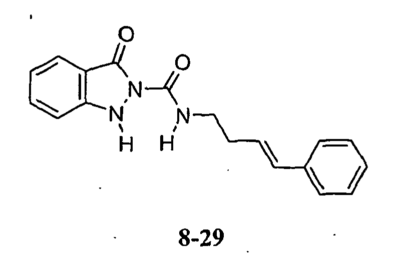

By following similar procedure as described in Example 18, the title compound **8-29** is obtained as a white solid (56% yield). ESIMS *m*/*e* 308 (M+H)⁺.

### Example 44

### 4-Methyl-3-oxo-1,3-dihydro-indazole-2-carboxylic acid 2-methyl-benzylamide

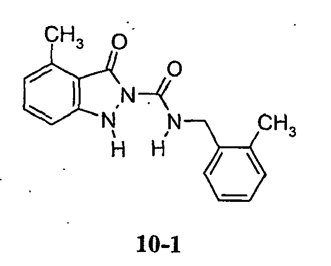

### A. Preparation of 4-methyl-1,2-dihydro-indazol-3-one

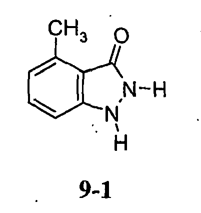

Concentrated hydrochloric acid (103 mL) is added slowly, in a period of 10 minutes, to a stirred suspension of 2-amino-6-methyl-benzoic acid (5.00 g, 33.1 mmol) in water (56 mL) at -25 °C, then a solution of sodium nitrite (2.29 g, 33.1 mmol) in water (28 mL) is added dropwise to the cold suspension. The clear solution is allowed to stir for 40 min at -25 °C before it is added in small portions to a stirred solution of Tin(II) chloride (37.6 g, 198 mmol) in concentrated hydrochloric acid (84 mL) at -35 °C. The resultant mixture is stirred for 30 minutes at -30 °C, then allowed to warm to ambient temperature where it is stirred for another 16 hours. The mixture is filtered and the white solid is washed with cold THF (200 mL) and vacuum dried to give the title compound **9-1** (2.68 g, yield 55%) as a white solid. FDMS *mle* 148 (M)⁺.

### B. Preparation of 4-methyl-3-oxo-1,3-dihydro-indazole-2-carboxylic acid 2-methyl-benzylamide

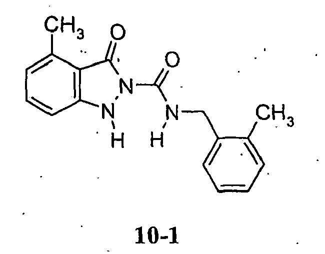

1-Isocyanatomethyl-2-methyl-benzene (0.191 mL, 1.30 mmol) is added dropwise to a stirred suspension of 4-methyl-1,2-dihydro-indazol-3-one 9-1 (200 mg, 1.35 mmol) in anhydrous THF (10 mL) at ambient temperature under nitrogen, and the resultant mixture is allowed to stir for 1 hour. After concentration and subsequent chromatography on silica (gradient 0-0.5% CH₃OH in CH₂Cl₂), the title compound **10-1** (119 mg, 31% yield) is obtained as a white solid. ESIMS *m*/*e* 296 (M+H)⁺.

### Example 45

### 6-Methyl-3-oxo-1,3-dihydro-indazole-2-carboxylic acid 2-methyl-benzylamide

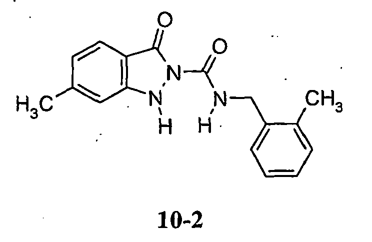

### A. Preparation of 6-methyl-1,2-dihydro-indazol-3-one

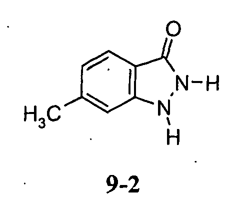

Concentrated hydrochloric acid (42 mL) is added slowly, in a period of 10 minutes, to a stirred suspension of 2-amino-4-methyl-benzoic acid (2.00 g, 13.2 mmol) in water (25 mL) at -25 °C, then a solution of sodium nitrite (0.912 g, 13.2 mmol) in water (11 mL) is added dropwise to the cold suspension. The clear solution is allowed to stir for 40 min at -25 °C before it is added in small portions to a stirred solution of Tin (II) chloride (15.0 g, 79.1 mmol) in concentrated hydrochloric acid (34 mL) at -35 °C. The resultant mixture is stirred for 30 minutes at -30 °C, then allowed to warm to ambient temperature where it is stirred for another 16 hours. The mixture is filtered and the white solid is washed with cold THF and vacuum dried to give a mixture of 2-hydrazino-4-methyl-benzoic acid and the title compound **9-2** (2.56 g). The white solid (2.48 g) is dissolved in methanol (150 mL) and the solution is heated to reflux under nitrogen for 2 days to give 2.33 g of the title compounds **9-2** (100% crude yield).

### B. Preparation of 6-methyl-3-oxo-1,3-dihydro-indazole-2-carboxylic acid 2-methyl-benzylamide

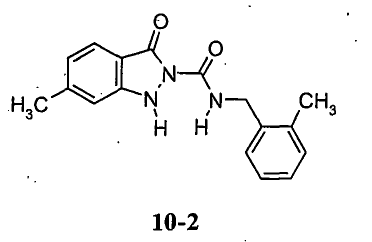

By following similar procedure as described in part B of Example 44, the title compound **10-2** is obtained as a white solid (39% yield). mp 235 °C (dec); ESIMS *m*/*e* 296 (M+H)⁺.

### Example 46

### 4-Chloro-3-oxo-1,3-dihydro-indazole-2-carboxylic acid 2-methyl-benzylamide

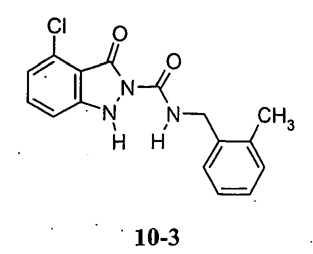

### A. Preparation of 4-chloro-1,2-dihydro-indazol-3-one

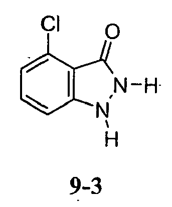

Concentrated hydrochloric acid (55 mL) is added slowly, in a period of 10 minutes, to a stirred suspension of 2-amino-6-chloro-benzoic acid (3.00 g, 17.2 mmol) in water (30 mL) at 0 °C. The resultant mixture is cooled to -30 °C before it is treated dropwise with a solution of sodium nitrite (1.19 g, 17.2 mmol) in water (15 mL). The clear solution is allowed to stir for 40 min at -25 °C before it is added in small portions to a stirred solution of Tin(II) chloride (19.5 g, 103 mmol) in concentrated hydrochloric acid (44 mL) at -35 °C. The resultant mixture is stirred for 30 minutes at -30 °C, then allowed to warm to ambient temperature where it is stirred for another 16 hours. The mixture is filtered and the white solid is washed with a small amount of cold THF and vacuum dried to give 2.82 g of the title compounds **9-2** (97% crude yield). ESIMS *m*/*e* 169 [(M+H)⁺, ³⁵Cl], 171 [(M+H)⁺, ³⁷Cl].

### B. Preparation of 4-chloro-3-oxo-1,3-dihydro-indazole-2-carboxylic acid 2-methyl-benzylamide

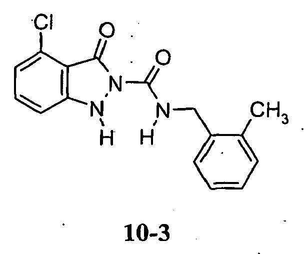

By following similar procedure as described in part B of Example 44, the title compound **10-3** is obtained as a white solid (48% yield). ESIMS *m*/*e* 314 [(M-H)⁻, ³⁵Cl], 316 [(M-H)⁻, ³⁷Cl].

### Example 47

### 5-Chloro-3-oxo-1,3-dihydro-indazole-2-carboxylic acid 2-methyl-benzylamide

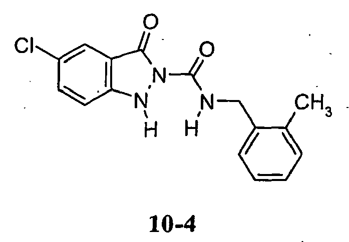

### A. Preparation of 5-chloro-1,2-dihydro-indazol-3-one

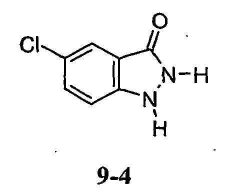

By following similar procedure as described in part A of Example 45, the title compound **9-4** is obtained as a solid (70% crude yield). ¹H-NMR (DMSO-d₆) □ 7.25 (d, *J* = 8.3 Hz, 1H), 7.30 (d, *J* = 8.3 Hz, 1H), 7.65 (s, 1H), 8.69 (br s, 2H).

### B. Preparation of 5-chloro-3-oxo-1,3-dihydro-indazole-2-carboxylic acid 2-methyl-benzylamide

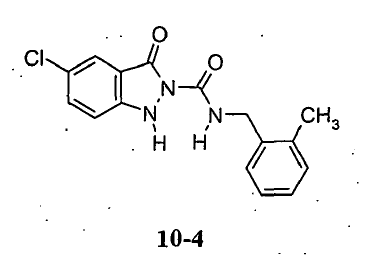

By following similar procedure as described in part B of Example 44, the title compound **10-4** is obtained as a white solid (51% yield). ESIMS *m*/*e* 316 [(M+H)⁺, ³⁵Cl], 318 [(M+H)⁺, ³⁷Cl]. Analysis for C₁₆H₁₄ClN₃O₂: calcd: C, 60.86; H, 4.47;N, 13.31; found: C, 60.74; H, 4.46; N, 13.16.

### Example 48

### 6-Chloro-3-oxo-1,3-dihydro-indazole-2-carboxylic acid 2-methyl-benzylamide

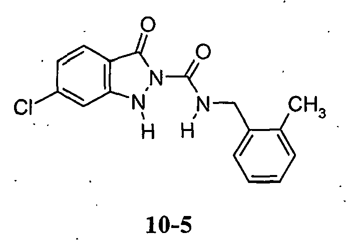

### A. Preparation of 6-chloro-1,2-dihydro-indazol-3-one

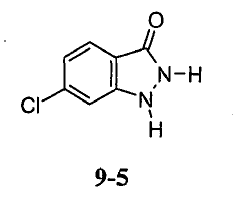

By following similar procedure as described in part A of Example 45, the title compound **9-5** is obtained as a solid (94% crude yield). ¹H-NMR (DMSO-d₆) δ6.95 (d, *J* = 8.3 Hz, 1H), 7.33 (s, 1H), 7.61 (d, *J* = 8.3 Hz, 1H), 8.50 (br s, 2H).

### B. Preparation of 6-chloro-3-oxo-1,3-dihydro-indazole-2-carboxylic acid 2-methyl-benzylamide

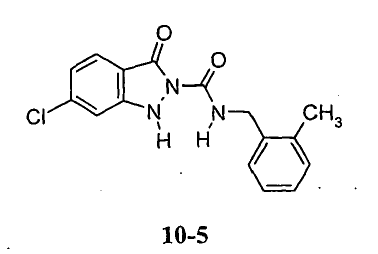

By following similar procedure as described in part B of Example 44, the title compound **10-5** is obtained as a white solid (44% yield). ¹H-NMR (DMSO-d₆) □ 2.31 (s, 3H), 4.52 (d, *J =* 5.3 Hz, 2H), 7.11-7.31 (m, 6H), 7.75 (d, *J* = 8.3 Hz, 1H), 8.98 (t, *J* = 5.3 Hz, 1H), 11.62 (s, 1H).

### Example 49

### 7-Methoxy-3-oxo-1,3-dihydro-indazole-2-carboxylic acid 2-methyl-benzylamide

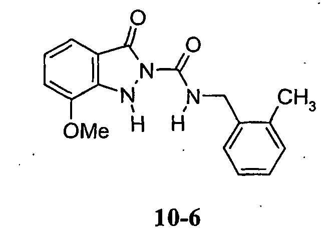

### A. Preparation of 7-methoxy-1,2-dihydro-indazol-3-one

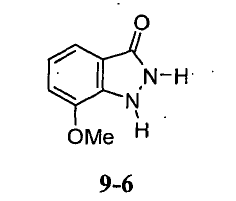

By following similar procedure as described in part A of Example 45, the title compound **9-6** is obtained as a solid. ¹H-NMR (DMSO-d₆) δ3.87 (s, 3H), 7.16 (t, *J* = 7.8 Hz, 1H), 7.32 (d, *J* = 7.8 Hz, 1H), 7.53 (d, *J* = 7.8 Hz, 1H), 9.03 (s, 1H), 9.74 (br s, 1H).

### B. Preparation of 7-methoxy-3-oxo-1,3-dihydro-indazole-2-carboxylic acid 2-methyl-benzylamide

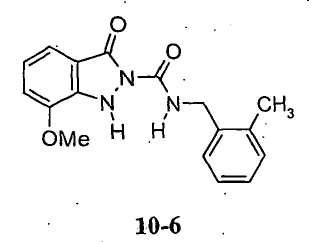

By following similar procedure as described in part B of Example 44, the title compound **10-6** is obtained as a white solid. ESIMS *m*/*e* 312 (M+H)⁺.

### Example 50

### 5-Hydroxy-3-oxo-1,3-dihydro-indazole-2-carboxylic acid 2-methyl-benzylamide

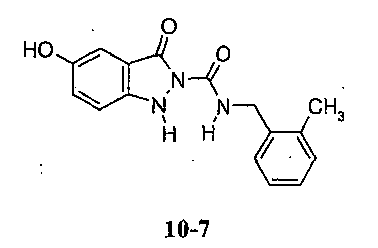

### A. Preparation of 5-hydroxy-1,2-dihydro-indazol-3-one

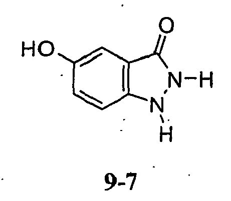

By following similar procedure as described in part A of Example 45, the title compound **9-7** is obtained as a solid (94% crude yield).

### B. Preparation of 5-hydroxy-3-oxo-1,3-dihydro-indazole-2-carboxylic acid 2-methyl-benzylamide

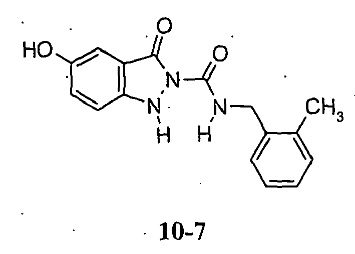

By following similar procedure as described in part B of Example 44, the title compound **10-7** is obtained as a white solid. ¹H-NMR (DMSO-d₆) δ2.31 (s, 3H), 4.51 (d, *J* = 5.9 Hz, 2H), 6.95 (s, 1H), 7.13-7.19 (m, 5H), 7.24-7.27 (m, 1H), 9.07 (t, *J* = 5.9 Hz, 1H), 9.52 (s, 1H), 10.59 (s, 1H).

### Example 51

### 5-Nitro-3-oxo-1,3-dihydro-indazole-2-carboxylic acid 2-methyl-benzylamide

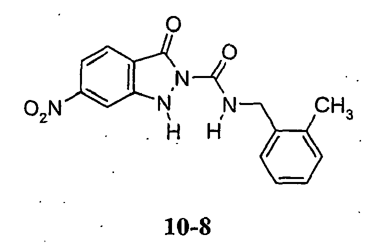

By following similar procedure as described in part B of Example 44, the title compound **10-8** is obtained as an orange solid (62% yield). ESIMS *m*/*e* 325 (M-H)⁻; Analysis for C₁₆H₁₄N₄O₄·2H₂O: calcd: C, 58.25; H, 4.40; N, 16.98; found: C, 58.45; H, 4.27; N, 16.79.

### Example 52

### 2-(2-Methyl-benzylcarbamoyl)-3-oxo-2,3-dihydro-1H-indazole-4-carboxylic acid

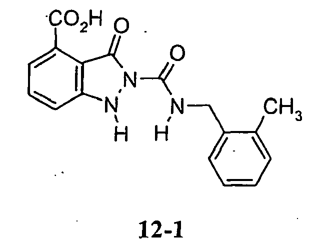

### A. Preparation of 3-oxo-2,3-dihydro-1H-indazole-4-carboxylic acid

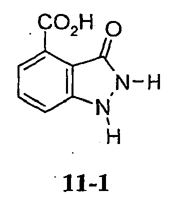

Concentrated hydrochloric acid (220 mL) is added slowly, in a period of 10 minutes, to a stirred suspension of 3-amino-phthalic acid hydrochloride (15.3 g, 70.3 mmol) in water (80 mL) at -10 °C. The resultant cold mixture is treated dropwise with a solution of sodium nitrite (4.86 g, 70.3 mmol) in water (60 mL). The clear solution is allowed to stir for 1 hour at -10 °C before it is added in small portions, for a period of 15 minutes, to a stirred solution of Tin(II) chloride (79.8 g, 420 mmol) in concentrated hydrochloric acid (179 mL) at -10 °C. The resultant mixture is stirred for 30 minutes at - 10 °C, then allowed to warm up to ambient temperature where it is stirred for another 16 hours. After filtration and vacuum drying, 11.0 g of the title compounds 11-1 (88% yield) is obtained as a white solid. mp >250 °C; ESIMS *m*/*e* 179 (M+H)⁺; ¹H-NMR (DMSO-d₆) δ7.49 (dd, *J* = 8.3 and 7.3 Hz, 1H), 7.61 (d, *J* = 8.3 Hz, 1H), 7.70 (d, *J* = 7.3 Hz, 1H), 12.14 (br s, 1H).

### B. Preparation of 2-(2-methyl-benzylcarbamoyl)-3-oxo-2,3-dihydro-1H-indazole-4-carboxylic acid

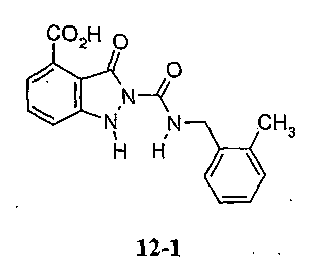

1-Isocyanatomethyl-2-methyl-benzene (0.095 mL, 0.68 mmol) is added dropwise to a stirred suspension of 3-oxo-2,3-dihydro-1*H*-indazole-4-carboxylic acid **11-1** (121 mg, 0.681 mmol) in anhydrous THF (10 mL) at ambient temperature under nitrogen, and the resultant mixture is allowed to stir for 16 hours. After concentration and subsequent chromatography on silica (0.1% HOAc and 0.5% CH₃OH in CHCl₃), the title compound **12-1** (85.4 mg, 39% yield) is obtained as a white solid. mp >250 °C; ESIMS *m*/*e* 326 (M+H)⁺. Analysis for C₁₇H₁₅N₃O₄: calcd: C, 62.76; H, 4.65; N, 12.92; found: C, 62.38; H, 4.35; N, 12.68.

### Example 53

### 2-(2-Methyl-benzylcarbamoyl)-3-oxo-2,3-dihydro-1H-indazole-5-carboxylic acid

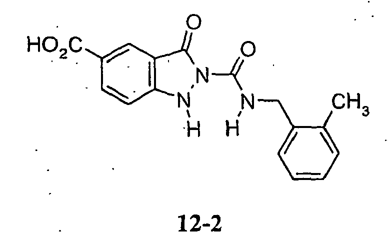

### A. Preparation of 3-oxo-2,3-dihydro-1H-indazole-5-carboxylic acid

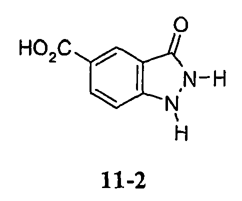

Concentrated hydrochloric acid (30 mL) is added slowly to a stirred suspension of 4-amino-isophthalic acid (0.855 g, 4.71 mmol) in water (45 mL)/THF (5 mL) at -10 °C. The resultant cold mixture is treated dropwise with a solution of sodium nitrite (0.325 g, 4.71 mmol) in water (5 mL). The clear solution is allowed to stir for 2 hours at -10 °C before it is added in small portions, for a period of 10 minutes, to a stirred solution of Tin(II) chloride (5.33 g, 28.1 mmol) in concentrated hydrochloric acid (12 mL) at -10 °C. The resultant mixture is stirred for 30 minutes at -10 °C, then allowed to warm up to ambient temperature where it is stirred for another 1 hour. After filtration and vacuum drying, the white solid is dissolved in DMF (20 mL) and the solution is heated at 120 °C for 2 hours. The mixture is then concentrated to a 5 mL solution at 50 °C under vacuum and diluted with xylene to form a white suspension. After filtration and vacuum drying, 803 mg of the title compounds **11-2** (96% yield) is obtained as a white solid. mp >250 °C; ESIMS *m*/*e* 179 (M+H)⁺; ¹H-NMR (DMSO-d₆) δ7.31 (d, *J* = 8.8 Hz, 1H), 7.83 (d, *J* = 8.8 Hz, 1H), 8.31 (s, 1H), 8.47 (br s, 1H), 11.92 (br s, 1H).

### B. Preparation of 2-(2-methyl-benzylcarbamoyl)-3-oxo-2,3-dihydro-1H-indazole-5-carboxylic acid

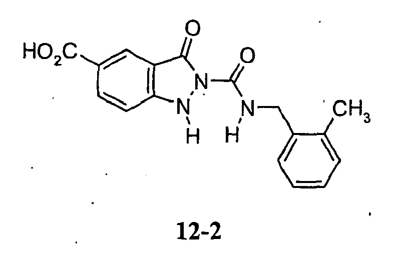

By following similar procedure as described in part B of Example 52, the title compound **12-2** is obtained as a white solid (25% yield). mp >250 °C; ESIMS *m*/*e* 326 (M+H)⁺.

### Example 54

### 2-(2-Methyl-benzylcarbamoyl)-3-oxo-2,3-dihydro-1H-indazole-6-carboxylic acid

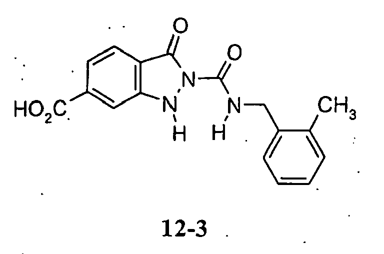

By following similar procedure as described in Example 53, the title compound **12-3** is obtained as a white solid. mp >25.0 °C; ¹H-NMR (DMSO-d₆) δ2.32 (s, 3H), 4.54 (d, *J* = 5.9 Hz, 2H), 7.15-7.19 (m, 3H), 7.27-7.29 (m, 1H), 7.61 (d, *J* = 8.3 Hz, 1H), 7.79 (s, 1H), 7.83 (d, *J* = 8.3 Hz, 1H), 9.06 (t, *J* = 5.9 Hz, 1H), 11.65 (br s, 1H).

### Example 55

### 2-(2-Methyl-benzylcarbamoyl)-3-oxo-2,3-dihydro-1H-indazole-7-carboxylic acid

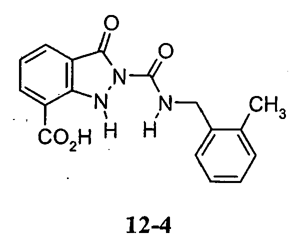

### A. Preparation of 3-oxo-2,3-dihydro-1H-indazole-7-carboxylic acid

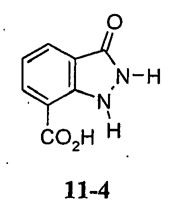

By following similar procedure as described in Part A of Example 52, the title compound **11-4** is obtained as a white solid (94% crude yield). mp >250 °C; ¹H-NMR (DMSO-d₆) δ7.07 (t, *J* = 7.4 Hz, 1H), 7.90 (d, *J* = 7.4 Hz, 2H), 11.58 (br s, 1H).

### B. Preparation of 2-(2-methyl-benzylcarbamoyl)-3-oxo-2,3-dihydro-1H-indazole-7-carboxylic acid

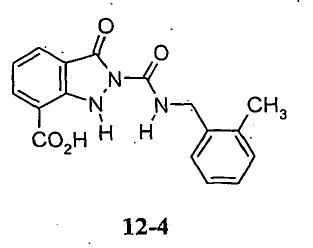

By following similar procedure as described in Part B of Example 52, the title compound **12-4** is obtained as a white solid (50% yield). mp >25.0 °C; ESIMS *m*/*e* 326 (M+H)⁺.

### Example 56

### 3-Oxo-2-(2-trifluoromelhyl-benzylcarbamoyl)-2,3-dihydro-1H-indazole-7-carboxylic acid

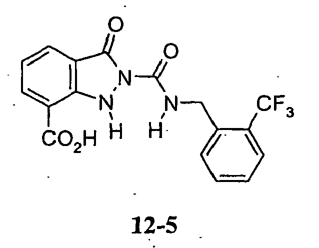

### A. Preparation of 1-isocyanatomethyl-2-trifluoromethyl-benzene

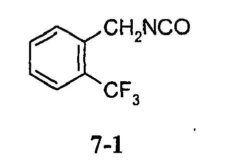

A solution of 2-trifluoromethyl-benzylamine (1.00 g, 5.71 mmol) and proton sponge (2.45 g, 11.4 mmol) in anhydrous CH₂Cl₂ (10 mL) is added dropwise to a stirred solution of triphosgene (678 mg, 2.28 mmol) in anhydrous CH₂Cl₂ (10 mL) at 0 °C. The resultant solution is allowed to stir at ambient temperature for 15 minutes. After dilution with CH₂Cl₂ (20 mL), the mixture is washed with 1N HCl (15x2 mL) and water (15 mL). The organic layer is dried over Na₂SO₄, filtered and concentrated to give the desired isocyanate **7-1** (1.07 g, 93% crude yield) as oil. ¹H-NMR (CDCl₃) δ4.02 (s, 2H), 7.35 (t, *J* = 7.5 Hz, 1H), 7.52-7.60 (m, 2H), 7.63 (d, *J* = 8.1 Hz, 1H).

### B. Preparation of 3-oxo-2-(2-trifluoromethyl-benzylcarbamoyl)-2,3-dihydro-1H-indazole-7-carboxylic acid

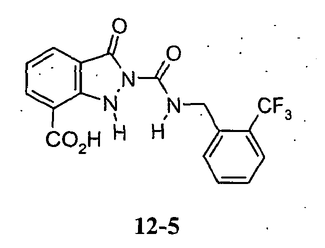

A solution of 1-isocyanatomethyl-2-trifluoromethyl-benzene **7-1** (1.07 g, 5.32 mmol) in anhydrous DMF (5 mL) is added to a stirred suspension of 3-oxo-2,3-dihydro-1*H*-indazole-7-carboxylic acid **11-4** (700 mg, 3.93 mmol) in anhydrous DMF (15 mL) at ambient temperature under nitrogen. The resultant solution is allowed to stir for 2 hours. Then the solution is treated dropwise with 0.5N HCl solution (20 mL) to form a white suspension. After filtration and vacuum drying at 50 °C, 855 mg of the title compound **12-5** is obtained as a white solid (57% yield). ESIMS *m*/*e* 380 (M+H)⁺.

### Example 57

### 2-(5-Fluoro-2-methyl-benzylcarbamoyl)-3-oxo-2,3-dihydro-1H-indazole-4-carboxylic acid

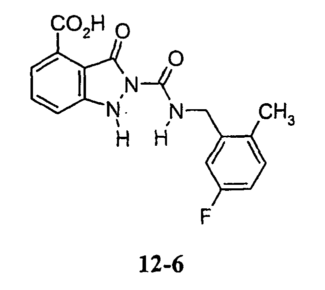

### A. Preparation of 4-fluoro-2-isocyanatomethyl-1-methyl-benzene

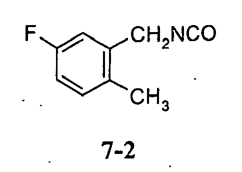

By following similar procedure as described in Part A of Example 56, the title compound **7-2** is synthesized from 5-fluoro-2-methyl-benzylamine as oil and used for the subsequent reaction.

### B. Preparation of 2-(5-fluoro-2-methyl-benzylcarbamoyl)-3-oxo-2,3-dihydro-1H-indazole-4-carboxylic acid

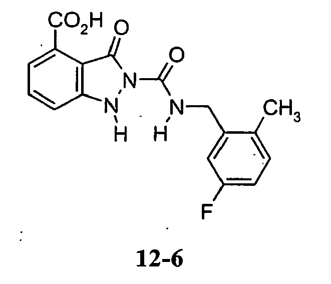

A solution of 4-fluoro-2-isocyanatomethyl-1-methyl-benzene **7-2** (313 mg, 1.89 mmol) in anhydrous DMF (2.5 mL) is added to a stirred solution of 3-oxo-2,3-dihydro-1*H*-indazole-4-carboxylic acid **11-1** (337 mg, 18.9 mmol) in anhydrous DMF (2.5 mL) at ambient temperature under nitrogen. The resultant solution is allowed to stir for 12 hours. Then the solution is concentrated under vacuum and the crude product is treated with CH*₃*OH/CH*2*Cl*2* to from a suspension. The solid is collected by filtration. The solid is then dissolved in DMF and water is added to precipitate out the product. After filtration and vacuum drying at 50 °C, the title compound **12-6** is obtained as a white solid. mp 230 °C (dec). ESIMS *m*/*e* 344 (M+H)⁺. Analysis for C₁₇H₁₄FN₃O₂·0.2H₂O: calcd: C, 58.70; H, 4.20; N, 12.08; found: C, 58.42; H, 3.88; N, 12.33.

### Example 58

### 2-(5-Fluoro-2-methyl-benzylcarbamoyl)-3-oxo-2,3-dihydro-1H-indazole-7-carboxylic acid

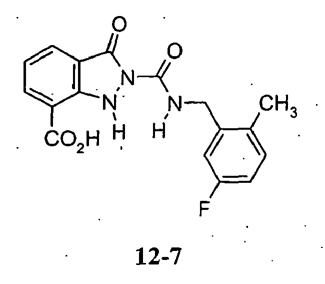

By following similar procedure as described in Example 57, the title compound **12-7** is obtained as a white solid. mp >250 °C; ESIMS *m*/*e* 344 (M+H)⁺.

### Example 59

### 2-(2,6-Dimethyl-benzylcarbamoyl)-3-oxo-2,3-dihydro-1H-indazole-4-carboxylic acid

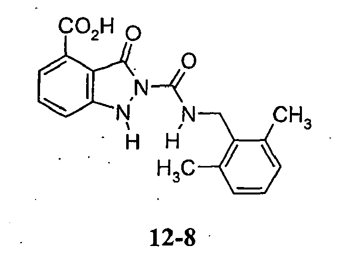

### A. Preparation of 2-isocyanatomethyl-1,3-dimethyl-benzene

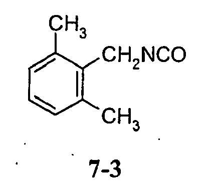

By following similar procedure as described in Part A of Example 56, the title compound **7-3** is synthesized from 2,6-dimethyl-benzylamine as oil (83% yield) and used for the subsequent reaction.

### B. Preparation of 2-(2,6-dimethyl-benzylcarbamoyl)-3-oxo-2,3-dihydro-1H-indazole-4-carboxylic acid

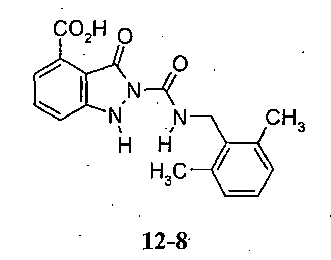

By following similar procedure as described in part B of Example 52, the title compound **12-8** is obtained as a solid (27% yield). mp >250 °C; ESIMS *m*/*e* 338 (M-H)⁻.

### Example 60

### 2-(2,6-Dimethyl-benzylcarbamoyl)-3-oxo-2,3-dihydro-1H-indazole-7-carboxylic acid

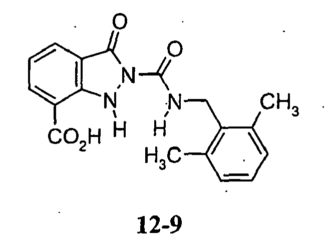

By following similar procedure as described in part B of Example 52, the title compound **12-9** is obtained as a solid (37% yield). mp >250 °C; ESIMS *m*/*e* 340 (M+H)⁺.

### Example 61

### 2-(2-Ethyl-6-methyl-benzylcarbamoyl)-3-oxo-2,3-dihydro-1H-indazole-4-carboxylic acid

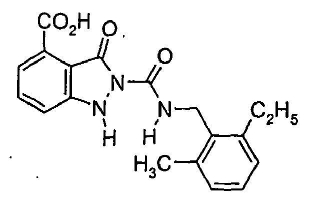

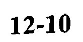

### A. Preparation of 1-ethyl-2-isocyanatomethyl-3-methyl-benzene

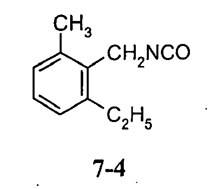

2-Ethyl-6-methyl-benzylamine **6-4** is synthesized by the LiAlH4 reduction of 2-ethyl-6-methyl-benzonitrile according to the procedure described in Part B of Example 33. Then, by following similar procedure as described in Part A of Example 56, the title compound **7-4** is obtained as oil (91% yield) from compound **6-4**. ¹H-NMR (DMSO-d₆) δ1.16 (t, *J* = 7.6 Hz, 3H), 2.69 (q, *J* = 7.6 Hz, 2H), 4.52 (s, 2H), 7.08 (d, *J* = 7.6 Hz, 2H), 7.16 (t, *J* = 7.6 Hz" 1H).

### B. Preparation of 2-(2-ethyl-6-methyl-benzylcarbamoyl)-3-oxo-2,3-dihydro-1H-indazole-4-carboxylic acid

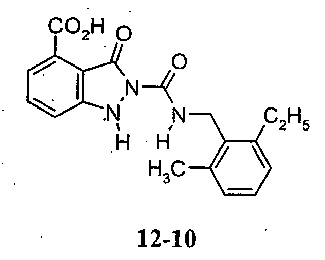

By following similar procedure as described in part B of Example 52, the title compound **12-10** is obtained as a solid (31% yield). mp >250 °C; ESIMS *m*/*e* 354 (M+H)⁺.

### Example 62

### 2-(2-Ethyl-6-methyl-benzylcarbamoyl)-3-oxo-2,3-dihydro-1H-indazole-5-carboxylic acid

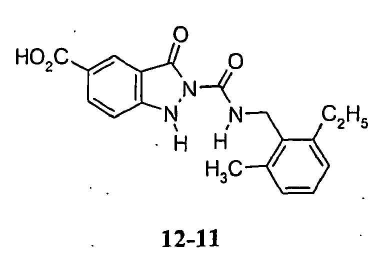

By following similar procedure as described in Example 61, the title compound **12-11** is obtained as a solid. mp >250 °C; ESIMS *m*/*e* 354 (M+H)⁺.

### Example 63

### 2-(2-Ethyl-6-methyl-benzylcarbamoyl)-3-oxo-2,3-dihydro-1H-indazole-6-carboxylic acid

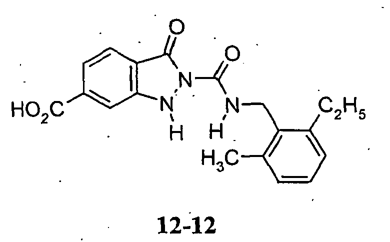

By following similar procedure as described in Example 61, the title compound **12-12** is obtained as a solid. mp >250 °C; ESIMS *m*/*e* 354 (M+H)⁺.

### Example 64

### 2-(2-Ethyl-6-methyl-benzylcarbamoyl)-3-oxo-2,3-dihydro-1H-indazole-7-carboxylic acid

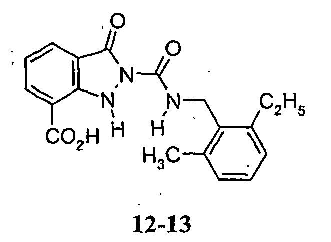

By following similar procedure as described in Example 61, the title compound **12-13** is obtained as a solid (39% yield). mp >250 °C; ESIMS *m*/*e* 354 (M+H)⁺. Analysis for C₁₉H₁₉N₃O₄: calcd: C, 64.58; H, 5.42; N, 11.89; found: C, 64.36; H, 5.39; N, 11.81.

### Example 65

### 2-(2-Isopropyl-6-methyl-benzylcarbamoyl)-3-oxo-2,3-dihydro-1H-indazole-4-carboxylic acid

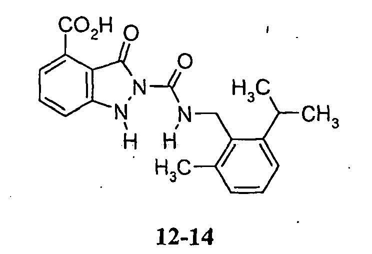

### A. Preparation of 2-isocyanatomethyl-1-isopropyl-3-methyl-benzene

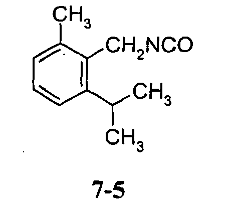

By following similar procedure as described in Part A of Example 56, the title compound 7-5 is obtained from 2-isopropyl-6-methyl-benzylamine **6-2** as oil (99% yield). ¹H-NMR (DMSO-d₆) δ1.18 (d, *J* = 6.9 Hz, 6H), 2.35 (s, 3H), 3.24 (h, *J* = 6.9 Hz, 1H), 4.55 (s, 2H), 7.03-7.06 (m, 1H), 7.17-7.23 (m, 2H).

### B. Preparation of 2-(2-isopropyl-6-methyl-benzylcarbamoyl)-3-oxo-2,3-dihydro-1H-indazole-4-carboxylic acid

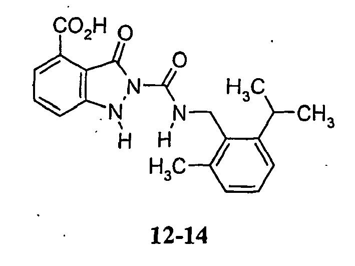

By following similar procedure as described in Part B of Example 52, the title compound **12-14** is obtained as a solid (69% yield). ESIMS *m*/*e* 368 (M+H)⁺.

### Example 66

### 2-(2-Isopropyl-6-methyl-benzylcarbamoyl)-3-oxo-2,3-dihydro-1H-indazole-7-carboxylic acid

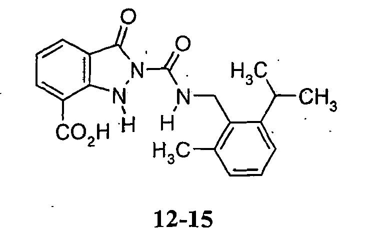

2-Isocyanatomethyl-1-isopropyl-3-methyl-benzene **7-5** (4.12 g, 21.8 mmol) is added to a stirred suspension of 3-oxo-2,3-dihydro-1*H*-indazole-7-carboxylic acid **11-4** (2.58 g, 14.5 mmol) in anhydrous DMF (90 mL) at ambient temperature under nitrogen and the resultant clear solution is allowed to stir for 1.5 hours. The solution is treated in small portions with 1N HCl (25 mL) to form a suspension, then followed by the addition of water (100 mL). The white solid is collected by filtration and dried at 50 °C in vacuum oven. A total of 4.69 g of the title compound **12-15** is obtained (88% yield). ESIMS *m*/*e* 368 (M+H)⁺. Analysis for C₂₀H₂₁N₃O₄: calcd: C, 65.38; H, 5.76; N, 11.44; found: C, 64.99; H, 5.87; N, 11.25.

### Example 67

### 2-(2-tert-Butyl-6-methyl-benzylcarbamoyl)-3-oxo-2,3-dihydro-1H-indazole-7-carboxylic acid

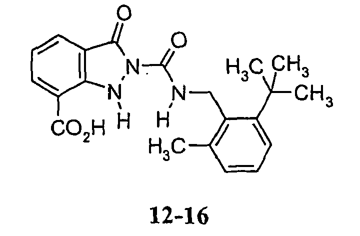

### A. Preparation of 1-tert-butyl-2-isocyanatomethyl-3-methyl-benzene

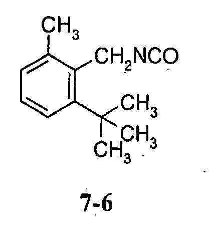

2-*tert*-Butyl-6-methyl-benzylamine **6-5** is synthesized from 2-*tert*-butyl-6-methyl-benzobromide according to the procedure described in Part A and B of Example 33. Then, by following similar procedure as described in Part A of Example 56, the title compound **7-6** is obtained as oil from compound **6-5** (90% yield) and used for the subsequent reaction.

### B. Preparation of 2-(2-tert-butyl-6-methyl-benzylcarbamoyl)-3-oxo-2,3-dihydro-1H-indazole-7-carboxylic acid

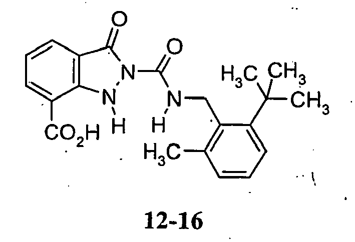

By following similar procedure as described in Example 66, the title compound **12-16** is obtained as solid (85% yield). ESIMS *m*/*e* 382 (M+H)⁺.

### Example 68

### 2-(4-Butyl-2-methyl-benzylcarbamoyl)-3-oxo-2,3-dihydro-1H-indazole-4-carboxylic acid

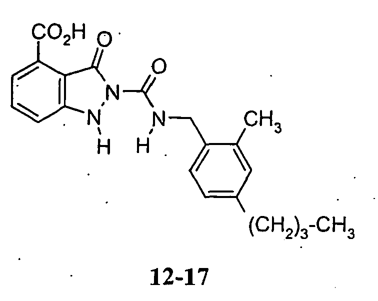

### A. Preparation of 4-butyl-1-isocyanatomethyl-2-methyl-benzene

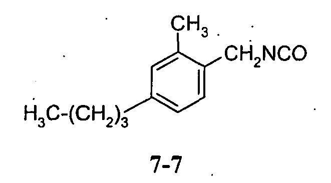

4-Butyl-2-methyl-benzylamine **6-6** is synthesized from 4-butyl-2-methyl-benzonitrile according to the procedure described in Part B of Example 33. Then, by following similar procedure as described in Part A of Example 56, the title compound 7-7 is obtained as oil from compound **6-6** (88% yield) and used for the subsequent reaction.

### B. Preparation of 2-(4-butyl-2-methyl-benzylcarbamoyl)-3-oxo-2,3-dihydro-1H-indazole-4-carboxylic acid

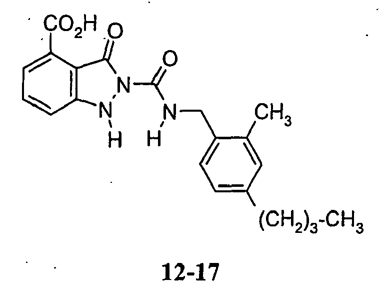

4-Butyl-1-isocyanatomethyl-2-methyl-benzene 7-7 (0.146 mL, 0.842 mmol) is added to a stirred suspension of 3-oxo-2,3-dihydro-1*H*-indazole-7-carboxylic acid **11-1** (150 mg, 0.842 mmol) in anhydrous DMF (5 mL) at ambient temperature under nitrogen and the resultant mixture is allowed to stir for 16 hours. After concentration the wet solid is suspended in CHCl₃ before it is collected by filtration to recover 45 mg of starting **11-1.** The filtrate is concentrated and chromatographed on silica (0.1 % HOAc and 0.5% CH₃OH in CHCl₃) to give 36.6 mg of the title compound **12-17** as a yellowish solid (11% yield). mp 173.0-175.0 °C; ESIMS *mle* 382 (M+H)⁺. Analysis for C₂₁H₂₃N₃O₄: calcd: C, 66.13; H, 6.08; N, 11.02; found: C, 65.87; H, 5.80; N, 10.85.

### Example 69

### 2-(2,6-Diethyl-benzylcarbamoyl)-3-oxo-2,3-dihydro-1H-indazole-4-carboxylic acid

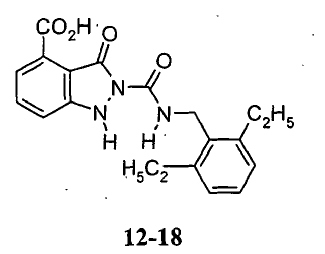

### A. Preparation of 1,3-diethyl-2-isocyanatomethyl-benzene

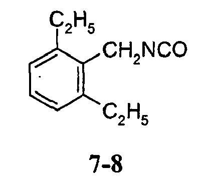

2,6-Diethyl-benzylamine **6-7** is synthesized from 2-bromo-1,3-diethyl-benzene according to the procedure described in Part A and B of Example 33. Then, by following similar procedure as described in Part A of Example 56, the title compound **7-8** is obtained as oil from compound **6-7** (100% yield). ¹H-NMR (DMSO-d₆) δ1.16 (t, *J* = 7.8 Hz, 6H), 2.68 (q, *J* = 7.8 Hz, 4H), 4.54 (s, 2H), 7.08 (d, *J* = 7.4 Hz" 2H), 7.21 (t, *J* = 7.4 Hz, 1H).

### B. Preparation of 2-(2,6-diethyl-benzylcarbamoyl)-3-oxo-2,3-dihydro-1H-indazole-4-carboxylic acid

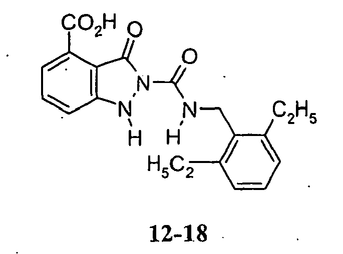

By following similar procedure as described in Part B of Example 68, the title compound **12-18** is obtained as a white solid (57% yield). mp >250 °C; ESIMS *m*/*e* 368 (M+H)⁺. Analysis for C₂₀H₂₁N₃O₄: calcd: C, 65.38; H, 5.76; N, 11.44; found: C, 65.18; H, 5.76; N, 11.32.

### Example 70

### 2-(2,6-Diethyl-benzylcarbamoyl)-3-oxo-2,3-dihydro-1H-indazole-7-carboxylic acid

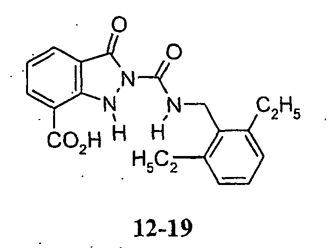

By following similar procedure as described in Example 69, the title compound **12-19** is obtained as a white solid (42% yield). mp >250 °C; ESIMS *m*/*e* 368 (M+H)⁺. Analysis for C₂₀H₂₁N₃O₄: calcd: C, 65.38; H, 5.76; N, 11.44; found: C, 65.55; H, 5.86; N, 11.47.

### Example 71

### 2-(2,6-Diisopropyl-benzylcarbamoyl)-3-oxo-2,3-dihydro-1H-indazole-4-carboxylic acid

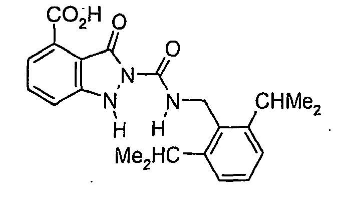

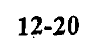

### A..Preparation of 1,3-diisopropyl-2-isocyanatomethyl-benzene

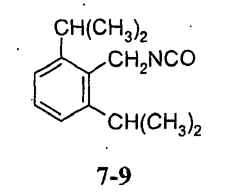

2,6-Diisopropyl-benzylamine **6-8** is synthesized from 2-bromo-1,3-diisopropylbenzene according to the procedure described in Part A and B of Example 33. Then, by following similar procedure as described in Part A of Example 56, the title compound **7-9** is obtained as oil from compound **6-8** (87% yield). ¹H-NMR (DMSO-d₆) δ1.18 (d, *J* = 6.8 Hz, 12H), 3.23 (h, *J* = 6.8 Hz, 2H), 4.57 (s, 2H), 7.18 (d, *J* = 7.8 Hz,, 2H), 7.28 (t, *J* = 7.8 Hz, 1H).

### B. Preparation of 2-(2,6-diisopropyl-benzylcarbamoyl)-3-oxo-2,3-dihydro-1H-indazole-4-carboxylic acid

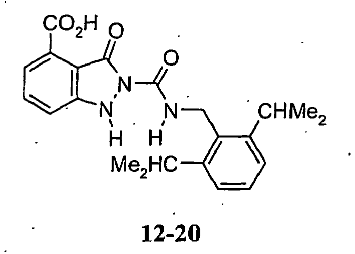

By following similar procedure as described in Part B of Example 68, the title compound **12-20** is obtained as a yellowish solid (15% yield). ESIMS *m*/*e* 396 (M+H)⁺. Analysis for C₂₂H₂₅N₃O₄: calcd: C, 66.82; H, 6.37; N, 10.63; found: C, 66.46; H, 6.35; N, 10.60.

### Example 72

### 2-(2,6-Diisopropyl-benzylcarbamoyl)-3-oxo-2,3-dihydro-1H-indazole-7-carboxylic acid

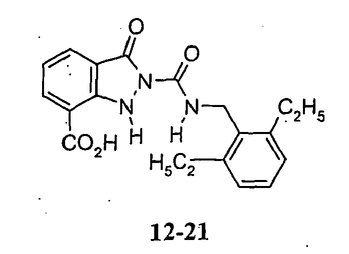

By following similar procedure as described in Example 71, the title compound **12-21** is obtained as a white solid (33% yield). mp >250 °C; ESIMS *m*/*e* 396 (M+H)⁺. Analysis for C₂₂H₂₅N₃O₄: calcd: C, 66.82; H, 6.37; N, 10.63; found: C, 66.51; H, 6.28; N, 10.59.

### Example 73

### 2-(2,4,6-Trimethyl-benzylcarbamoyl)-3-oxo-2,3-dihydro-1H-indazole-4-carboxylic acid

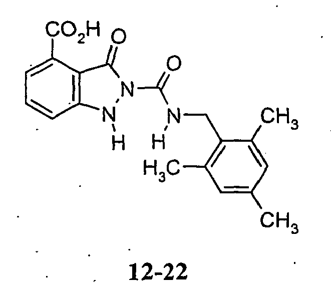

### A. Preparation of 2-isocyanatomethyl-1,3,5-trimethyl-benzene

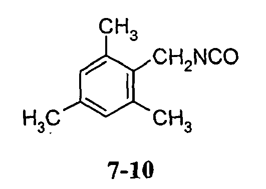

2,4,6-Trimethyl-benzylamine **6-9** is synthesized from 2-bromo-1,3,5-trimethylbenzene according to the procedure described in Part A and B of Example 33. Then, by following similar procedure as described in Part A of Example 56, the title compound **7-10** is obtained as oil from compound **6-9** (84% yield). ¹H-NMR (CDCl₃) δ2.27 (s, 3H), 2.37 (s, 6H), 4.40 (s, 2H), 6.88 (s, 2H).

### B. Preparation of 2-(2,4,6-trimethyl-benzylcarbamoyl)-3-oxo-2,3-dihydro-1H-indazole-4-carboxylic acid

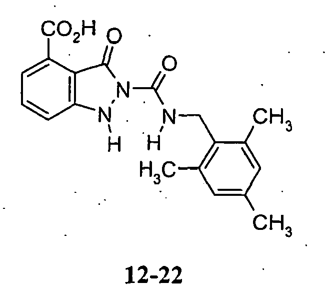

By following similar procedure as described in Part B of Example 68, the title compound **12-22** is obtained as a white solid (45% yield). ESIMS *m*/*e* 354 (M+H)⁺.

### Example 74

### 2-[(4-Methyl-naphthalen-1-ylmethyl)-carbamoyl]-3-oxo-2,3-dihydro-1H-indazole-4-carboxylic acid

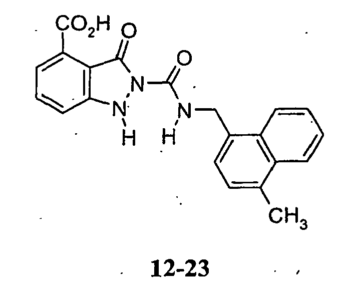

### A. Preparation of 1-isocyanatomethyl-4-methyl-naphthalene

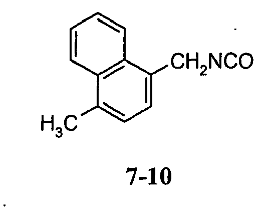

(4-Methyl-naphthalen-1-yl)-methylamine **6-10** is synthesized from 4-methylnaphthalene-1-carbonitrile according to the procedure described in Part B of Example 33. Then, by following similar procedure as described in Part A of Example 56, the title compound **7-10** is obtained as oil from compound **6-10** (96% yield). ¹H-NMR (DMSO-d₆) δ2.64 (s, 3H), 5.00 (s, 2H), 7.35 (d, *J* = 7.0 Hz, 1H), 7.45 (d, *J*= 7.0 Hz, 1H), 7.59-7.63 (m, 2H), 8.05-8.08 (m, 2H).

### B. Preparation of 2-[(4-methyl-naphthalen-1-ylmethyl)-carbamoyl]-3-oxo-2,3-dihydro-1H-indazole-4-carboxylic acid

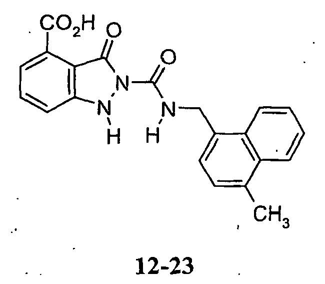

By following similar procedure as described in Part B of Example 68, the title compound **12-23** is obtained as a yellowish solid (24% yield). ESIMS *m*/*e* 376 (M+H)⁺.

### Example 75

### 3-Oxo-2-(4-phenyl-butylcarbamoyl)-2,3-dihydro-1H-indazole-4-carboxylic acid

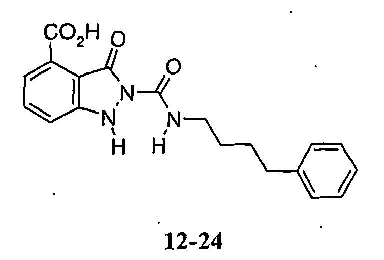

### A. Preparation of (4-isocyanato-butyl)-benzene

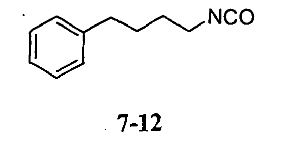

By following similar procedure as described in Part A of Example 56, the title compound **7-12** is synthesized from 4-phenyl-butylamine as oil (81% yield) and used for the subsequent reaction.

### B. Preparation of 3-oxo-2-(4-phenyl-butylcarbamoyl)-2,3-dihydro-1H-indazole-4-carboxylic acid

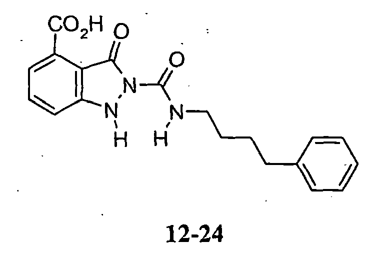

By following similar procedure as described in Part B of Example 68, the title compound **12-24** is obtained as a white solid (34% yield). mp >250 °C; ESIMS *m*/*e* 354 (M+H)⁺. Analysis for C₁₉H₁₉N₃O₄: calcd: C, 64.58; H, 5.42; N, 11.89; found: C, 64.42; H, 5.42; N, 11.88.

### Example 76

### 2-(4-Cyclohexyl-butylcarbamoyl)-3-oxo-2,3-dihydro-1H-indazole-4-carboxylic acid

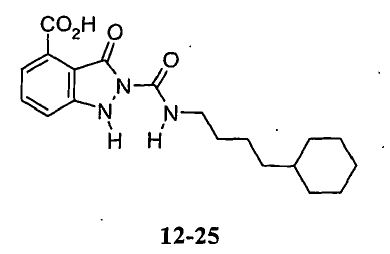

### A. Preparation of (4-isocyanato-butyl)-cyclohexane

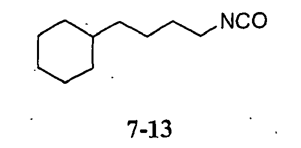

4-Cyclohexyl-butylamine is synthesized from 4-cyclohexyl-butyronitrile according to the procedure described in Part B of Example 33. Then, by following similar procedure as described in Part A of Example 56, 4-cyclohexyl-butylamine is converted to the title compound **7-13** as oil (91 % yield) and used for the subsequent reaction.

### B. Preparation of 2-(4-cyclohexyl-butylcarbamoyl)-3-oxo-2,3-dihydro-1H-indazole-4-carboxylic acid

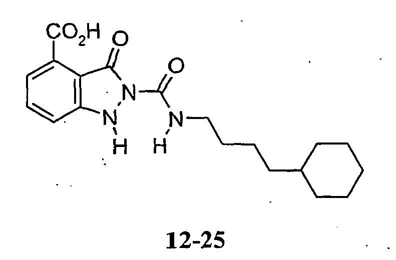

By following similar procedure as described in Part B of Example 68, the title compound **12-25** is obtained as a white solid. mp 196.0-198.0 °C; ESIMS *m*/*e 360* (M+H)⁺. Analysis for C₁₉H₂₅N₃O₄·0.1H₂O: calcd: C, 63.18; H, 7.03; N, 11.63; found: C, 62.96; H, 6.93; N, 11.45.

### Example 77

### 3-Oxo-2-(3-phenoxy-propylcarbamoyl)-2,3-dihydro-1H-indazole-4-carboxylic acid

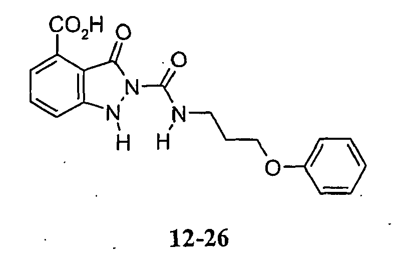

### A. Preparation of (3-isocyanato-propoxy)-benzene

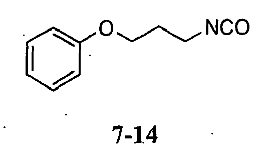

By following similar procedure as described in Part A of Example 56, the title compound **7-14** is synthesized from 3-phenoxy-propylamine as oil (95% yield) and used for the subsequent reaction.

### B. Preparation of 3-oxo-2-(3-phenoxy-propylcarbamoyl)-2,3-dihydro-1H-indazole-4-carboxylic acid

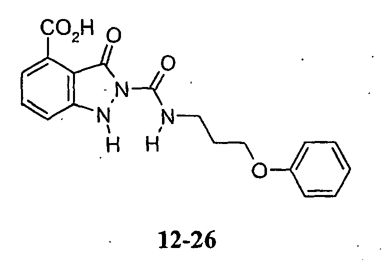

By following similar procedure as described in Part B of Example 68, the title compound **12-26** is obtained as a white solid. mp 206.0-208.0 °C; ESIMS *m*/*e* 356 (M+H)⁺.

### Example 78

### 2-Hexylcarbamoyl-3-oxo-2,3-dihydro-1H-indazole-4-carboxylic acid

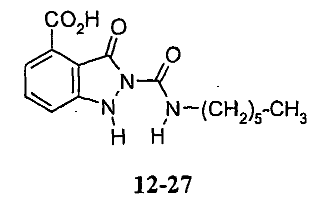

By following similar procedure as described in Part B of Example 52, the title compound **12-27** is obtained as a white solid (40% yield). mp >250 °C; ESIMS *m*/*e* 304 (M-H)⁻.

### Example 79

### 2-(2-Ethyl-6-methyl-benzylcarbamoyl)-1-methyl-3-oxo-2,3-dihydro-1H-indazole-7-carboxylic acid

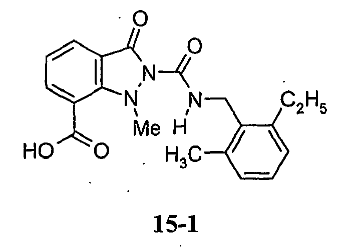

### A. Preparation of 2-(2-ethyl-6-methyl-benzylcarbamoyl)-3-oxo-2,3-dihydro-1H-indazole-7-carboxylic acid benzhydryl ester

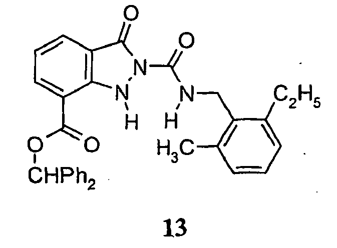

A solution of diphenyldiazomethane (646 mg, 3.32 mmol) in CH₂Cl₂ (10 mL) is added dropwise to a stirred suspension of 2-(2-ethyl-6-methyl-benzylcarbamoyl)-3-oxo-2,3-dihydro-1*H*-indazole-7-carboxylic acid **12-13** (1.12 g, 3.17 mmol) in CH₂Cl₂ (30 mL) at ambient temperature. The resultant mixture is allowed to stir for 1 hour. The clear solution is concentrated and the crude product is chromatographed on silica (gradient 0-0.5% CH₃OH in CHCl₃) to give 1.38 g of the title compound **13** (84% yield) as white foam. ESIMS *m*/*e* 520 (M+H)⁺. Analysis for C₃₂H₂₉N₃O₄: calcd: C, 73.97; H, 5.63; N, 8.09; found: C, 74.28; H, 5.90; N, 7.95.

### B. Preparation of 2-(2-ethyl-6-methyl-benzylcarbamoyl)-1-methyl-3-oxo-2,3-dihydro-1H-indazole-7-carboxylic acid benzhydryl ester

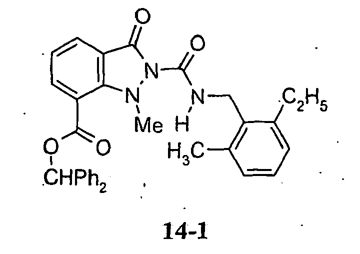

(Trimethylsilyl)diazomethane (0.42 mL, 2M in hexane) is added dropwise to a stirred solution of 2-(2-ethyl-6-methyl-benzylcarbamoyl)-3-oxo-2,3-dihydro-1*H-*indazole-7-carboxylic acid benzhydryl ester **13** (424 mg, 0.816 mmol) in CH₃OH (5 mL)/CH₂Cl₂ (10 mL) at ambient temperature. The resultant solution is allowed to stir for 4 hours. The solution is concentrated and the crude product is chromatographed on silica (gradient 1-20% EtOAc in hexane) to give 309 mg of the title compound **14-1** (71% yield) as white foam. ESIMS *m*/*e* 534 (M+H)⁺. Analysis for C₃₃H₃₁N₃O₄: calcd: C, 74.28; H, 5.86; N, 7.87; found: C, 73.89; H, 5.74; N, 7.65.

### C. Preparation of 2-(2-ethyl-6-methyl-benzylcarbamoyl)-1-methyl-3-oxo-2,3-dihydro-1H-indazole-7-carboxylic acid

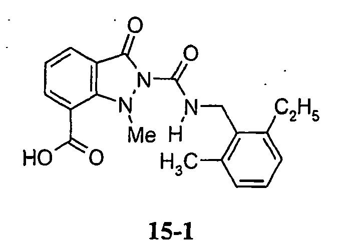

Hydrogen chloride gas is bubbled slowly through a stirred solution of 2-(2-ethyl-6-methyl-benzylcarbamoyl)-1-methyl-3-oxo-2,3-dihydro-1*H*-indazole-7-carboxylic acrid benzhydryl ester **14-1** (280 mg, 0.525 mmol) in CH₂Cl₂ (25 mL) at ambient temperature for a few minutes and the resultant solution is allowed to stir for 1 hour. After concentration, the crude product is chromatographed on silica (gradient 0.1/0.5/99.4 to 0.5/1/98.5 HOAc/CH₃OH/CHCl₃) to give 135 mg of the title compound **15-1** (70% yield) as a white solid. mp >250 °C; ESIMS *m*/*e* 368 (M+H)⁺.

### Example 80

### 1-Ethyl-2-(2-ethyl-6-methyl-benzylcarbamoyl)-3-oxo-2,3-dihydro-1H-indazole-7-carboxylic acid

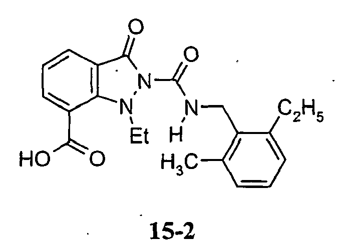

### A. Preparation of 1-ethyl-2-(2-ethyl-6-methyl-benzylcarbamoyl)-3-oxo-2,3-dihydro-1H-indazole-7-carboxylic acid benzhydryl ester

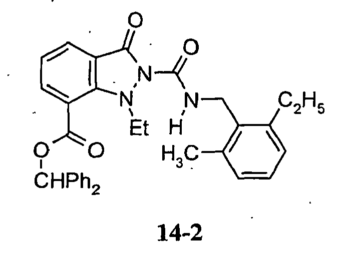

Ethyl iodide (0.52 mL, 6.5 mmol) and potassium carbonate (44 mg, 0.32 mmol) are added successively to a stirred solution of 2-(2-ethyl-6-methyl-benzylcarbamoyl)-3-oxo-2,3-dihydro-1*H*-indazole-7-carboxylic acid benzhydryl ester **13** (83.6 mg, 0.161 mmol) in anhydrous DMF (4 mL) at ambient temperature under nitrogen. The resultant mixture is allowed to stir for 3 hours. Xylene (5 mL) is added to the mixture. After filtration and subsequent concentration *in vacuo,* the crude product is chromatographed on silica (gradient 1-20% EtOAc in hexane) to give 67.8 mg of the title compound **14-2** (77% yield) as white foam. ESIMS *m*/*e* 548 (M+H)⁺.

### B. Preparation of 1-ethyl-2-(2-ethyl-6-methyl-benzylcarbamoyl)-3-oxo-2,3-dihydro-1H-indazole-7-carboxylic acid

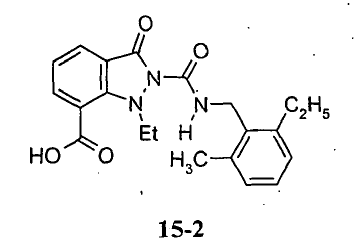

By following similar procedure as described in Part C of Example 79, the title compound **15-2** is obtained as a white solid (55% yield). mp >250 °C; ESIMS *m*/*e* 382 (M+H)⁺.

### Example 81

### 2-(2-Ethyl-6-methyl-benzylcarbamoyl)-3-oxo-1-propyl-2,3-dihydro-1H-indazole-7-carboxylic acid

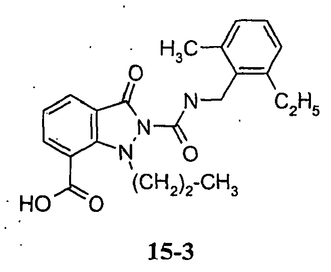

By following similar procedure as described in Example 80, the title compound **15-3** is obtained as a white solid (56% yield). mp >250 °C; ESIMS *m*/*e* 396 (M+H)⁺. Analysis for C₂₂H₂₅N₃O₄·0.2H₂O: calcd: C, 66.22; H, 6.42; N, 10.53; found: C, 66.04; H, 6.36; N, 10.45.

### Example 82

### 1-Carboxymethyl-2-(2-ethyl-6-methyl-benzylcarbamoyl)-3-oxo-2,3-dihydro-1H-indazole-7-carboxylic acid

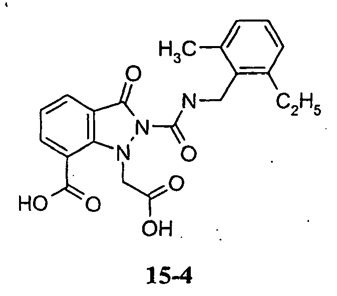

### A. Preparation of 1-tert-butoxycarbonylmethyl-2-(2-ethyl-6-methyl-benzylcarbamoyl)-3-oxo-2,3-dihydro-1H-indazole-7-carboxylic acid benzhydryl ester

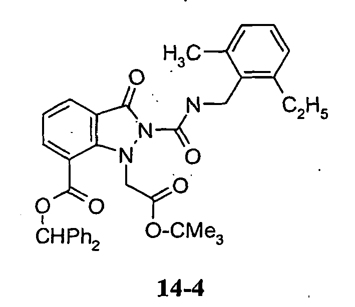

*tert*-Butyl bromoacetate (0.89 mL, 5.8 mmol) and potassium carbonate (0.24 g, 1.7 mmol) are added successively to a stirred solution of 2-(2-ethyl-6-methyl-benzylcarbamoyl)-3-oxo-2,3-dihydro-1*H*-indazole-7-carboxylic acid benzhydryl ester **13** (0.30 g, 0.58 mmol) in anhydrous DMF (8 mL) at ambient temperature under nitrogen. The resultant mixture is allowed to stir for 1 hour. Xylene (10 mL) is added to the mixture. After filtration and subsequent concentration *in vacuo,* the crude product is chromatographed on silica (gradient 1-20% EtOAc in hexane) to give 278 mg of the title compound **14-4** (76% yield) as white foam. ESIMS *m*/*e* 634 (M+H)⁺. Analysis for C₃₈H₃₉N₃O₆·0.2H₂O: calcd: C, 71.61; H, 6.23; N, 6.59; found: C, 71.48; H, 6.21; N, 6.62

### B. Preparation of 1-carboxymethyl-2-(2-ethyl-6-methyl-benzylcarbamoyl)-3-oxo-2,3-dihydro-1H-indazole-7-carboxylic acid

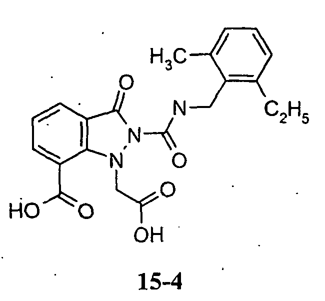

By following similar procedure as described in Part C of Example 79, the title compound **15-4** is obtained as a white solid (68% yield). mp 172.0-174.0 °C; ESIMS *m*/*e* 412 (M+H)⁺. Analysis for C₂₁H₂₁N₃O₆·0.2H₂O: calcd; C, 60.78; H, 5.20; N, 10.12; found: C, 60.51; H, 4.84; N, 9.88.

### Example 83

### 2-(2-Ethyl-6-methyl-benzylcarbamoyl)-1-methoxycarbonylmethyl-3-oxo-2,3-dihydro-1H-indazole-7-carboxylic acid

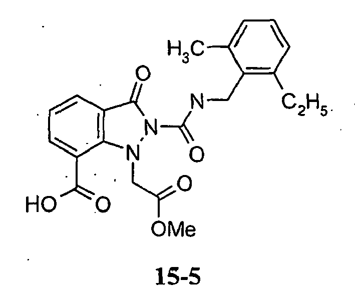

By following similar procedure as described in Example 82, the title compound **15-5** is obtained as a white solid. mp 220.0-222.0 °C; ESIMS *m*/*e* 426 (M+H)⁺.

### Example 84

### 2-(2-Isopropyl-6-methyl-benzylcarbamoyl)-1-methyl-3-oxo-2,3-dihydro-1H-indazole-7-carboxylic acid

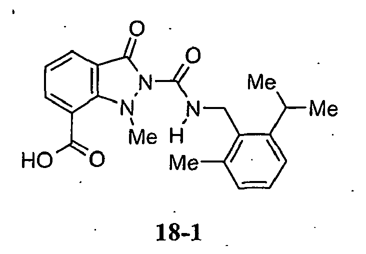

### A. Preparation of 2-(2-isopropyl-6-methyl-benzylcarbamoyl)-3-oxo-2,3-dihydro-1H-indazole-7-carboxylic acid benzhydryl ester

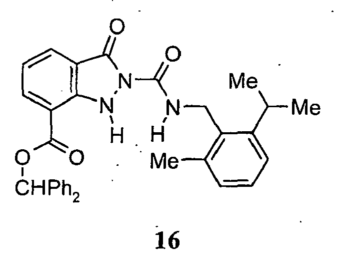

By following similar procedure as described in Part A of Example 79, the title compound **16** is obtained as a white solid (88% yield). ESIMS *m*/*e* 532 (M-H)⁻. Analysis for C₃₃H₃₁N₃O₄·0.2C₆H₁₄: calcd: C, 74.57; H, 6.18; N, 7.63; found: C, 74.96; H, 5.78; N, 7.64.

### B. Preparation of 2-(2-isopropyl-6-methyl-benzylcarbamoyl)-1-methyl-3-oxo-2,3-dihydro-1H-indazole-7-carboxylic acid benzhydryl ester

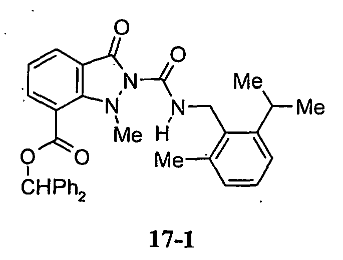

By following similar procedure as described in Part B of Example 79, the title compound **17-1** is obtained as white foam (79% yield). ESIMS *m*/*e* 548 (M+H)⁺. Analysis for C₃₄H₃₃N₃O₄: calcd: C, 74.57; H, 6.07; N, 7.67; found: C, 74.78; H, 6.19; N, 7.65.

### C. Preparation of 2-(2-isopropyl-6-methyl-benzylcarbamoyl)-1-methyl-3-oxo-2,3-dihydro-1H-indazole-7-carboxylic acid

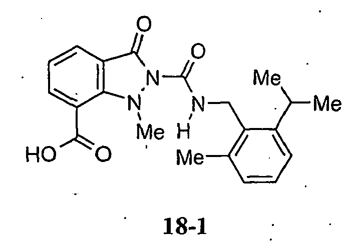

By following similar procedure as described in Part C of Example 79, the title compound **18-1** is obtained as a white solid (97% yield). ESIMS *m*/*e* 382 (M+H)⁺.

### Example 85

### 1-Ethyl-2-(2-isopropyl-6-methyl-benzylcarbamoyl)-3-oxo-2,3-dihydro-1H-indazole-7-carboxylic acid

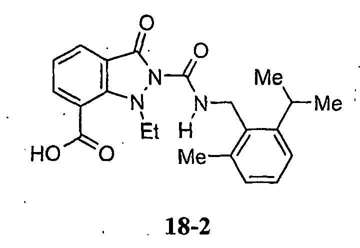

### A. Preparation of 1-ethyl-2-(2-isopropyl-6-methyl-benzylcarbamoyl)-3-oxo-2,3-dihydro-1H-indazole-7-carboxylic acid benzhydryl ester

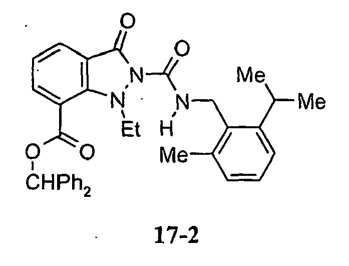

Ethyl iodide (0.60 mL, 7.5 mmol) and potassium carbonate (104 mg, 0.750 mmol) are added successively to a stirred solution of 2-(2-isopropyl-6-methyl-benzylcarbamoyl)-3-oxo-2,3-dihydro-1*H*-indazole-7-carboxylic acid benzhydryl ester **16** (200 mg, 0.375 mmol) in anhydrous DMF (4 mL) at ambient temperature under nitrogen. The resultant mixture is allowed to stir for 5 hours. Xylene (5 mL) is added to the mixture. After filtration and subsequent concentration *in vacuo*, the crude product is chromatographed on silica (gradient 1-15% EtOAc in hexane) to give 167 mg of the title compound **17-2** (79% yield) as white foam. ESIMS *m*/*e* 562 (M+H)⁺. Analysis for C₃₅H₃₅N₃O₄: calcd: C, 74.84; H, 6.28; N, 7.48; found: C, 74.60; H, 6.14; N, 7.33.

### B. Preparation of 1-ethyl-2-(2-isopropyl-6-methyl-benzylcarbamoyl)-3-oxo-2,3-dihydro-1H-indazole-7-carboxylic acid

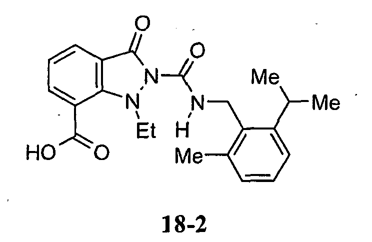

By following similar procedure as described in Part C of Example 79, the title compound **18-2** is obtained as a white solid (79% yield). ESIMS *m*/*e* 396 (M+H)⁺. Analysis for C₂₂H₂₅N₃O₄·0.2H₂O: calcd: C, 66.22; H, 6.42; N, 10.53; found: C, 66.30; H, 6.36; N, 10.41.

### Example 86

### 2-(2-Isopropyl-6-methyl-benzylcarbamoyl)-3-oxo-1-propyl-2,3-dihydro-1H-indazole-7-carboxylic acid

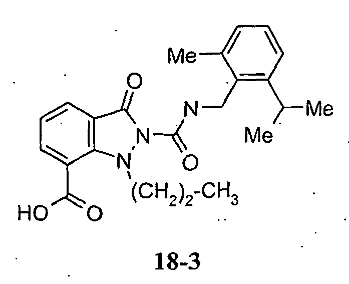

By following similar procedure as described in Part A and B of Example 85, the title compound **18-3** is obtained as a white solid (50% total yield). ESIMS *m*/*e* 410 (M+H)⁺.

### Example 87

### 1-(3-Chloro-propyl)-2-(2-isopropyl-6-methyl-benzylcarbamoyl)-3-oxo-2,3-dihydro-1H-indazole-7-carboxylic acid

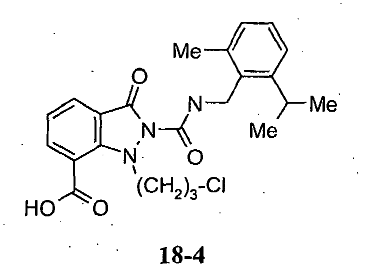

### A. Preparation of 1-(3-chloro-propyl)-2-(2-isopropyl-6-methyl-benzylcarbamoyl)-3-oxo-2,3-dihydro-1H-indazole-7-carboxylic acid benzhydryl ester

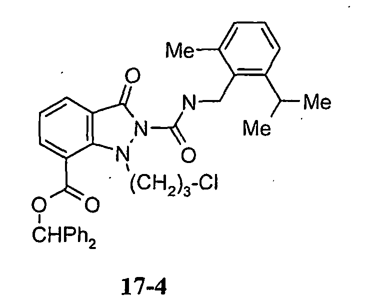

By following similar procedure as described in Part A of Example 85, the title compound **17-4** is obtained as white foam (52% total yield). ESIMS *m*/*e* 610 [(M+H)⁺, ³⁶Cl], 612 [(M+H)⁺, ³⁷Cl].

### B. Preparation of 1-(3-chloro-propyl)-2-(2-isopropyl-6-methyl-benzylcarbamoyl)-3-oxo-2,3-dihydro-1H indazole-7-carboxylic acid

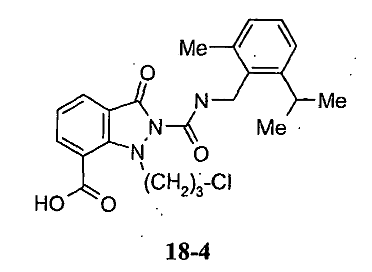

Triethylsilane (92.2 □L, 0.576 mmol)) and trifluoroacetic acid (0.222 mL, 2.88 mmol) are added successively to a stirred solution of 1-(3-chloro-propyl)-2-(2-isopropyl-6-methyl-benzylcarbamoyl)-3-oxo-2,3-dihydro-1*H*-indazole-7-carboxylic acid benzhydryl ester **17-4** (176 mg, 0.288 mmol) in anhydrous CH₂Cl₂ (3 mL) at ambient temperature under nitrogen. The resultant solution is allowed to stir for 2 hours. After concentration, chromatography on silica [gradient 0-2% HOAc in CH₃OH (8)/CH₂Cl₂ (92)] and subsequent crystallization from CH₂Cl₂/hexane, 126 mg of the title compound **18-4** is obtained as a white solid (99% yield). ESIMS *m*/*e* 444 [(M+H)⁺, ³⁵Cl], 446 [(M+H)⁺, ³⁷Cl]. Analysis for C₂₃H₂₆ClN₃O₄: calcd: C, 62.23; H, 5.90; N, 9.47; found: C, 62.01; H, 5.98; N, 9.39.

### Example 88

### 2-(2-Isopropyl-6-methyl-benzylcarbamoyl)-3-oxo-1-(prop-2-yriyl)-2,3-dihydro-1H-indazole-7-carboxylic acid

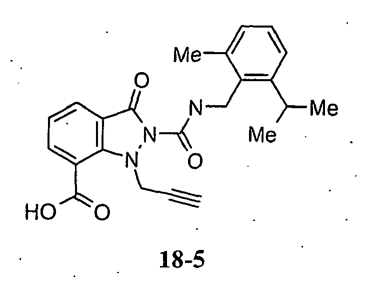

By following similar procedure as described in Part A and B of Example 87, the title compound **18-5** is obtained as a white solid (86% total yield). ESIMS *m*/*e* 406 (M+H)⁺. Analysis for C₂₃H₂₃N₃O₄·0.3H₂O: calcd: C, 67.24; H, 5.79; N, 10.23; found: C, 67.21; H, 5.67; N, 10.09.

### Example 89

### 1-Allyl-2-(2-isopropyl-6-methyl-benzylcarbamoyl)-3-oxo-2,3-dihydro-1H-indazole-7-carboxylic acid

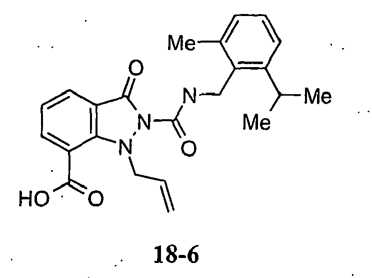

By following similar procedure as described in Part A and B of Example 87, the title compound **18-6** is obtained as a white solid (96% total yield). ESIMS *m*/*e* 408 (M+H)⁺. Analysis for C₂₃H₂₅N₃O₄·0.6H₂O: calcd: C, 67.24; H, 5.79; N, 10.23; found: C, 67.21; H, 5.67; N, 10.09.

### Example 90

### 1-Butyl-2-(2-isopropyl-6-methyl-benzylcarbamoyl)-3-oxo-2,3-dihydro-1H-indazole-7-carboxylic acid

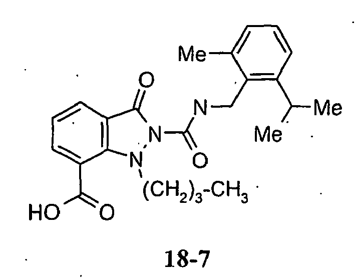

By following similar procedure as described in Part A and B of Example 87, the title compound **18-7** is obtained as a white solid (70% total yield). ESIMS *m*/*e* 424 (M+H)⁺. Analysis for C₂₄H₂₉N₃O₄: calcd: C, 68.07; H, 6.90; N, 9.92; found: C, 67.85; H, 6.87; N, 9.83.

### Example 91

### 1-Cyanomethyl-2-(2-isopropyl-6-methyl-benzylcarbamoyl)-3-oxo-2,3-dihydro-1H-indazole-7-carboxylic acid

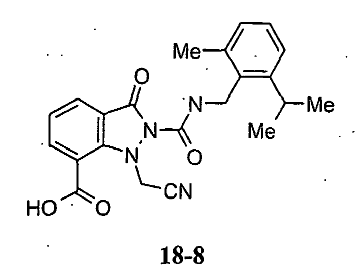

By following similar procedure as described in Part A and B of Example 87, the title compound **18-8** is obtained as a white solid (77% total yield). ESIMS *m*/*e* 407 (M+H)⁺. Analysis for C₂₂H₂₂N₄O₄: calcd: C, 65.01; H, 5.46; N, 13.78; found: C, 65.01; H, 5.43; N, 13.71.

### Example 92

### 1-Cyclopropylmethyl-2-(2-isopropyl-6-methyl-benzylcarbamoyl)-3-oxo-2,3-dihydro-1H-indazole-7-carboxylic acid

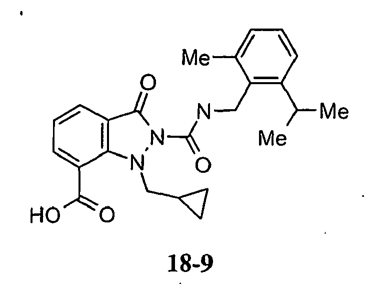

By following similar procedure as described in Part A and B of Example 87, the title compound **18-9** is obtained as a white solid (77% total yield). ESIMS *m*/*e* 422 (M+H)⁺. Analysis for C₂₄H₂₇N₃O₄·0.2H₂O: calcd: C, 67.81; H, 6.50; N, 9.88; found: C, 67.97; H, 6.56; N, 9.58.

### Example 93

### 1-Carboxymethyl-2-(2-isopropyl-6-methyl-benzylcarbamoyl)-3-oxo-2,3-dihydro-1H-indazole-7-carboxylic acid

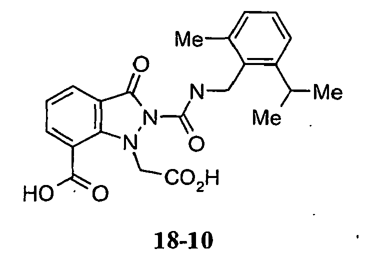

### A. Preparation of 1-tert-butoxycarbonylmethyl-2-(2-isopropyl-6-methyl-benzylcarbamoyl)-3-oxo-2,3-dihydro-1H-indazole-7-carboxylic acid benzhydryl ester

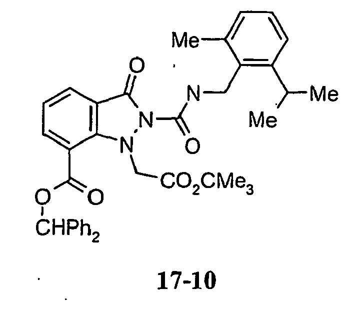

By following similar procedure as described in Part A Example 85, the title compound **17-10** is obtained as white foam (61% yield). Analysis for C₃₉H₄₁N₃O₆·0.1H₂O: calcd: C, 72.11; H, 6.39; N, 6.47; found: C, 71.93; H, 6.18; N, 6.27.

### B. Preparation of 1-carboxymethyl-2-(2-isopropyl-6-methyl-benzylcarbamoyl)-3-oxo-2,3-dihydrb-1H-indazole-7-carboxylic acid

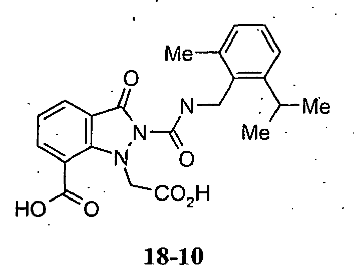

By following similar procedure as described in Part C of Example 79, the title compound **18-10** is obtained as a white solid (37% total yield). ESIMS *m*/*e* 424 (M-H)⁻. Analysis for C₂₂H₂₃N₃O₆·0.4H₂O: calcd: C, 61.08; H, 5.54; N, 9.71; found: C, 60.96; H, 5.37; N, 9.60.

### Example 94

### 2-(2-Isopropyl-6-methyl-benzylcarbamoyl)-1-methoxycarbonylmethyl-3-oxo-2,3-dihydro-1H-indazole-7-carboxylic acid

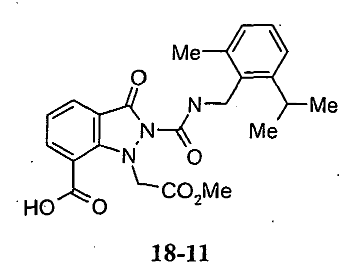

By following similar procedure as described in Part A and B of Example 87, the title compound **18-11** is obtained as a white solid. ESIMS *m*/*e* 440 (M+H)⁺. Analysis for C₂₃H₂₅N₃O₆: calcd: C, 62.86; H, 5.73; N, 9.56; found: C, 62.91; H, 5.78; N, 9.22.

### Example 95

### 1-Carbamoylmethyl-2-(2-isopropyl-6-methyl-benzylcarbamoyl)-3-oxo-2,3-dihydro-1H-indazole-7-carboxylic acid

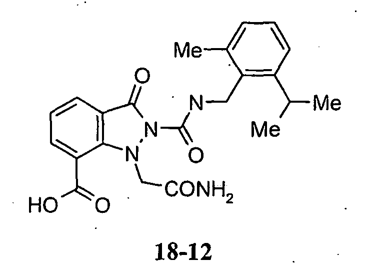

By following similar procedure as described in Part A and B of Example 87, the title compound **18-12** is obtained as a white solid (55% total yield). ESIMS *m*/*e* 425 (M+H)⁺. Analysis for C₂₂H₂₄N₄O₅·0.35H₂O: calcd: C, 61.34; H, 5.78; N, 13.01; found: C, 60.94; H, 5.38; N, 12.84.

### Example 96

### 1-Benzyl-2-(2-isopropyl-6-methyl-benzylcarbamoyl)-3-oxo-2,3-dihydro-1H-indazole-7-carboxylic acid

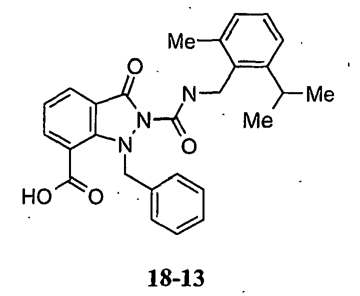

By following similar procedure as described in Part A and B of Example 85, the title compound **18-13** is obtained as a white solid (64% total yield). ESIMS *m*/*e* 458 (M+H)⁺. Analysis for C₂₇H₂₇N₃O₄: calcd: C, 70.88; H, 5.95; N, 9.18; found: C, 70.53; H, 5.93; N, 9.10.

### Example 97

### 2-(2-tert-Butyl-6-methyl-benzylcarbamoyl)-1-methyl-3-oxo-2,3-dihydro-1N-indazole-7-carboxylic acid

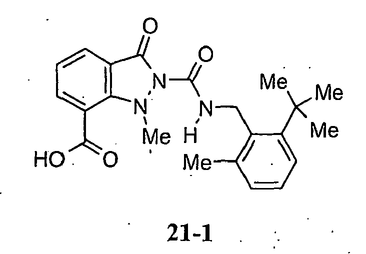

### A. Preparation of 2-(2-tert-butyl-6-methyl-benzylcarbamoyl)-3-oxo-2,3-dihydro-1H-indazole-7-carboxylic acid benzhydryl ester

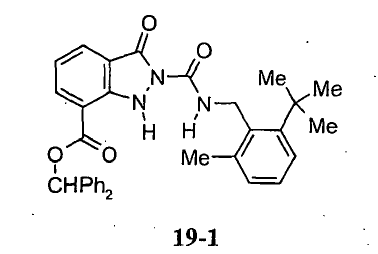

By following similar procedure as described in Part A of Example 79, the title compound **19-1** is synthesized from **12-16** as white foam (52% yield). ESIMS *m*/*e* 548 (M+H)⁺. Analysis for C₃₄H₃₃N₃O₄: calcd: C, 74.57; H, 6.07; N; 7.67; found: C, 74.20; H, 6.05; N, 7.99.

### B. Preparation of 2-(2-tert-butyl-6-methyl-benzylcarbamoyl)-1-methyl-3-exo-2,3-dihydro-1H-indazole-7-carboxylic acid benzhydryl ester

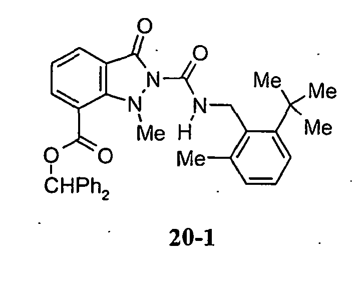

By following similar procedure as described in Part B of Example 79, the title compound **20-1** is synthesized from **19-1** as white foam (81% yield). ESIMS *m*/*e* 562 (M+H)⁺. Analysis for C₃₅H₃₅N₃O₄: calcd: C, 74.84; H, 6.28; N, 7.48; found: C, 74.78; H, 6.48; N, 7.26.

### C. Preparation of 2-(2-tert-butyl-6-methyl-benzylcarbanloyl)-1-methyl-3-oxo-2,3-dihydro-1H-indazole-7-carboxylic acid

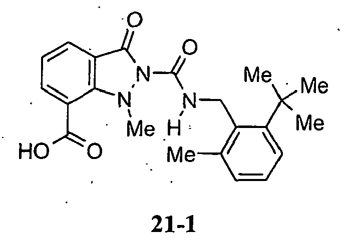

By following similar procedure as described in Part C of Example 79, the title compound **21-1** is synthesized from **20-1** as a white solid (73% yield). mp >250 °C; ESIMS *m*/*e* 396 (M+H)⁺. Analysis for C₂₂H₂₅N₃O₄: calcd: C, 66.82; H, 6.37; N, 10.63; found: C, 66.54; H, 6.38; N, 10.54.

### Example 98

### 2-(2,6-Diethyl-benzylcarbamoyl)-1-methyl-3-oxo-2,3-dihydro-1H-indazole-7-carboxylic acid

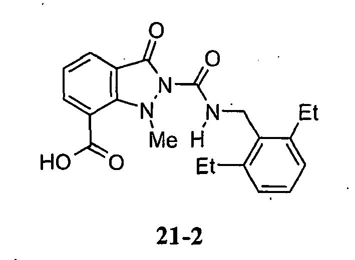

By following similar procedure as described in Part A, B and C of Example 79, the title compound **21-2** is synthesized from **12-19** as a white solid. mp >250 °C; ESIMS *m*/*e* 382 (M+H)⁺.

### Example 99

### 2-Hexylcarbamoyl-3-oxo-2,3-dihydro-1H-indazole-4-carboxylic acid methyl ester

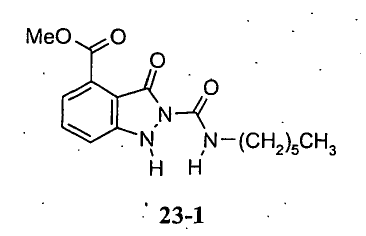

### A. Preparation of 3-oxo-2,3-dihydro-1H-indazole-4-carboxylic acid methyl ester

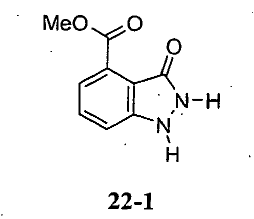

Concentrated HCl (10 mL) is added to a stirred suspension of 3-oxo-2,3-dihydro-1*H*-indazole-4-carboxylic acid **11-1** (5.02 g, 28.2 mmol) in anhydrous CH₃OH (50 mL). The resultant mixture is heated to reflux for 4 hours. The mixture is concentrated and the crude product is chromatographed on silica (gradient 3-10% CH₃OH in CHCl₃) to give 3.01 g of the title compound **22-1** (55% yield) as a white solid. mp >250 °C; ESIMS *m*/*e* 193 (M+H)⁺. Analysis for C₉H₈N₂O₃: calcd: C, 56.25; H, 4.20; N, 14.58; found: C, 56.03; H, 4.10; N, 14.41.

### B. Preparation of 2-hexylcarbamoyl-3-oxo-2,3-dihydro-1H-indazole-4-carboxylic acid methyl ester

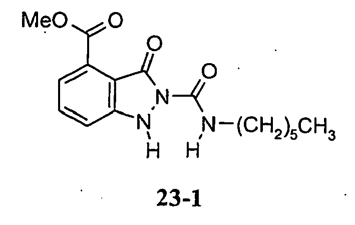

By following similar procedure as described in Example 1, the title compound **23-1** is synthesized from compound **22-1** as a white solid (42% yield). mp 63.0-65.0 °C; ESIMS *m*/*e* 320 (M+H)⁺. Analysis for C₁₆H₂₁N₃O₄·0.2H₂O: calcd: C, 59.50; H, 6.68; N, 13.01; found: C, 59.36; H, 6.37; N, 13.16.

### Example 100

### 2-Hexylcarbamoyl-3-oxo-2,3-dihydro-1H-indazole-4-carboxylic acid allyl ester

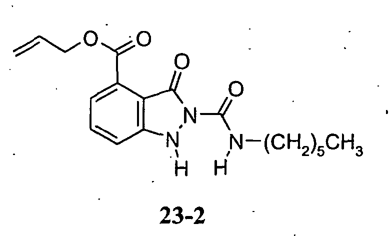

### A. Preparation of 3-oxo-2,3-dihydro-1H-indazole-4-carboxylic acid allyl ester

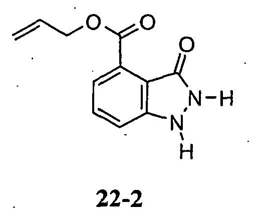

Concentrated H₂SO₄ (1 mL) is added to a stirred suspension of 3-oxo-2,3-dihydro-1*H*-indazole-4-carboxylic acid **11-1** (6.02 g, 33.8 mmol) in anhydrous allyl alcohol (100 mL). The resultant mixture is heated to reflux for 8 hours. At ambient temperature the mixture is diluted with EtOAc (150 mL) before it is washed with cold water (80 mL x 3). The organic layer is dried over MgSO₄, filtered and concentrated. After chromatography on silica (gradient 0-5% CH₃OH in CHCl₃), 4.10 g of the title compound 22-2 (56% yield) is obtained as a yellowish solid. mp 148.0-150.0 °C; ESIMS *m*/*e* 219 (M+H)⁺. Analysis for C₁₁H₁₀N₂O₃·0.1H₂O: calcd: C, 60.05; H, 4.67; N, 12.73; found: C, 60.08; H, 4.45; N, 12.58.

### B. Preparation of 2-hexylcarbamoyl-3-oxo-2,3-dihydro-1H-indazole-4-carboxylic acid allyl ester

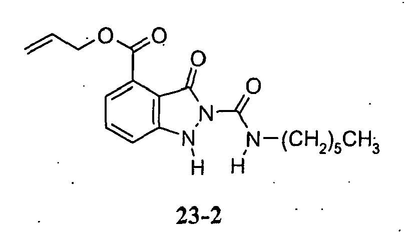

By following similar procedure as described in Example 1, the title compound **23-2** is synthesized from compound **22-2** as a white solid (67% yield). mp 67.0-69.0 °C; ESTMS *m*/*e* 346 (M+H)⁺.

### Example 101

### 2-Hexylcarbamoyl-3-oxo-2,3-dihydro-1H-indazole-7-carboxylic acid methyl ester

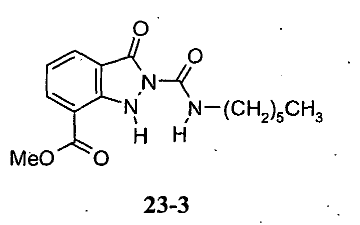

### A. Preparation of 3-oxo-2,3-dihydro-1H-indazole-7-carboxylic acid methyl ester

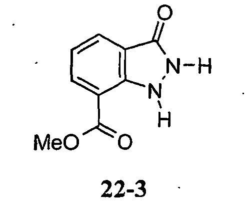

By following similar procedure as described in Part A of Example 100, the title compound **22-3** is synthesized from 3-oxo-2,3-dihydro-1*H*-indazole-7-carboxylic acid **11-4** as a yellowish solid (48% yield). mp 245.0-246.0 °C; ESIMS *m*/*e* 193 (M+H)⁺.

### B. Preparation of 2-hexylcarbamoyl-3-oxo-2,3-dihydro-1H-indazole-7-carboxylic acid methyl ester

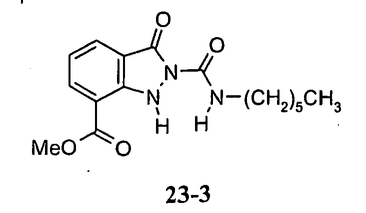

By following similar procedure as described in Example 1, the title compound **23-3** is synthesized from compound **22-3** as yellowish oil (98% yield). ESIMS *m*/*e* 320 (M+H)⁺. Analysis for C₁₆H₂₁N₃O₄: calcd: C, 60.18; H, 6.63; N, 13.16; found: C, 60.09; H, 6.57; N, 12.82.

### Example 102

### 2-(2-Isopropyl-6-methyl-benzylcarbamoyl)-3-oxo-2,3-dihydro-1H-indazole-4-carboxylic acid methyl ester

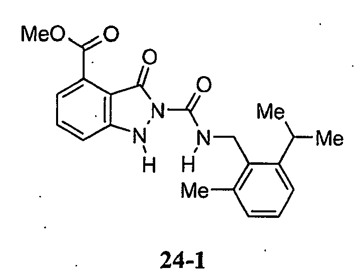

2-Isocyanatomethyl-1-isopropyl-3-methyl-benzene 7-5 (229 mg, 1.21 mmol) is added to a stirred solution of compound **22-1** (250 mg, 1.21 mmol) in anhydrous DMF (5 mL) at ambient temperature under nitrogen. The resultant mixture is allowed to stir for 3 hours. After concentration and subsequent chromatography on silica (gradient 0-1 % CH₃OH in CH₂Cl₂), 394 mg of the title compound **24-1** is obtained as a yellowish solid (85% yield). ESIMS *m*/*e* 382 (M+H)⁺. Analysis for C₂₁H₂₃N₃O₄: calcd: C, 66.13; H, 6.08; N, 11.02; found: C, 66.29; H, 6.20; N, 11.00.

### Example 103

### 2-(2-Isopropyl-6-methyl-benzylcarbamoyl)-3-oxo-2,3-dihydro-1H-indazole-7-carboxylic acid methyl ester

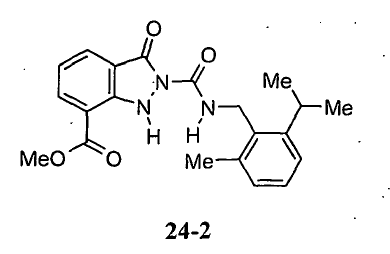

By following similar procedure as described in Example 102, the title compound **24-2** is synthesized from compound **22-3** as a yellowish solid (32% yield). ESIMS *m*/*e* 382 (M+H)⁺. Analysis for C₂₁H₂₃N₃O₄: calcd: C, 66.13; H, 6.08; N, 11.02; found: C, 66.00; H, 6.21; N, 10.89.

## Claims

1. A dihydro-1*H*-indazole-5-carboxylic acid derivative compound of formula (I) 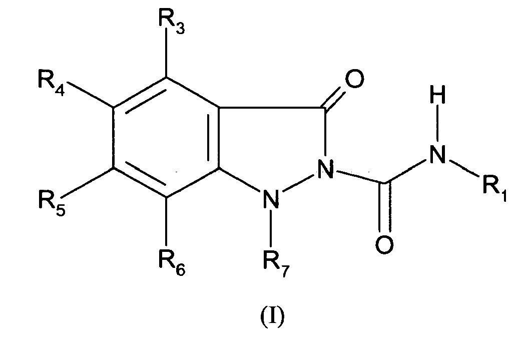 wherein;
R₁ is selected from C₅-C₁₃alkyl, C₁-C₁₂haloalkyl, C₄-C₁₂alkenyl, C₄-C₁₂lalkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₂-C₁₂alkynyl, C₂-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, with the proviso that the compound of formula (I) is not: 4,6-dichloro-3-oxo-1,3-dihydro-indazole-2-carboxylic acid phenylamide; 3-oxo-1,3-dihydro-indazole-2,4-dicarboxylic acid-2-butylamide-4-octylamide; 2-octylcarbamoyl-3-oxo-2,3-dihydro-1H-indazole-4-carboxylic acid; 3-oxo-1,3-dihydro-indazole-2-carboxylic acid dodecylamide; 3-oxo-1,3-dihydro-indazole-2-carboxylic acid octylamide; or 3-oxo-1-phenyl-1,3-dihydro-indazole-2-carboxylic acid phenylamide; and with the further proviso that when R₃, R₄, R₅, R₆ and R₇ are each hydrogen, R₁ is not phenyl, an alkyl group having up to seven carbons or an alkylphenyl group where the alkyl group has up to seven carbons.

2. The compound according to claim 1 wherein R₁ is selected from C₅-C₁₂alkyl, C₄-C₁₂alkenyl, C₃-C₇cycloalkyl, -CF₃, C₁-C₃ alkylindole, C₁-C₃alkylbenzothiophene and a substituted or unsubstituted benzyl group.

3. The compound according to claim 2 wherein the benzyl group is 2,4-, 3,5- or 2,5-disubsituted, or 2 or 4 monosubstituted.

4. The compound according to claim 3 wherein the substituent on the benzyl group is selected from methyl, ethyl, propyl, isopropyl, isobutyl, tert-butyl and pentyl.

5. The compound according to claim 1 wherein R₃, R₄, R₅ and R₆ are independently selected from hydrogen, C₁-C₄alkyl, C₂-C₄alkenyl, halo, C₁-C₄haloalkyl, phenyl and aryl.

6. The compound according to claim 1 wherein R₇ is selected from methyl, ethyl, propenyl, ethynyl, propynyl, phenyl, benzyl, and C₁-C₁₀ haloalkyl.

7. A compound according to claim 1 selected from: 3-Oxo-1,3-dihydro-indazole-2-carboxylic acid (2-ethyl-hexyl)-amide, 3-Oxo-1,3-dihydro-indazole-2-carboxylic acid (3,7-dimethyl-octa-2,6-dienyl)-amide, 3-Oxo-1,3-dihydro-indazole-2-carboxylic acid cyclohexylmethyl-amide, 1-Methyl-3-oxo-1,3-dihydro-indazole-2-carboxylic acid pentylamide, 1-Butyl-3-oxo-1,3-dihydro-indazole-2-carboxylic acid pentylamide, 1-Benzyl-3-oxo-1,3-dihydro-indazole-2-carboxylic acid pentylamide, 3-Oxo-1-phenethyl-1,3-dihydro-indazole-2-carboxylic acid pentylamide, 3-Oxo-1,3-dihydro-indazole-2-carboxylic acid 2-methyl-benzylamide, 3-Oxo-1,3-dihydro-indazole-2-carboxylic acid 3-methyl-benzylamide, 3-Oxo-1,3-dihydro-indazole-2-carboxylic acid 4-methyl-benzylamide, 3-Oxo-1,3-dihydro-indazole-2-carboxylic acid 2-trifluoromethyl-benzylamide, 3-Oxo-1,3-dihydro-indazole-2-carboxylic acid 2-ethyl-benzylamide, 3-Oxo-1,3-dihydro-indazole-2-carboxylic acid 3-ethyl-benzylamide, 3-Oxo-1,3-dihydro-indazole-2-carboxylic acid 3-methoxy-benzylamide, 3-Oxo-1,3-dihydro-indazole-2-carboxylic acid 4-amino-benzylamide, 3-Oxo-1,3-dihydro-indazole-2-carboxylic acid 4-dimethylamino-benzylamide, 3-Oxo-1,3-dihydro-indazole-2-carboxylic acid (biphenyl-3-ylmethyl)-amide, 3-Oxo-1,3-dihydro-indazole-2-carboxylic acid 2-isopropyl-6-methyl-benzylamide, 3-Oxo-1,3-dihydro-indazole-2-carboxylic acid 2,4-dimethoxy-benzylamide, 3-Oxo-1,3-dihydro-indazole-2-carboxylic acid 3,4-dihydroxy-benzylamide, 3-Oxo-1,3-dihydro-indazole-2-carboxylic acid (2,3-dihydro-benzo[1,4]dioxin-6-ylmethyl)-amide, 3-Oxo-1,3-dihydro-indazole-2-carboxylic acid 2,4,6-trimethyl-benzylamide, 3-Oxo-1,3-dihydro-indazole-2-carboxylic acid 3,4,5-trimethoxy-benzylamide, 4-Methyl-3-oxo-1,3-dihydro-indazole-2-carboxylic acid 2-methyl-benzylamide, 6-Methyl-3-oxo-1,3-dihydro-indazole-2-carboxylic acid 2-methyl-benzylamide, 4-Chloro-3-oxo-1,3-dihydro-indazole-2-carboxylic acid 2-methyl-benzylamide, 5-Chloro-3-oxo-1,3-dihydro-indazole-2-carboxylic acid 2-methyl-benzylamide, 6-Chloro-3-oxo-1,3-dihydro-indazole-2-carboxylic acid 2-methyl-benzylamide, 2-(2-Methyl-benzylcarbamoyl)-3-oxo-2,3-dihydro-1*H*-indazole-4-carboxylic acid, 2-(2-Methyl-benzylcarbamoyl)-3-oxo-2,3-dihydro-1*H*-indazole-5-carboxylic acid, 2-(2-Methyl-benzylcarbamoyl)-3-oxo-2,3-dihydro-1*H*-indazole-6-carboxylic acid, 2-(2-Methyl-benzylcarbamoyl)-3-oxo-2,3-dihydro-1*H*-indazole-7-carboxylic acid, 3-Oxo-2-(2-trifluoromethyl-benzylcarbamoyl)-2,3-dihydro-1*H*-indazole-7-carboxylic acid, 2-(2,6-Dimethyl-benzylcarbamoyl)-3-oxo-2,3-dihydro-1*H*-indazole-4-carboxylic acid, 2-(2,6-Dimethyl-benzylcarbamoyl)-3-oxo-2,3-dihydro-1*H*-indazole-7-carboxylic acid, 2-(2-Ethyl-6-methyl-benzylcarbamoyl)-3-oxo-2,3-dihydro-1*H*-indazole-4-carboxylic acid, 2-(2-Ethyl-6-methyl-benzylcarbamoyl)-3-oxo-2,3-dihydro-1*H*-indazole-5-carboxylic acid, 2-(2-Ethyl-6-methyl-benzylcarbamoyl)-3-oxo-2,3-dihydro-1*H*-indazole-6-carboxylic acid, 2-(2-Ethyl-6-methyl-benzylcarbamoyl)-3-oxo-2,3-dihydro-1*H*-indazole-7-carboxylic acid, 2-(2-Isopropyl-6-methyl-benzylcarbamoyl)-3-oxo-2,3-dihydro-1*H*-indazole-4-carboxylic acid, 2-(2-Isopropyl-6-methyl-benzylcarbamoyl)-3-oxo-2,3-dihydro-1*H*-indazole-7-carboxylic acid, 2-(2-*tert*-Butyl-6-methyl-benzylcarbamoyl)-3-oxo-2,3-dihydro-1*H*-indazole-7-carboxylic acid, 2-(4-Butyl-2-methyl-benzylcarbamoyl)-3-oxo-2,3-dihydro-1*H*-indazole-4-carboxylic acid, 2-(2,6-Diethyl-benzylcarbamoyl)-3-oxo-2,3-dihydro-1*H*-indazole-4-carboxylic acid, ' 2-(2,6-Diethyl-benzylcarbamoyl)-3-oxo-2,3-dihydro-1*H*-indazole-7-carboxylic acid, 2-(2,6-Diisopropyl-benzylcarbamoyl)-3-oxo-2,3-dihydro-1*H*-indazole-4-carboxylic acid, 2-(2,6-Diisopropyl-benzylcarbamoyl)-3-oxo-2,3-dihydro-1*H*-indazole-7-carboxylic acid, 2-(2,4,6-Trimethyl-benzylcarbamoyl)-3-oxo-2,3-dihydro-1*H*-indazole-4-carboxylic acid, 3-Oxo-2-(4-phenyl-butylcarbamoyl)-2,3-dihydro-1*H*-indazole-4-carboxylic acid, 2-(4-Cyclohexyl-butylcarbamoyl)-3-oxo-2,3-dihydro-1*H*-indazole-4-carboxylic acid, 2-Hexylcarbamoyl-3-oxo-2,3-dihydro-1*H*-indazole-4-carboxylic acid, 2-(2-Ethyl-6-methyl-benzylcarbamoyl)-1-methyl-3-oxo-2,3-dihydro-1*H*-indazole-7-carboxylic acid, 1-Ethyl-2-(2-ethyl-6-methyl-benzylcarbamoyl)-3-oxo-2,3-dihydro-1*H*-indazole-7-carboxylic acid, 2-(2-Ethyl-6-methyl-benzylcarbamoyl)-3-oxo-1-propyl-2,3-dihydro-1*H*-indazole-7-carboxylic acid, 2-(2-Isopropyl-6-methyl-benzylcarbamoyl)-1-methyl-3-oxo-2,3-dihydro-1*H*-indazole-7-carboxylic acid, 1-Ethyl-2-(2-isopropyl-6-methyl-benzylcarbamoyl)-3-oxo-2,3-dihydro-1*H*-indazole-7-carboxylic acid, 2-(2-Isopropyl-6-methyl-benzylcarbamoyl)-3-oxo-1-propyl-2,3-dihydro-1*H*-indazole-7-carboxylic acid, 1-(3-Chloro-propyl)-2-(2-isopropyl-6-methyl-benzylcarbamoyl)-3-oxo-2,3-dihydro-1*H-*indazole-7-carboxylic acid, 2-(2-Isopropyl-6-methyl-benzylcarbamoyl)-3-oxo-1-(prop-2-ynyl)-2,3-dihydro-1*H-*indazole-7-carboxylic acid, 1-Allyl-2-(2-isopropyl-6-methyl-benzylcarbamoyl)-3-oxo-2,3-dihydro-1*H*-indazole-7-carboxylic acid, 1-Butyl-2-(2-isopropyl-6-methyl-benzylcarbamoyl)-3-oxo-2,3-dihydro-1*H*-indazole-7-carboxylic acid, 1-Benzyl-2-(2-isopropyl-6-methyl-benzylcarbamoyl)-3-oxo-2,3-dihydro-1*H*-indazole-7-carboxylic acid, 2-(2-*tert*-Butyl-6-methyl-benzylcarbamoyl)-1-methyl-3-oxo-2,3-dihydro-1*H*-indazole-7-carboxylic acid, 2-(2,6-Diethyl-benzylcarbamoyl)-1-methyl-3-oxo-2,3-dihydro-1*H*-indazole-7-carboxylic acid, 2-Hexylcarbamoyl-3-oxo-2,3-dihydro-1*H*-indazole-4-carboxylic acid methyl ester, 2-Hexylcarbamoyl-3-oxo-2,3-dihydro-1*H*-indazole-7-carboxylic acid methyl ester, 2-(2-Isopropyl-6-methyl-benzylcarbamoyl)-3-oxo-2,3-dihydro-1*H*-indazole-4-carboxylic acid methyl ester, and 2-(2-Isopropyl-6-methyl-benzylcarbamoyl)-3-oxo-2,3-dihydro-1*H*-indazole-7-carboxylic acid methyl ester.

8. The compound according to claim 1 wherein the compound is represented by the formulae (C1), (C2), (C3), (C4), or (C5):

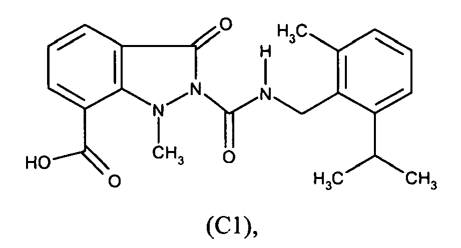

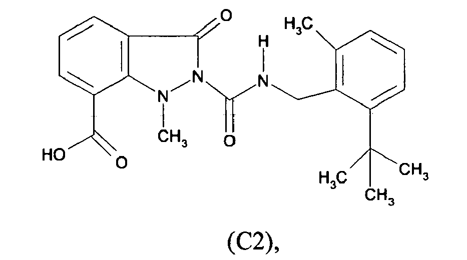

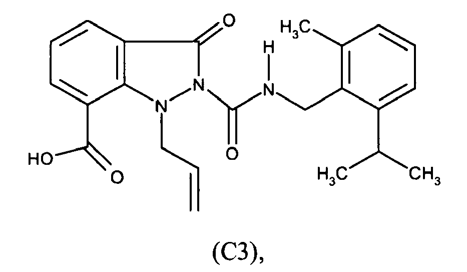

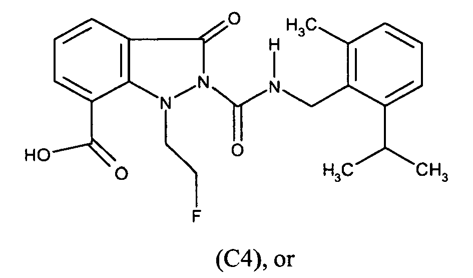

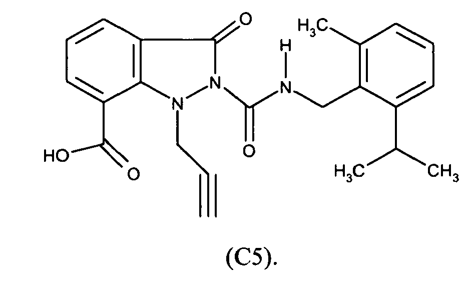

9. A compound of formula (I) 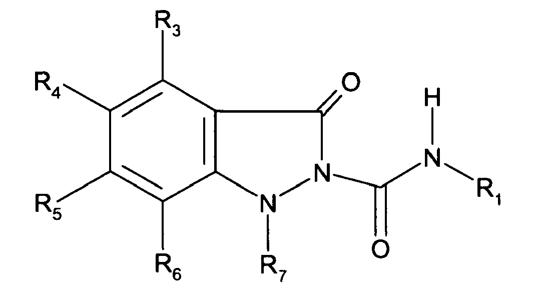 wherein R¹ and R³ to R⁷ are selected to provide a compound selected from: 1-(2-Methoxy-ethyl)-3-oxo-1,3-dihydro-indazole-2-carboxylic acid hexylamide, (2-Hexylcarbamoyl-3-oxo-2,3-dihydro-indazol-1-yl)-acetic acid methyl ester, 3-Oxo-1,3-dihydro-indazole-2-carboxylic acid 3-fluoro-benzylamide, 3-Oxo-1,3-dihydro-indazole-2-carboxylic acid 3-chloro-benzylamide, 3-Oxo-1,3-dihydro-indazole-2-carboxylic acid 3-trifluoromethoxy-benzylamide, 3-Oxo-1,3-dihydro-indazole-2-carboxylic acid 2-methylsulfanyl-benzylamide, 3-Oxo-1,3-dihydro-indazole-2-carboxylic acid (naphthalen-1-ylmethyl)-amide, 3-Oxo-1,3-dihydro-indazole-2-carboxylic acid (4-methyl-naphthalen-1-ylmethyl)-amide, 3-Oxo-1,3-dihydro-indazole-2-carboxylic acid 5-chloro-2-methyl-benzylamide, 3-Oxo-1,3-dihydro-indazole-2-carboxylic acid 2,4-dichloro-benzylamide, 3-Oxo-1,3-dihydro-indazole-2-carboxylic acid 5-chloro-2-methoxy-benzylamide 3-Oxo-1,3-dihydro-indazole-2-carboxylic acid 4,6-dichloro-2-methyl-benzylamide, 3-Oxo-1,3-dihydro-indazole-2-carboxylic acid (4-phenyl-but-3-enyl)-amide, 7-Methoxy-3-oxo-1,3-dihydro-indazole-2-carboxylic acid 2-methyl-benzylamide, 5-Hydroxy-3-oxo-1,3-dihydro-indazole-2-carboxylic acid 2-methyl-benzylamide, 5-Nitro-3-oxo-1,3-dihydro-indazole-2-carboxylic acid 2-methyl-benzylamide, 2-(5-Fluoro-2-methyl-benzylcarbamoyl)-3-oxo-2,3-dihydro-1*H*-indazole-4-carboxylic acid, 2-(5-Fluoro-2-methyl-benzylcarbamoyl)-3-oxo-2,3-dihydro-1*H*-indazole-7-carboxylic acid, 2-[(4-Methyl-naphthalen-1-ylmethyl)-carbamoyl]-3-oxo-2,3-dihydro-1*H*-indazole-4-carboxylic acid, 3-Oxo-2-(3-phenoxy-propylcarbamoyl)-2,3-dihydro-1*H*-indazole-4-carboxylic acid, 1-Carboxymethyl-2-(2-ethyl-6-methyl-benzylcarbamoyl)-3-oxo-2,3-dihydro-1*H*-indazole-7-carboxylic acid, 2-(2-Ethyl-6-methyl-benzylcarbamoyl)-1-methoxycarbonylmethyl-3-oxo-2,3-dihydro-1*H-*indazole-7-carboxylic acid, 1-Cyanomethyl-2-(2-isopropyl-6-methyl-benzylcarbamoyl)-3-oxo-2,3-dihydro-1*H-*indazole-7-carboxylic acid, 1-Cyclopropylmethyl-2-(2-isopropyl-6-methyl-benzylcarbamoyl)-3-oxo-2,3-dihydro-1*H-*indazole-7-carboxylic acid, 1-Carboxymethyl-2-(2-isopropyl-6-methyl-benzylcarbamoyl)-3-oxo-2,3-dihydro-1*H-*indazole-7-carboxylic acid, 2-(2-Isopropyl-6-methyl-benzylcarbamoyl)-1-methoxycarbonylmethyl-3-oxo-2,3-dihydro-1*H*-indazole-7-carboxylic acid, 1-Carbamoylmethyl-2-(2-isopropyl-6-methyl-benzylcarbamoyl)-3-oxo-2,3-dihydro-1*H-*indazole-7-carboxylic acid, 2-Hexylcarbamoyl-3-oxo-2,3-dihydro-1*H*-indazole-4-carboxylic acid allyl ester, and 2-Hexylcarbamoyl-3-oxo-2,3-dihydro-1*H*-indazole-7-carboxylic acid allyl ester.

10. A pharmaceutical formulation comprising a dihydro-1*H*-indazole-5-carboxylic acid derivative compound according to any one of claims 1 to 9 together with a pharmaceutically acceptable carrier or diluent.

11. A compound according to claim 9 for use as a medicament.

12. Use of a dihydro-1*H*-indolazole-5-carboxylic acid derivative compound according to claim 9 for the manufacture of a medicament for the treatment of a mammal to alleviate the pathological effects of low HDL.

13. Use of a dihydro-1*H*-indazole-5-carboxylic acid derivative compound according to claim 9 for the manufacture of a medicament for the treatment or prevention of hepatic lipase and/or endothelial lipase-mediated disease.

14. A dihydro-1H-indazole-5-carboxylic acid derivative compound of formula (I) 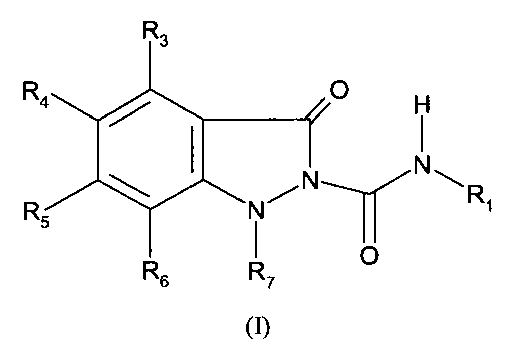 wherein;
R₁ is selected from the C₅-C₁₃alkyl, C₁-C₁₂haloalkyl, C₄-C₁₂alkenyl, C₄-C₁₂alkynyl, C₁-C₅alkylC₃-C₈Cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloatkyl, C₂-C₈alkenyl, C₁-C₈alkynyl, phenyl, benzyl, hydroxy, C₁-C₅ alkoxy, -(CH₂)ₘCOOC₁-C₅alkyl, -(CH₂)ₘNR^{a}R^{b}, and C₁-C₄akylC₃-C₈cycloalkyl; wherein R^{a} and R^{b} are independently selected from hydrogen, C₁-C₈alky, C₂-C₈alkenyl, C₂-C₈alkynyl, and C₁-C₅alkylC₃-C₈cycloalkyl;
R₃, R₄, R₅, and R₆ are independently selected from hydrogen, C₁-C₁₂alky" C₂-C₁₂haloalkyl, C₂-C₁₁alkenyl, C₁-C₁₁alkynyl, C₁-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₂)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₁-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for use as a medicament.

15. Use of a dihydro-1H-indazole-5-carboxylic acid derivative compound of formula (I) 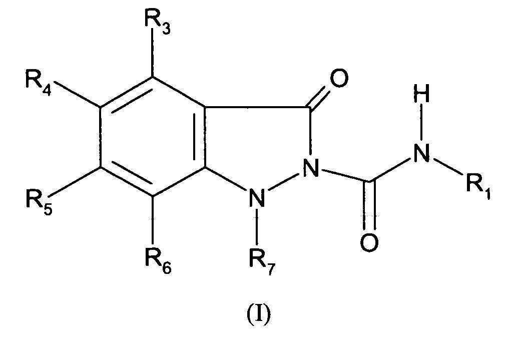 wherein;
R₁ is selected from C₅-C₁₃alkyl, C₁-C₁₂haloalkyl, C₄-C₁₂alkenyl, C₄-C₁₂alkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloaLkyl, C₂-C₈alkenyl, C₂-C₈alkynyl, phenyl, benzyl, hydroxy, C₁-C₅alkoxy, -(CH₂)ₘCOOC₁-C₅atkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈atkenyl, C₂-C₈alkynyl, and C₁-C₅alkylC₃-C₈cycloalkyl;
R₃, R₄, R₅, and R₆ are independently selected from hydrogen, C₁-C₁₂alkyl, C₂-C₁₂haloalkyl, C₂-C₁₂alkenyl, C₁-C₁₂alkynyl, C₁-C₁₂alkylaryl, C₁C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₁)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloakyl; and wherein m is 0, 1, 2, or 3;
R₇ is selected from hydrogen, C₁-C₁₀alkyl, C₁-C₁₀haloalkyl, C₁-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for the manufacture of a medicament for the treatment of a mammal to alleviate the pathological effects of low HDL.

16. Use of a dihydro-1H-indazole-5-carboxylic acid derivative compound of formula (I) 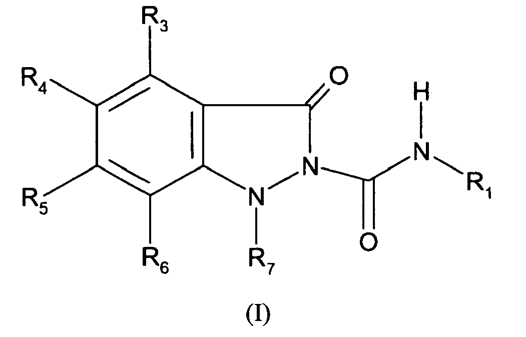 wherein;
R₁ is selected from C₅-C₁₃alkyl, C₁-C₁₂haloalkyl_{,} C₄-C₁₂alkenyl, C₄-C₁₂alkynyl, C₁-C₅alkylC₃-C₈cycloalkyl, C₃-C₈cycloalkyl, C₁-C₅alkylheterocyclic, benzyl and aryl, wherein the cycloalkyl, heterocyclic, benzyl and aryl substituents are optionally substituted with one to three substituents independently selected from C₁-C₈alkyl, C₁-C₈haloalkyl, C₂-C₈alkenyl, C₂-C₈alkynyl, phenyl, benzyl, hydroxy, C₁-C₅alkoxy, -(CH₂)ₘCOOC₁-C₅alkyl, -(CH₂)ₘNR^{a}R^{b}, and C₁-C₄alkylC₃-C₈cycloalkyl; wherein R^{a} and R^{b} are independently selected from hydrogen, C₁-C₈alkyl, C₂-C₈alkenyl, C₂-C₈alkynyl, and C₁-C₆alkylC₃-C₈cycloalkyl;
R₃, R₄, R₅, and R₆ are independently selected from hydrogen, C₁-C₁₂alkyl, C₂-C₁₁haloalkyl, C₂-C₁₂alkenyl, C₁-C₁₂alkynyl, C₂-C₁₂alkylaryl, C₁-C₁₂alkylcyclohexyl, C₁-C₁₂alkylcyclopentyl, C₁-C₁₂alkylheterocyclic, -(CH₂)ₘCOOH, -(CH₂)ₘCO(C₁-C₁₀)alkyl, -(CH₂)ₘ COO(C₁-C₁₀)alkyl, -(CH₁)ₘCOO(C₁-C₁₀)alkylaryl, C₁-C₁₀alkylamino, halo, -(CH₂)ₘCONR^{a}R^{b}, phenyl, benzyl or aryl wherein each of the phenyl, benzyl or aryl groups is optionally substituted with one to three groups independently selected from C₁-C₈alkyl, C₁-C₈haloakl, C₂-C₈alkenyl, C₂-C₈alkynyl, phenyl, benzyl, hydroxy, C₁-C₅alkoxy, -(CH₂)ₘCOOC₁-C₅alkyl, and C₁-C₄alkylC₃-C₈cycloalkyl; and wherein m is 0, 1, 2, or 3;
R₇ is selected from hydrogen, C₁-C₁₀alkyl, C₁-C₁₀haloalkyl, C₂-C₁₀alkenyl, C₂-C₁₀alkynyl, C₁-C₆alkylaryl, C₁-C₆alkylcyclohexyl, C₁-C₆alkylcyclopentyl, C₁-C₆alkylheterocyclic, or aryl; or a pharmaceutically acceptable salt or solvate thereof, for the manufacture of a medicament for the treatment or prevention of hepatic lipase and/or endothelial lipase-mediated disease.

## Patentansprüche

1. Dihydro-1H-indazol-5-carbonsäurederivatverbindung der Formel (I) 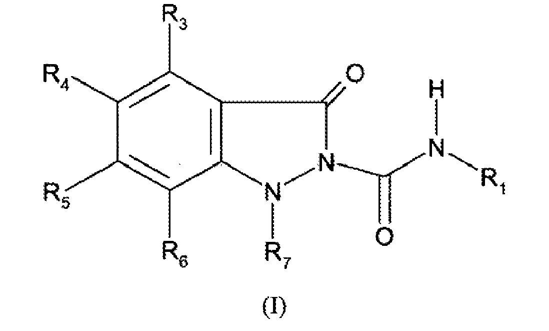 worin
R₁ aus C₅-C₁₃-Alkyl, C₁-C₁₂-Halogenalkyl, C₄-C₁₂-Alkenyl, C₄-C₁₂-Alkinyl, C₁-C₅-Alkyl-C₃-C₈-cycloalkyl, C₃-C₈-Cycloalkyl, C₁-C₅-Alkylheterocyclyl, Benzyl und Aryl ausgewählt ist, wobei der Cycloalkylsubstituent, heterocyclische Substituent, Benzylsubstituent und Arylsubstituent optional mit einem bis drei Substituenten substituiert sind, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ-COOC₁-C₅-Alkyl, -(CH₂)ₘ-NR^{a}R^{b} und C₁-C₄-Alkyl-C₃-C₈-cycloalkyl ausgewählt sind; worin R^{a} und R^{b} unabhängig voneinander aus Wasserstoff, C₁-C₈-Alkyl, C₂-C₈-Alkenyl, C₂-C₈-Alkinyl und C₁-C₅-Alkyl-C₃-C₈-cycloalkyl ausgewählt sind;
R₃, R₄, R₅ und R₆ unabhängig voneinander aus Wasserstoff, C₁-C₁₂-Alkyl, C₂-C₁₂-Halogenalkyl, C₂-C₁₂-Alkenyl, C₂-C₁₂-Alkinyl, C₂-C₁₂-Alkylaryl, C₁-C₁₂-Alkylcyclohexyl, C₁-C₁₂-Alkylcyclopentyl, C₁-C₁₂-Alkylheterocyclyl, -(CH₂)ₘ-COOH, -(CH₂)ₘ-CO(C₁-C₁₀)-Alkyl, -(CH₂)ₘ-COO(C₁-C₁₀)-Alkyl, -(CH₂)ₘCOO(C₁-C₁₀)-Alkylaryl, C₁-C₁₀-Alkylamino, Halogen, -(CH₂)ₘ-CONR^{a}R^{b}, Phenyl, Benzyl oder Aryl ausgewählt sind, wobei jede der Phenyl-, Benzyl- oder Arylgruppen optional mit einer bis drei Gruppen substituiert ist, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ-COOC₁-C₅-Alkyl und C₁-C₄-Alkyl-C₃-C₈-cycloalkyl ausgewählt sind; und wobei m 0, 1, 2 oder 3 bedeutet;
R₇ aus Wasserstoff, C₁-C₁₀-Alkyl, C₁-C₁₀-Halogenalkyl, C₂-C₁₀-Alkenyl, C₂-C₁₀-Alkinyl, C₁-C₆-Alkylaryl, C₁-C₆-Alkylcyclohexyl, C₁-C₆-Alkylcyclopentyl, C₁-C₆-Alkylheterocyclyl oder Aryl ausgewählt ist; oder ein pharmazeutisch akzeptables Salz oder Solvat hiervon, wobei gilt, dass die Verbindungen der Formel (I) nicht:
4,6-Dichlor-3-oxo-1,3-dihydro-indazol-2-carbonsäurephenylamid;
3-Oxo-1,3-dihydro-indazol-2,4-dicarbonsäure-2-butylamid-4-octylamid;
2-Octylcarbamoyl-3-oxo-2,3-dihydro-1H-indazol-4-carbonsäure;
3-Oxo-1,3-dihydro-indazol-2-carbonsäure-dodecylamid;
3-Oxo-1,3-dihydro-indazol-2-carbonsäure-octylamid oder
3-Oxo-1-phenyl-1,3-dihydro-indazol-2-carbonsäure-phenylamid sind; wobei ferner gilt, dass, wenn R₃, R₄, R₅, R₆ und R₇ jeweils für Wasserstoff stehen, R₁ nicht Phenyl, eine Alkylgruppe mit bis zu sieben Kohlenstoffatomen oder eine Alkylphenylgruppe, worin die Alkylgruppe bis zu sieben Kohlenstoffatome aufweist, bedeutet.

2. Verbindung nach Anspruch 1, worin R₁ aus C₅-C₁₂-Alkyl, C₄-C₁₂-Alkenyl, C₃-C₇-Cycloalkyl, -CF₃, C₁-C₃-Alkylindol, C₁-C₃-Alkylbenzothiophen und einer substituierten oder nicht substituierten Benzylgruppe ausgewählt ist.

3. Verbindung nach Anspruch 2, worin die Benzylgruppe 2,4-, 3,5- oder 2,5-disubstituiert oder 2- oder 4-monosubstituiert ist.

4. Verbindung nach Anspruch 3, worin der Substituent an der Benzylgruppe aus Methyl, Ethyl, Propyl, Isopropyl, Isobutyl, tert-Butyl und Pentyl ausgewählt ist.

5. Verbindung nach Anspruch 1, worin R₃, R₄, R₅ und R₆ unabhängig voneinander aus Wasserstoff, C₁-C₄-Alkyl, C₂-C₄-Alkenyl, Halogen, C₂-C₄-Halogenalkyl, Phenyl und Aryl ausgewählt sind.

6. Verbindung nach Anspruch 1, worin R₇ aus Methyl, Ethyl, Propenyl, Ethinyl, Propinyl, Phenyl, Benzyl und C₁-C₁₀-Halogenalkyl ausgewählt ist.

7. Verbindung nach Anspruch 1, ausgewählt aus:
3-Oxo-1,3-dihydro-indazol-2-carbonsäure-(2-ethyl-hexyl)-amid,
3-Oxo-1,3-dihydro-indazol-2-carbonsäure-(3,7-dimethyl-octa-2,6-dienyl)-amid,
3-Oxo-1,3-dihydro-indazol-2-carbonsäure-cyclohexylmethyl-amid,
1-Methyl-3-oxo-1,3-dihydro-indazol-2-carbonsäure-pentylamid,
1-Butyl-3-oxo-1,3-dihydro-indazol-2-carbonsäure-pentylamid,
1-Benzyl-3-oxo-1,3-dihydro-indazol-2-carbonsäure-pentylamid,
3-Oxo-1-phenethyl-1,3-dihydro-indazol-2-carbonsäure-pentylamid,
3-Oxo-1,3-dihydro-indazol-2-carbonsäure-2-methyl-benzylamid,
3-Oxo-1,3-dihydro-indazol-2-carbonsäure-3-methyl-benzylamid,
3-Oxo-1,3-dihydro-indazol-2-carbonsäure-4-methyl-benzylamid,
3-Oxo-1,3-dihydro-indazol-2-carbonsäure-2-trifluormethyl-benzylamid,
3-Oxo-1,3-dihydro-indazol-2-carbonsäure-2-ethyl-benzylamid,
3-Oxo-1,3-dihydro-indazol-2-carbonsäure-3-ethyl-benzylamid,
3-Oxo-1,3-dihydro-indazol-2-carbonsäure-3-methoxy-benzylamid,
3-Oxo-1,3-dihydro-indazol-2-carbonsäure-4-amino-benzylamid,
3-Oxo-1,3-dihydro-indazol-2-carbonsäure-4-dimethylamino-benzylamid,
3-Oxo-1,3-dihydro-indazol-2-carbonsäure-(biphenyl-3-ylmethyl)-amid,
3-Oxo-1,3-dihydro-indazol-2-carbonsäure-2-isopropyl-6-methyl-benzylamid,
3-Oxo-1,3-dihydro-indazol-2-carbonsäure-2,4-dimethoxy-benzylamid,
3-Oxo-1,3-dihydro-indazol-2-carbonsäure-3,4-dihydroxy-benzylamid,
3-Oxo-1,3-dihydro-indazol-2-carbonsäure-(2,3-dihydro-benzo[1,4]dioxin-6-ylmethyl)-amid,
3-Oxo-1,3-dihydro-indazol-2-carbonsäure-2,4,6-trimethyl-benzylamid,
3-Oxo-1,3-dihydro-indazol-2-carbonsäure-3,4,5-trimethoxy-benzylamid,
4-Methyl-3-oxo-1,3-dihydro-indazol-2-carbonsäure-2-methyl-benzylamid,
6-Methyl-3-oxo-1,3-dihydro-indazol-2-carbonsäure-2-methyl-benzylamid,
4-Chlor-3-oxo-1,3-dihydro-indazol-2-carbonsäure-2-methyl-benzylamid,
5-Chlor-3-oxo-1,3-dihydro-indazol-2-carbonsäure-2-methyl-benzylamid,
6-Chlor-3-oxo-1,3-dihydro-indazol-2-carbonsäure-2-methyl-benzylamid,
2-(2-Methyl-benzylcarbamoyl)-3-oxo-2,3-dihydro-1H-indazol-4-carbonsäure,
2-(2-Methyl-benzylcarbamoyl)-3-oxo-2,3-dihydro-1H-indazol-5-carbonsäure,
2-(2-Methyl-benzylcarbamoyl)-3-oxo-2,3-dihydro-1H-indazol-6-carbonsäure,
2-(2-Methyl-benzylcarbamoyl)-3-oxo-2,3-dihydro-1H-indazol-7-carbonsäure,
3-Oxo-2-(2-trifluormethyl-benzylcarbamoyl)-2,3-dihydro-1H-indazol-7-carbonsäure,
2-(2,6-Dimethyl-benzylcarbamoyl)-3-oxo-2,3-dihydro-1H-indazol-4-carbonsäure,
2-(2,6-Dimethyl-benzylcarbamoyl)-3-oxo-2,3-dihydro-1H-indazol-7-carbonsäure,
2-(2-Ethyl-6-methyl-benzylcarbamoyl)-3-oxo-2,3-dihydro-1H-indazol-4-carbonsäure,
2-(2-Ethyl-6-methyl-benzylcarbamoyl)-3-oxo-2,3-dihydro-1H-indazol-5-carbonsäure,
2-(2-Ethyl-6-methyl-benzylcarbamoyl)-3-oxo-2,3-dihydro-1H-indazol-6-carbonsäure,
2-(2-Ethyl-6-methyl-benzylcarbamoyl)-3-oxo-2,3-dihydro-1H-indazol-7-carbonsäure,
2-(2-Isopropyl-6-methyl-benzylcarbamoyl)-3-oxo-2,3-dihydro-1H-indazol-4-carbonsäure,
2-(2-Isopropyl-6-methyl-benzylcarbamoyl)-3-oxo-2,3-dihydro-1H-indazol-7-carbonsäure,
2-(2-tert-Butyl-6-methyl-benzylcarbamoyl)-3-oxo-2,3-dihydro-1H-indazol-7-carbonsäure,
2-(4-Butyl-2-methyl-benzylcarbamoyl)-3-oxo-2,3-dihydro-1H-indazol-4-carbonsäure,
2-(2,6-Diethyl-benzylcarbamoyl)-3-oxo-2,3-dihydro-1H-indazol-4-carbonsäure,
2-(2,6-Diethyl-benzylcarbamoyl)-3-oxo-2,3-dihydro-1H-indazol-7-carbonsäure,
2-(2,6-Diisopropyl-benzylcarbamoyl)-3-oxo-2,3-dihydro-1H-indazol-4-carbonsäure,
2-(2,6-Diisopropyl-benzylcarbamoyl)-3-oxo-2,3-dihydro-1H-indazol-7-carbonsäure,
2-(2,4,6-Trimethyl-benzylcarbamoyl)-3-oxo-2,3-dihydro-1H-indazol-4-carbonsäure,
3-Oxo-2-(4-phenyl-butylcarbamoyl)-2,3-dihydro-1H-indazol-4-carbonsäure,
2-(4-Cyclohexyl-butylcarbamoyl)-3-oxo-2,3-dihydro-1H-indazol-4-carbonsäure,
2-Hexylcarbamoyl-3-oxo-2,3-dihydro-1H-indazol-4-carbonsäure,
2-(2-Ethyl-6-methyl-benzylcarbamoyl)-1-methyl-3-oxo-2,3-dihydro-1H-indazol-7-carbonsäure,
1-Ethyl-2-(2-ethyl-6-methyl-benzylcarbamoyl)-3-oxo-2,3-dihydro-1H-indazol-7-carbonsäure,
2-(2-Ethyl-6-methyl-benzylcarbamoyl)-3-oxo-1-propyl-2,3-dihydro-1H-indazol-7-carbonsäure,
2-(2-Isopropyl-6-methyl-benzylcarbamoyl)-1-methyl-3-oxo-2,3-dihydro-1H-indazol-7-carbonsäure,
1-Ethyl-2-(2-isopropyl-6-methyl-benzylcarbamoyl)-3-oxo-2,3-dihydro-1H-indazol-7-carbonsäure,
2-(2-Isopropyl-6-methyl-benzylcarbamoyl)-3-oxo-1-propyl-2,3-dihydro-1H-indazol-7-carbonsäure,
1-(3-Chlor-propyl)-2-(2-isopropyl-6-methyl-benzylcarbamoyl)-3-oxo-2,3-dihydro-1H-indazol-7-carbonsäure,
2-(2-Isopropyl-6-methyl-benzylcarbamoyl)-3-oxo-1-(prop-2-inyl)-2,3-dihydro-1H-indazol-7-carbonsäure,
1-Allyl-2-(2-isopropyl-6-methyl-benzylcarbamoyl)-3-oxo-2,3-dihydro-1H-indazol-7-carbonsäure,
1-Butyl-2-(2-isopropyl-6-methyl-benzylcarbamoyl)-3-oxo-2,3-dihydro-1H-indazol-7-carbonsäure,
1-Benzyl-2-(2-isopropyl-6-methyl-benzylcarbamoyl)-3-oxo-2,3-dihydro-1H-indazol-7-carbonsäure,
2-(2-tert-Butyl-6-methyl-benzylcarbamoyl)-1-methyl-3-oxo-2,3-dihydro-1H-indazol-7-carbonsäure,
2-(2,6-Diethyl-benzylcarbamoyl)-1-methyl-3-oxo-2,3-dihydro-1H-indazol-7-carbonsäure,
2-Hexylcarbamoyl-3-oxo-2,3-dihydro-1H-indazol-4-carbonsäure-methylester,
2-Hexylcarbamoyl-3-oxo-2,3-dihydro-1H-indazol-7-carbonsäure-methylester,
2-(2-Isopropyl-6-methyl-benzylcarbamoyl)-3-oxo-2,3-dihydro-1H-indazol-4-carbonsäuremethylester und
2-(2-Isopropyl-6-methyl-benzylcarbamoyl)-3-oxo-2,3-dihydro-1H-indazol-7-carbonsäuremethylester.

8. Verbindung nach Anspruch 1, worin die Verbindung durch die Formeln (C1), (C2), (C3), (C4) oder (C5) wiedergegeben wird:

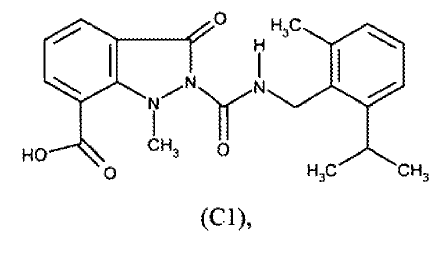

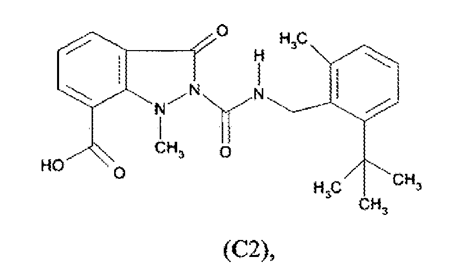

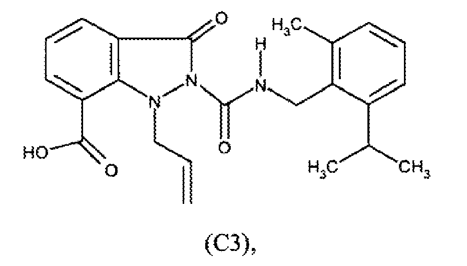

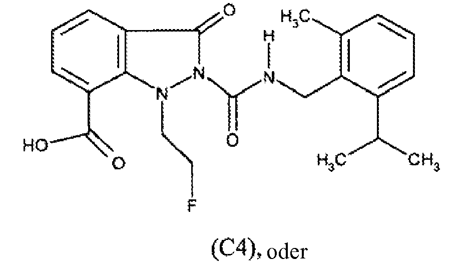

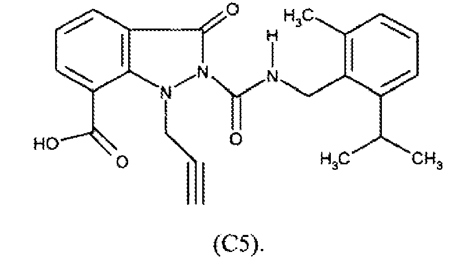

9. Verbindung der Formel (I) 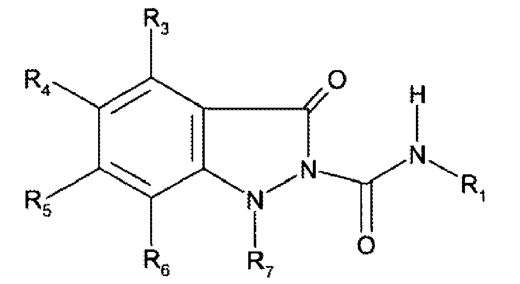 worin R₁ und R₃ bis R₇ so gewählt sind, dass sie eine Verbindung liefern, die aus den folgenden ausgewählt ist:
1-(2-Methoxy-ethyl)-3-oxo-1,3-dihydro-indazol-2-carbonsäure-hexylamid,
(2-Hexylcarbamoyl-3-oxo-2,3-dihydro-indazol-1-yl)-essigsäure-methylester,
3-Oxo-1,3-dihydro-indazol-2-carbonsäure-3-fluor-benzylamid,
3-Oxo-1,3-dihydro-indazol-2-carbonsäure-3-chlor-benzylamid,
3-Oxo-1,3-dihydro-indazol-2-carbonsäure-3-trifluormethoxy-benzylamid,
3-Oxo-1,3-dihydro-indazol-2-carbonsäure-2-methylsulfanyl-benzylamid,
3-Oxo-1,3-dihydro-indazol-2-carbonsäure-(naphthalin-1-ylmethyl)-amid,
3-Oxo-1,3-dihydro-indazol-2-carbonsäure-(4-methyl-naphthalin-1-ylmethyl)-amid,
3-Oxo-1,3-dihydro-indazol-2-carbonsäure-5-chlor-2-methyl-benzylamid,
3-Oxo-1,3-dihydro-indazol-2-carbonsäure-2,4-dichlor-benzylamid,
3-Oxo-1,3-dihydro-indazol-2-carbonsäure-5-chlor-2-methoxy-benzylamid,
3-Oxo-1,3-dihydro-indazol-2-carbonsäure-4,6-dichlor-2-methyl-benzylamid,
3-Oxo-1,3-dihydro-indazol-2-carbonsäure-(4-phenyl-but-3-enyl)-amid,
7-Methoxy-3-oxo-1,3-dihydro-indazol-2-carbonsäure-2-methyl-benzylamid,
5-Hydroxy-3-oxo-1,3-dihydro-indazol-2-carbonsäure-2-methyl-benzylamid,
5-Nitro-3-oxo-1,3-dihydro-indazol-2-carbonsäure-2-methyl-benzylamid,
2-(5-Fluor-2-methyl-benzylcarbamoyl)-3-oxo-2,3-dihydro-1H-indazol-4-carbonsäure,
2-(5-Fluor-2-methyl-benzylcarbamoyl)-3-oxo-2,3-dihydro-1H-indazol-7-carbonsäure,
2-[(4-Methylnaphthalin-1-ylmethyl)-carbamoyl]-3-oxo-2,3-dihydro-1H-indazol-4-carbonsäure,
3-Oxo-2-(3-phenoxy-propylcarbamoyl)-2,3-dihydro-1H-indazol-4-carbonsäure,
1-Carboxymethyl-2-(2-ethyl-6-methyl-benzylcarbamoyl)-3-oxo-2,3-dihydro-1H-indazol-7-carbonsäure,
2-(2-Ethyl-6-methyl-benzylcarbamoyl)-1-methoxycarbonylmethyl-3-oxo-2,3-dihydro-1H-indazol-7-carbonsäure,
1-Cyanomethyl-2-(2-isopropyl-6-methyl-benzylcarbamoyl)-3-oxo-2,3-dihydro-1H-indazol-7-carbonsäure,
1-Cyclopropylmethyl-2-(2-isopropyl-6-methyl-benzylcarbamoyl)-3-oxo-2,3-dihydro-1H-indazol-7-carbonsäure,
1-Carboxymethyl-2-(2-isopropyl-6-methyl-benzylcarbamoyl)-3-oxo-2,3-dihydro-1H-indazol-7-carbonsäure,
2-(2-Isopropyl-6-methyl-benzylcarbamoyl)-1-methoxycarbonylmethyl-3-oxo-2,3-dihydro-1H-indazol-7-carbonsäure,
1-Carbamoylmethyl-2-(2-isopropyl-6-methyl-benzylcarbamoyl)-3-oxo-2,3-dihydro-1H-indazol-7-carbonsäure,
2-Hexylcarbamoyl-3-oxo-2,3-dihydro-1H-indazol-4-carbonsäure-allylester und
2-Hexylcarbamoyl-3-oxo-2,3-dihydro-1H-indazol-7-carbonsäure-allylester.

10. Pharmazeutische Formulierung, die eine Dihydro-1H-indazol-5-carbonsäurederivatverbindung nach einem der Ansprüche 1 bis 9 zusammen mit einem pharmazeutisch akzeptablen Träger oder Verdünnungsmittel umfasst.

11. Verwendung nach Anspruch 9 zur Verwendung als Medikament.

12. Verwendung einer Dihydro-1H-indazol-5-carbonsäurederivatverbindung nach Anspruch 9 zur Herstellung eines Medikaments zur Behandlung eines Säugers zur Linderung der pathologischen Effekte von niedrigem HDL.

13. Verwendung einer Dihydro-1H-indazol-5-carbonsäurederivatverbindung nach Anspruch 9 zur Herstellung eines Medikaments zur Behandlung oder Verhinderung einer durch Leberlipase und/oder Endothellipase vermittelten Erkrankung.

14. Dihydro-1H-indazol-5-carbonsäurederivatverbindung der Formel (I) 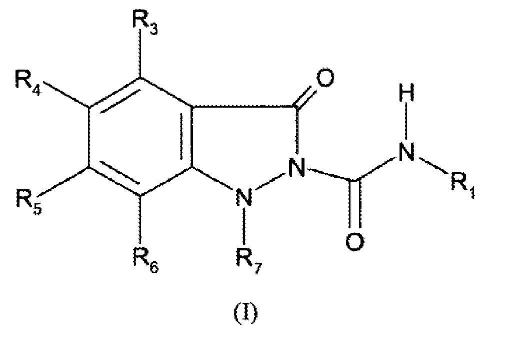 worin
R₁ aus C₅-C₁₃-Alkyl, C₁-C₁₂-Halogenalkyl, C₄-C₁₂-Alkenyl, C₄-C₁₂-Alkinyl, C₁-C₅-Alkyl-C₃-C₈-cycloalkyl, C₃-C₈-Cycloalkyl, C₁-C₅-Alkylheterocyclyl, Benzyl und Aryl ausgewählt ist, wobei der Cycloalkylsubstituent, heterocyclische Substituent, Benzylsubstituent und Arylsubstituent optional mit einem bis drei Substituenten substituiert sind, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ-COOC₁-C₅-Alkyl, -(CH₂)ₘ-NR^{a}R^{b} und C₁-C₄-Alkyl-C₃-C₈-cycloalkyl ausgewählt sind; worin R^{a} und R^{b} unabhängig voneinander aus Wasserstoff, C₁-C₈-Alkyl, C₂-C₈-Alkenyl, C₂-C₈-Alkinyl und C₁-C₅-Alkyl-C₃-C₈-cycloalkyl ausgewählt sind;
R₃, R₄, R₅ und R₆ unabhängig voneinander aus Wasserstoff, C₁-C₁₂-Alkyl, C₂-C₁₂-Halogenalkyl, C₂-C₁₂-Alkenyl, C₂-C₁₂-Alkinyl, C₂-C₁₂-Alkylaryl, C₁-C₁₂-Alkylcyclohexyl, C₁-C₁₂-Alkylcyclopentyl, C₁-C₁₂-Alkylheterocyclyl, -(CH₂)ₘ-COOH, -(CH₂)ₘ-CO(C₁-C₁₀)-Alkyl, -(CH₂)ₘ-COO(C₁-C₁₀)-Alkyl, -(CH₂)ₘ-COO(C₁-C₁₀)-Alkylaryl, C₁-C₁₀-Alkylamino, Halogen, -(CH₂)ₘ-CONR^{a}R^{b}, Phenyl, Benzyl oder Aryl ausgewählt sind, wobei jede der Phenyl-, Benzyl- oder Arylgruppen optional mit einer bis drei Gruppen substituiert ist, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ COOC₁-C₅-Alkyl und C₁-C₄-Alkyl-C₃-C₈-cydoalkyl ausgewählt sind; und wobei m 0, 1, 2 oder 3 bedeutet;
R₇ aus Wasserstoff, C₁-C₁₀-Alkyl, C₁-C₁₀-Halogenalkyl, C₂-C₁₀-Alkenyl, C₂-C₁₀-Alkinyl, C₁-C₆-Alkylaryl, C₁-C₆-Alkylcyclohexyl, C₁-C₆-Alkylcyclopentyl, C₁-C₆-Alkylheterocyclyl oder Aryl ausgewählt ist; oder ein pharmazeutisch akzeptables Salz oder Solvat hiervon zur Verwendung als Medikament.

15. Verwendung einer Dihydro-1H-indazol-5-carbonsäurederivatverbindung der Formel (I) 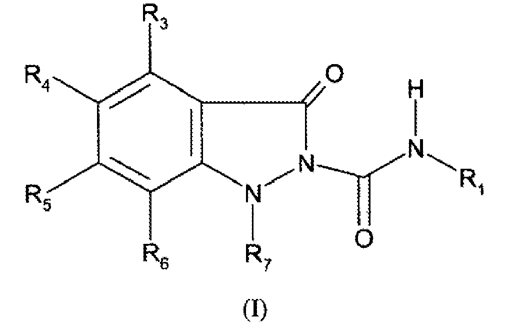 worin
R₁ aus C₅-C₁₃-Alkyl, C₁-C₁₂-Halogenalkyl, C₄-C₁₂-Alkenyl, C₄-C₁₂-Alkinyl, C₁-C₅-Alkyl-C₃-C₈-cycloalkyl, C₃-C₈-Cycloalkyl, C₁-C₅-Alkylheterocyclyl, Benzyl und Aryl ausgewählt ist, wobei der Cycloalkylsubstituent, heterocyclische Substituent, Benzylsubstituent und Arylsubstituent optional mit einem bis drei Substituenten substituiert sind, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ-COOC₁-C₅-Aikyi, -(CH₂)ₘ-NR^{a}R^{b} und C₁-C₄-Alkyl-C₃-C₈-cycloalkyl ausgewählt sind; worin R^{a} und R^{b} unabhängig voneinander aus Wasserstoff, C₁-C₈-Alkyl, C₂-C₈-Alkenyl, C₂-C₈-Alkinyl und C₁-C₅-Alkyl-C₃-C₈-cycloalkyl ausgewählt sind;
R₃, R₄, R₅ und R₆ unabhängig voneinander aus Wasserstoff, C₁-C₁₂-Alkyl, C₂-C₁₂-Halogenalkyl, C₂-C₁₂-Alkenyl, C₂-C₁₂-Alkinyl, C₂-C₁₂-Alkylaryl, C₁-C₁₂-Alkylcyclohexyl, C₁-C₁₂-Alkylcyclopentyl, C₁-C₁₂-Alkylheterocyclyl, -(CH₂)ₘ-COOH, -(CH₂)ₘ-CO(C₁-C₁₀)-Alkyl, -(CH₂)ₘ-COO(C₁-C₁₀)-Alkyl, -(CH₂)ₘ-COO(C₁-C₁₀)-Alkylaryl, C₁-C₁₀-Alkylamino, Halogen, -(CH₂)ₘ-CONR^{a}R^{b}, Phenyl, Benzyl oder Aryl ausgewählt sind, wobei jede der Phenyl-, Benzyl- oder Arylgruppen optional mit einer bis drei Gruppen substituiert ist, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ COOC₁-C₅-Alkyl und C₁-C₄-Alkyl-C₃-C₈-cycloalkyl ausgewählt sind; und wobei m 0, 1, 2 oder 3 bedeutet;
R₇ aus Wasserstoff, C₁-C₁₀-Alkyl, C₁-C₁₀-Halogenalkyl, C₂-C₁₀-Alkenyl, C₂-C₁₀-Alkinyl, C₁-C₆-Alkylaryl, C₁-C₆-Alkylcyclohexyl, C₁-C₆-Alkylcyclopentyl, C₁-C₆-Alkylheterocyclyl oder Aryl ausgewählt ist; oder eines pharmazeutisch akzeptablen Salzes oder Solvats hiervon zur Herstellung eines Medikaments zur Behandlung eines Säugers zur Linderung der pathologischen Effekte von niedrigem HDL.

16. Verwendung einer Dihydro-1H-indazol-5-carbonsäurederivatverbindung der Formel (I) 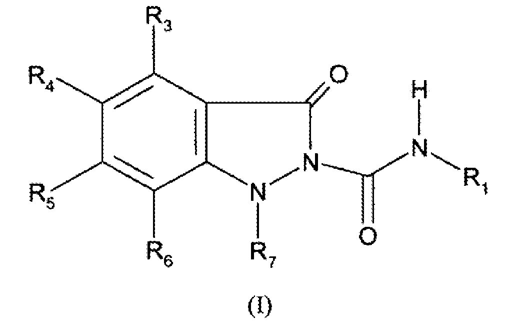 worin
R₁ aus C₅-C₁₃-Alkyl, C₁-C₁₂-Halogenalkyl, C₄-C₁₂-Alkenyl, C₄-C₁₂-Alkinyl, C₁-C₅-Alkyl-C₃-C₈-cycloalkyl, C₃-C₈-Cycloalkyl, C₁-C₅-Alkylheterocyclyl, Benzyl und Aryl ausgewählt ist, wobei der Cycloalkylsubstituent, heterocyclische Substituent, Benzylsubstituent und Arylsubstituent optional mit einem bis drei Substituenten substituiert sind, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ-COOC₁-C₅-Alkyl, -(CH₂)ₘ-NR^{a}R^{b} und C₁-C₄-Alkyl-C₃-C₈-cycloalkyl ausgewählt sind; worin R^{a} und R^{b} unabhängig voneinander aus Wasserstoff, C₁-C₈-Alkyl, C₂-C₈-Alkenyl, C₂-C₈-Alkinyl und C₁-C₅-Alkyl-C₃-C₈-cycloalkyl ausgewählt sind;
R₃, R₄, R₅ und R₆ unabhängig voneinander aus Wasserstoff, C₁-C₁₂-Alkyl, C₂-C₁₂-Halogenalkyl, C₂-C₁₂-Alkenyl, C₂-C₁₂-Alkinyl, C₂-C₁₂-Alkylaryl, C₁-C₁₂-Alkylcyclohexyl, C₁-C₁₂-Alkylcyclopentyl, C₁-C₁₂-Alkylheterocyclyl, -(CH₂)ₘ-COOH, -(CH₂)ₘ-CO(C₁-C₁₀)-Alkyl, -(CH₂)ₘ-COO(C₁-C₁₀)-Alkyl, -(CH₂)ₘ-COO(C₁-C₁₀)-Alkylaryl, C₁-C₁₀-Alkylamino, Halogen, -(CH₂)ₘ-CONR^{a}R^{b}, Phenyl, Benzyl oder Aryl ausgewählt sind, wobei jede der Phenyl-, Benzyl- oder Arylgruppen optional mit einer bis drei Gruppen substituiert ist, die unabhängig voneinander aus C₁-C₈-Alkyl, C₁-C₈-Halogenalkyl, C₂-C₈-Alkenyl, C₂-C₈-Alkinyl, Phenyl, Benzyl, Hydroxy, C₁-C₅-Alkoxy, -(CH₂)ₘ COOC₁-C₅-Alkyl und C₁-C₄-Alkyl-C₃-C₈-cycloalkyl ausgewählt sind; und wobei m 0, 1, 2 oder 3 bedeutet;
R₇ aus Wasserstoff, C₁-C₁₀-Alkyl, C₁-C₁₀-Halogenalkyl, C₂-C₁₀-Alkenyl, C₂-C₁₀-Alkinyl, C₁-C₆-Alkylaryl, C₁-C₆-Alkylcyclohexyl, C₁-C₆-Alkylcyclopentyl, C₁-C₆-Alkylheterocyclyl oder Aryl ausgewählt ist; oder eines pharmazeutisch akzeptablen Salzes oder Solvats hiervon zur Herstellung eines Medikaments zur Behandlung oder Verhinderung einer durch Leberlipase und/oder Endothellipase vermittelten Erkrankung.

## Revendications

1. Composé dérivé de l'acide dihydro-1*H*-indazole-5-carboxylique répondant à la formule (I) 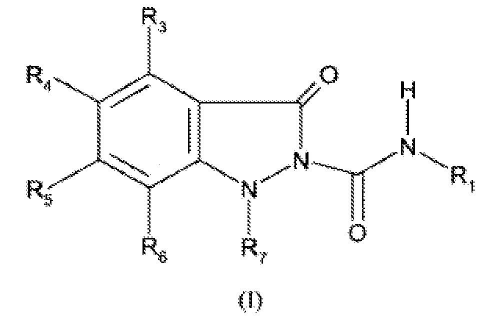 dans laquelle
R₁ est choisi parmi un groupe alkyle en C₅-C₁₃, un groupe halogénoalkyle en C₁-C₁₂, un groupe alcényle en C₄-C₁₂, un groupe alcynyle en C₄-C₁₂, un groupe alkyl(en C₁-C₅)cycloalkyle en C₃-C₈, un groupe cycloalkyle en C₃-C₈, un groupe alkyl(en C₁-C₅)hétérocyclique, un groupe benzyle et un groupe aryle, les substituants du groupe cycloalkyle, du groupe hétérocyclique, du groupe benzyle et du groupe aryle étant substitués de manière facultative avec de un à trois substituants choisis, de manière indépendante, parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, un groupe -(CH₂)ₘNRₐR_{b}, et un groupe alkyl(en C₁-C₄)cycloalkyle en C₃-C₈, R^{a} et R^{b} étant choisis, de manière indépendante, parmi un atome d'hydrogène, un groupe alkyle en C₁-C₈, un groupe alcényle en C₂-C₈, un groupe alcynyle en C₂-C₈, et un groupe alkyl(en C₁-C₅)cycloalkyle en C₃-C₈;
R₃, R₄, R₅, et R₆ sont choisis, de manière indépendante, parmi un atome d'hydrogène, un groupe alkyle en C₁-C₁₂, un groupe halogénoalkyle en C₂-C₁₂, un groupe alcényle en C₂-C₁₂, un groupe alcynyle en C₂-C₁₂, un groupe alkyl(en C₂-C₁₂)aryle, un groupe alkyl(en C₁-C₁₂)cyclohexyle, un groupe alkyl(en C₁-C₁₂)cyclopentyle, un groupe alkyl(en C₁-C₁₂)hétérocyclique, un groupe -(CH₂)ₘCOOH, un groupe -(CH₂)ₘCO-alkyle en C₁-C₁₀, un groupe -(CH₂)ₘCOO-alkyle en C₁-C₁₀, un groupe -(CH₂)ₘCOO-alkyl(en C₁-C₁₀)aryle, un groupe alkyl(en C₁-C₁₀)amino, un atome d'halogène, un groupe -(CH₂)ₘCONR^{a}R^{b}, un groupe phényle, un groupe benzyle ou un groupe aryle, chacun des groupes phényle, benzyle ou aryle étant substitué, de manière facultative, avec de un à trois groupes, choisis de manière indépendante parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, et un groupe alkyl(en C₁-C₄)cycloalkyle en C₃-C₈, et m étant égal à 0, 1, 2, ou 3;
R₇ est choisi parmi un atome d'hydrogène, un groupe alkyle en C₁-C₁₀, un groupe halogénoalkyle en C₁-C₁₀, un groupe alcényle en C₂-C₁₀, un groupe alcynyle en C₂-C₁₀, un groupe alkyl(en C₁-C₆)aryle, un groupe alkyl(en C₁-C₆)cyclohexyle, un groupe alkyl(en C₁-C₆)cyclopentyle, un groupe alkyl(en C₁-C₆)hétérocyclique ou un groupe aryle ;
ou un de ses sels ou de ses solvates pharmaceutiquement acceptables, avec cette réserve que le composé répondant à la formule (I) ne représente pas :
le phénylamide de l'acide 4,6-dichloro-3-oxo-1,3-dihydro-indazole-2-carboxylique ;
le 2-butylamide-4-octylamide de l'acide 3-oxo-1,3-dihydro-indazole-2,4-dicarboxylique ;
l'acide 2-octylcarbamoyl-3-oxo-2,3-dihydro-1H-indazole-4-carboxylique ;
le dodécylamide de l'acide 3-oxo-1,3-dihydro-indazole-2-carboxylique ;
l'octylamide de l'acide 3-oxo-1,3-dihydro-indazole-2-carboxylique ; ou
le phénylamide de l'acide 3-oxo-1-phényl-1,3-dihydro-indazole-2-carboxylique ; et avec la réserve supplémentaire que, lorsque R₃, R₄, R₅, R₆ et R₇ représentent chacun un atome d'hydrogène, R₁ ne représente pas un groupe phényle, un groupe alkyle contenant jusqu'à sept atomes de carbone ou un groupe alkylphényle dans lequel le groupe alkyle contient jusqu'à sept atomes de carbone.

2. Composé selon la revendication 1, dans lequel R₁ est choisi parmi un groupe alkyle en C₅-C₁₂, un groupe alcényle en C₄-C₁₂, un groupe cycloalkyle en C₃-C₇, un groupe -CF3, un groupe alkyl(en C₁-C₃)indole, un groupe alkyl(en C₁-C₃) benzothiophène et un groupe benzyle substitué ou non substitué.

3. Composé selon la revendication 2, dans lequel le groupe benzyle est disubstitué aux positions 2,4, 3,5 ou 2,5 ou bien est monosubstitué en position 2 ou 4.

4. Composé selon la revendication 3, dans lequel le substituant sur le groupe benzyle est choisi parmi un groupe méthyle, un groupe éthyle, un groupe propyle, un groupe isopropyle, un groupe isobutyle, un groupe tert-butyle et un groupe pentyle.

5. Composé selon la revendication 1, dans lequel R₃, R₄, R₅ et R₆ sont choisis, de manière indépendante, parmi un atome d'hydrogène, un groupe alkyle en C₁-C₄, un groupe alcényle en C₂-C₄, un atome d'halogène, un groupe halogénoalkyle en C₂-C₄, un groupe phényle et un groupe aryle.

6. Composé selon la revendication 1, dans lequel R₇ est choisi parmi un groupe méthyle, un groupe éthyle, un groupe propényle, un groupe éthynyle, un groupe propynyle, un groupe phényle, un groupe benzyle et un groupe halogénoalkyle en C₁-C₁₀.

7. Composé selon la revendication 1, choisi parmi :
le (2-éthyl-hexyl)-amide de l'acide 3-oxo-1,3-dihydro-indazole-2-carboxylique,
le (3,7-diméthyl-octa-2,6-diényl)-amide de l'acide 3-oxo-1,3-dihydro-indazole-2-carboxylique,
le cyclohexylméthyl-amide de l'acide 3-oxo-1,3-dihydro-indazole-2-carboxylique,
le pentylamide de l'acide 1-méthyl-3-oxo-1,3-dihydro-indazole-2-carboxylique,
le pentylamide de l'acide 1-butyl-3-oxo-1,3-dihydro-indazole-2-carboxylique,
le pentylamide de l'acide 1-benzyl-3-oxo-1,3-dihydro-indazole-2-carboxylique,
le pentylamide de l'acide 3-oxo-1-phénéthyl-1,3-dihydro-indazole-2-carboxylique,
le 2-méthyl-benzylamide de l'acide 3-oxo-1,3-dihydro-indazole-2-carboxylique,
le 3-méthyl-benzylamide de l'acide 3-oxo-1,3-dihydro-indazole-2-carboxylique,
le 4-méthyl-benzylamide de l'acide 3-oxo-1,3-dihydro-indazole-2-carboxylique,
le 2-trifluorométhyl-benzylamide de l'acide 3-oxo-1,3-dihydro-indazole-2-carboxylique,
le 2-éthyl-benzylamide de l'acide 3-oxo-1,3-dihydro-indazole-2-carboxylique,
le 3-éthyl-benzylamide de l'acide 3-oxo-1,3-dihydro-indazole-2-carboxylique,
le 3-méthoxy-benzylamide de l'acide 3-oxo-1,3-dihydro-indazole-2-carboxylique,
le 4-amino-benzylamide de l'acide 3-oxo-1,3-dihydro-indazole-2-carboxylique,
le 4-diméthylamino-benzylamide de l'acide 3-oxo-1,3-dihydro-indazole-2-carboxylique
le (biphényl-3-ylméthyl)-amide de l'acide 3-oxo-1,3-dihydro-indazole-2-carboxylique,
le 2-isopropyl-6-méthyl-benzylamide de l'acide 3-oxo-1,3-dihydro-indazole-2-carboxylique,
le 2,4-diméthoxy-benzylamide de l'acide 3-oxo-1,3-dihydro-indazole-2-carboxylique,
le 3,4-dihydroxy-benzylamide de l'acide 3-oxo-1,3-dihydro-indazole-2-carboxylique,
le (2,3-dihydro-benzo[1,4]dioxin-6-ylméthyl)amide de l'acide 3-oxo-1,3-dihydro-indazole-2-carboxylique,
le 2,4,6-triméthyl-benzylamide de l'acide 3-oxo-1,3-dihydro-indazole-2-carboxylique,
le 3,4,5-triméthoxy-benzylamide de l'acide 3-oxo-1,3-dihydro-indazole-2-carboxylique,
le 2-méthyl-benzylamide de l'acide 4-méthyl-3-oxo-1,3-dihydro-indazole-2-carboxylique,
le 2-méthyl-benzylamide de l'acide 6-méthyl-3-oxo-1,3-dihydro-indazole-2-carboxylique,
le 2-méthyl-benzylamide de l'acide 4-chloro-3-oxo-1,3-dihydro-indazole-2-carboxylique,
le 2-méthyl-benzylamide de l'acide 5-chloro-3-oxo-1,3-dihydro-indazole-2-carboxylique,
le 2-méthyl-benzylamide de l'acide 6-chloro-3-oxo-1,3-dihydro-indazole-2-carboxylique,
l'acide 2-(2-méthyl-benzylcarbamoyl)-3-oxo-2,3-dihydro-1H-indazole-4-carboxylique,
l'acide 2-(2-méthyl-benzylcarbamoyl)-3-oxo-2,3-dihydro-1H-indazole-5-carboxylique,
l'acide 2-(2-méthyl-benzylcarbamoyl)-3-oxo-2,3-dihydro-1H-indazole-6-carboxylique,
l'acide 2-(2-méthyl-benzylcarbamoyl)-3-oxo-2,3-dihydro-1H-indazole-7-carboxylique,
l'acide 3-oxo-2-(2-trifluorométhyl-benzylcarbamoyl)-2,3-dihydro-1H-indazole-7-carboxylique,
l'acide 2-(2,6-diméthyl-benzylcarbamoyl)-3-oxo-2,3-dihydro-1H-indazole-4-carboxylique,
l'acide 2-(2,6-diméthyl-benzylcarbamoyl)-3-oxo-2,3-dihydro-1H-indazole-7-carboxylique,
l'acide 2-(2-éthyl-6-méthyl-benzylcarbamoyl)-3-oxo-2,3-dihydro-1H-indazole-4-carboxylique,
l'acide 2-(2-éthyl-6-méthyl-benzylcarbamoyl)-3-oxo-2,3-dihydro-1H-indazole-5-carboxylique,
l'acide 2-(2-éthyl-6-méthyl-benzylcarbamoyl)-3-oxo-2,3-dihydro-1H-indazole-6-carboxylique,
l'acide 2-(2-éthyl-6-méthyl-benzylcarbamoyl)-3-oxo-2,3-dihydro-1H-indazole-7-carboxylique,
l'acide 2-(2-isopropyl-6-méthyl-benzylcarbamoyl)-3-oxo-2,3-dihydro-1H-indazole-4-carboxylique,
l'acide 2-(2-isopropyl-6-méthyl-benzylcarbamoyl)-3-oxo-2,3-dihydro-1H-indazole-7-carboxylique,
l'acide 2-(2-tert-butyl-6-méthyl-benzylcarbamoyl)-3-oxo-2,3-dihydro-1H-indazole-7-carboxylique,
l'acide 2-(4-butyl-2-méthyl-benzylcarbamoyl)-3-oxo-2,3-dihydro-1H-indazole-4-carboxylique,
l'acide 2-(2,6-diéthyl-benzylcarbamoyl)-3-oxo-2,3-dihydro-1H-indazole-4-carboxylique,
l'acide 2-(2,6-diéthyl-benzylcarbamoyl)-3-oxo-2,3-dihydro-1H-indazole-7-carboxylique,
l'acide 2-(2,6-diisopropyl-benzylcarbamoyl)-3-oxo-2,3-dihydro-1H-indazole-4-carboxylique,
l'acide 2-(2,6-diisopropyl-benzylcarbamoyl)-3-oxo-2,3-dihydro-1H-indazole-7-carboxylique,
l'acide 2-(2,4,6-triméthyl-benzylcarbamoyl)-3-oxo-2,3-dihydro-1H-indazole-4-carboxylique,
l'acide 3-oxo-2-(4-phényl-butylcarbamoyl)-2,3-dihydro-1H-indazole-4-carboxylique,
l'acide 2-(4-cyclohexyl-butylcarbamoyl)-3-oxo-2,3-dihydro-1H-indazole-4-carboxylique,
l'acide 2-hexylcarbamoyl-3-oxo-2,3-dihydro-1H-indazole-4-carboxylique,
l'acide 2-(2-éthyl-6-méthyl-benzylcarbamoyl)-1-méthyl-3-oxo-2,3-dihydro-1H-indazole-7-carboxylique,
l'acide 1-éthyl-2-(2-éthyl-6-méthyl-benzylcarbamoyl)-3-oxo-2,3-dihydro-1H-indazole-7-carboxylique,
l'acide 2-(2-éthyl-6-méthyl-benzylcarbamoyl)-3-oxo-1-propyl-2,3-dihydro-1H-indazole-7-carboxylique,
l'acide 2-(2-isopropyl-6-méthyl-benzylcarbamoyl)-1-méthyl-3-oxo-2,3-dihydro-1H-indazole-7-carboxylique,
l'acide 1-éthyl-2-(2-isopropyl-6-méthyl-benzylcarbamoyl)-3-oxo-2,3-dihydro-1H-indazole-7-carboxylique,
l'acide 2-(2-isopropyl-6-méthyl-benzylcarbamoyl)-3-oxo-1-propyl-2,3-dihydro-1 H-indazole-7-carboxylique,
l'acide 1-(3-chloro-propyl)-2-(2-isopropyl-6-méthyl-benzylcarbamoyl)-3-oxo-2,3-dihydro-1H-indazole-7-carboxylique,
l'acide 2-(2-isopropyl-6-méthyl-benzylcarbamoyl)-3-oxo-1-(prop-2-ynyl)-2,3-dihydro-1H-indazole-7-carboxylique,
l'acide 1-allyl-2-(2-isopropyl-6-méthyl-benzylcarbamoyl)-3-oxo-2,3-dihydro-1H-indazole-7-carboxylique,
l'acide 1-butyl-2-(2-isopropyl-6-méthyl-benzylcarbamoyl)-3-oxo-2,3-dihydro-1H-indazole-7-carboxylique,
l'acide 1-benzyl-2-(2-isopropyl-6-méthyl-benzylcarbamoyl)-3-oxo-2,3-dihydro-1 H-indazole-7-carboxylique,
l'acide 2-(2-tert-butyl-6-méthyl-benzylcarbamoyl)-1-méthyl-3-oxo-2,3-dihydro-1 H-indazole-7-carboxylique,
l'acide 2-(2,6-diéthyl-benzylcarbamoyl)-1-méthyl-3-oxo-2,3-dihydro-1H-indazole-7-carboxylique,
l'ester méthylique de l'acide 2-hexylcarbamoyl-3-oxo-2,3-dihydro-1H-indazole-4-carboxylique,
l'ester méthylique de l'acide 2-hexylcarbamoyl-3-oxo-2,3-dihydro-1H-indazole-7-carboxylique,
l'ester méthylique de l'acide 2-(2-isopropyl-6-méthyl-benzylcarbamoyl)-3-oxo-2,3-dihydro-1H-indazole-4-carboxylique, et
l'ester méthylique de l'acide 2-(2-isopropyl-6-méthyl-benzylcarbamoyl)-3-oxo-2,3-dihydro-1H-indazole-7-carboxylique.

8. Composé selon la revendication 1, dans lequel le composé répond aux formules (C1), (C2), (C3), (C4), ou (C5) :

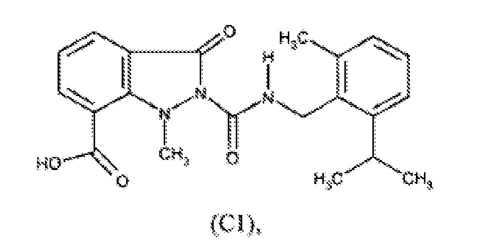

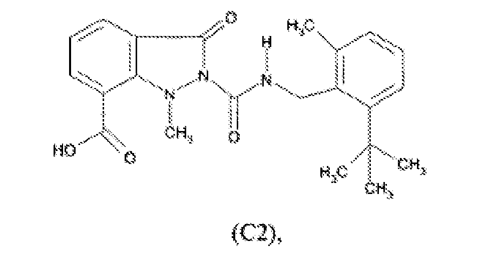

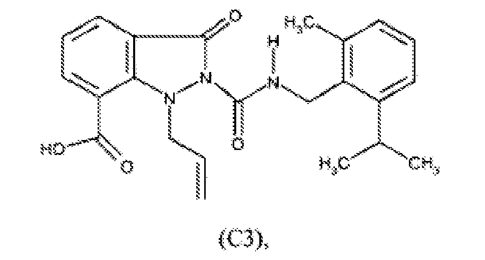

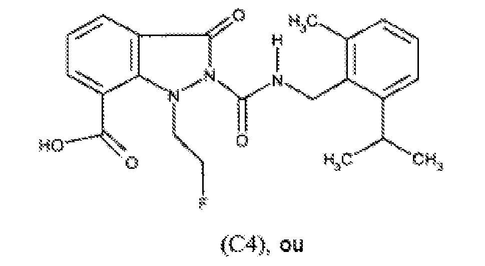

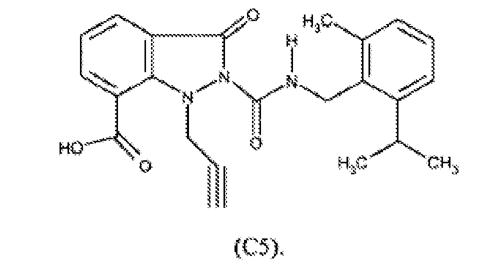

9. Composé répondant à la formule (I) 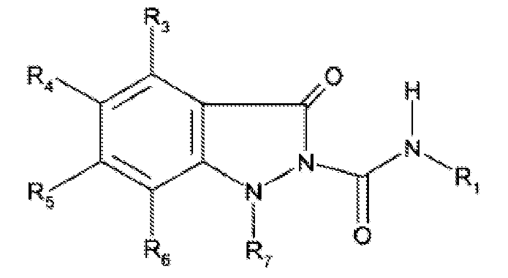 dans laquelle R₁ et R₃ à R₇ sont choisis pour produire un composé choisi parmi :
l'hexylamide de l'acide 1-(2-méthoxy-éthyl)-3-oxo-1,3-dihydro-indazole-2-carboxylique,
l'ester méthylique de l'acide (2-hexylcarbamoyl-3-oxo-2,3-dihydro-indazol-1-yl)-acétique,
le 3-fluoro-benzylamide de l'acide 3-oxo-1,3-dihydro-indazole-2-carboxylique,
le 3-chloro-benzylamide de l'acide 3-oxo-1,3-dihydro-indazole-2-carboxylique,
le 3-trifluorométhoxy-benzylamide de l'acide 3-oxo-1,3-dihydro-indazole-2-carboxylique,
le 2-méthylsulfanyl-benzylamide de l'acide 3-oxo-1,3-dihydro-indazole-2-carboxylique,
le (naphtalén-1-ylméthyl)-amide de l'acide 3-oxo-1,3-dihydro-indazole-2-carboxylique,
le (4-méthyl-naphtalén-1-ylméthyl)-amide de l'acide 3-oxo-1,3-dihydro-indazole-2-carboxylique,
le 5-chloro-2-méthyl-benzylamide de l'acide 3-oxo-1,3-dihydro-indazole-2-carboxylique,
le 2,4-dichloro-benzylamide de l'acide 3-oxo-1,3-dihydro-indazole-2-carboxylique,
le 5-chloro-2-méthoxy-benzylamide de l'acide 3-oxo-1,3-dihydro-indazole-2-carboxylique,
le 4,6-dichloro-2-méthyl-benzylamide de l'acide 3-oxo-1,3-dihydro-indazole-2-carboxylique,
le (4-phényl-but-3-ényl)-amide de l'acide 3-oxo-1,3-dihydro-indazole-2-carboxylique,
le 2-méthyl-benzylamide de l'acide 7-méthoxy-3-oxo-1,3-dihydro-indazole-2-carboxylique,
le 2-méthyl-benzylamide de l'acide 5-hydroxy-3-oxo-1,3-dihydro-indazole-2-carboxylique,
le 2-méthyl-benzylamide de l'acide 5-nitro-3-oxo-1,3-dihydro-indazole-2-carboxylique,
l'acide 2-(5-fluoro-2-méthyl-benzylcarbamoyl)-3-oxo-2,3-dihydro-1H-indazole-4-carboxylique,
l'acide 2-(5-fluoro-2-méthyl-benzylcarbamoyl)-3-oxo-2,3-dihydro-1H-indazole-7-carboxylique,
l'acide 2-[(4-méthyl-naphtalén-1-ylméthyl)-carbamoyl]-3-oxo-2,3-dihydro-1H-indazole-4carboxylique,
l'acide 3-oxo-2-(3-phénoxy-propylcarbamoyl)-2,3-dihydro-1H-indazole-4-carboxylique,
l'acide 1-carboxyméthyl-2-(2-éthyl-6-méthyl-benzylcarbamoyl)-3-oxo-2,3-dihydro-1H-indazole-7-carboxylique,
l'acide 2-(2-éthyl-6-méthyl-benzylcarbamoyl)-1-méthoxycarbonylméthyl-3-oxo-2,3-dihydro-1H-indazole-7-carboxylique,
l'acide 1-cyanométhyl-2-(2-isopropyl-6-méthyl-benzylcarbamoyl)-3-oxo-2,3-dihydro-1H-indazole-7-carboxylique,
l'acide 1-cyclopropylméthyl-2-(2-isopropyl-6-méthyl-benzylcarbamoyl)-3-oxo-2,3-dihydro-1H-indazole-7-carboxylique,
l'acide 1-carboxyméthyl-2-(2-isopropyl-6-méthyl-benzylcarbamoyl)-3-oxo-2,3-dihydro-1H-indazole-7-carboxylique,
l'acide 2-(2-isopropyl-6-méthyl-benzylcarbamoyl)-1-méthoxycarbonylméthyl-3-oxo-2,3-dihydridro-1H-indazole-7-carboxylique,
l'acide 1-carbamoylméthyl-2-(2-isopropyl-6-méthyl-benzylcarbamoyl)-3-oxo-2,3-dihydro-1H-indazole-7-carboxylique,
l'ester allylique de l'acide 2-hexylcarbamoyl-3-oxo-2,3-dihydro-1H-indazole-4-carboxylique, et
l'ester allylique de l'acide 2-hexylcarbamoyl-3-oxo-2,3-dihydro-1H-indazole-7-carboxylique.

10. Formulation pharmaceutique comprenant un composé dérivé de l'acide dihydro-1H-indazole-5-carboxylique selon l'une quelconque des revendications 1 à 9 de manière conjointe avec un support ou un diluant pharmaceutiquement acceptable.

11. Composé selon la revendication 9, à utiliser comme médicament.

12. Utilisation d'un composé dérivé de l'acide dihydro-1H-indazole-5-carboxylique selon la revendication 9 pour la préparation d'un médicament destiné au traitement d'un mammifère dans le but de soulager les effets pathologiques d'un faible taux de HDL.

13. Utilisation d'un composé dérivé de l'acide dihydro-1H-indazole-5-carboxylique selon la revendication 9 pour la préparation d'un médicament destiné au traitement ou à la prévention de la lipase hépatique et/ou d'une maladie dans laquelle la lipase endothéliale intervient comme médiateur.

14. Composé dérivé de l'acide dihydro-1H-indazole-5-carboxylique répondant à la formule (I) 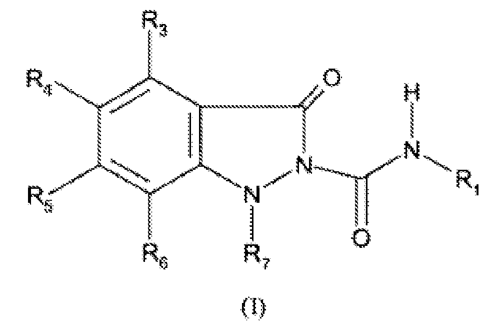 dans laquelle
R₁ est choisi parmi un groupe alkyle en C₅-C₁₃, un groupe halogénoalkyle en C₁-C₁₂, un groupe alcényle en C₄-C₁₂, un groupe alcynyle en C₄-C₁₂, un groupe alkyl(en C₁-C₅)cycloalkyle en C₃-C₈, un groupe cycloalkyle en C₃-C₈, un groupe alkyl(en C₁-C₅)hétérocyclique, un groupe benzyle et un groupe aryle, les substituants du groupe cycloalkyle, du groupe hétérocyclique, du groupe benzyle et du groupe aryle étant substitués de manière facultative avec de un à trois substituants choisis, de manière indépendante, parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, un groupe -(CH₂)ₘNRₐR_{b}, et un groupe alkyl(en C₁-C₄)cycloalkyle en C₃-C₈, R^{a} et R^{b} étant choisis, de manière indépendante, parmi un atome d'hydrogène, un groupe alkyle en C₁-C₈, un groupe alcényle en C₂-C₈, un groupe alcynyle en C₂-C₈, et un groupe alkyl(en C₁-C₅)cycloalkyle en C₃-C₈;
R₃, R₄, R₅ et R₆ sont choisis, de manière indépendante, parmi un atome d'hydrogène, un groupe alkyle en C₁-C₁₂, un groupe halogénoalkyle en C₂-C₁₂, un groupe alcényle en C₂-C₁₂, un groupe alcynyle en C₂-C₁₂, un groupe alkyl(en C₂-C₁₂)aryle, un groupe alkyl(en C₁-C₁₂)cyclohexyle, un groupe alkyl(en C₁-C₁₂)cyclopentyle, un groupe alkyl(en C₁-C₁₂)hétérocyclique, un groupe -(CH₂)ₘCOOH, un groupe -(CH₂)ₘCO-alkyle en C₁-C₁₀, un groupe -(CH₂)ₘCOO-alkyle en C₁-C₁₀, un groupe -(CH₂)ₘCOO-alkyl(en C₁-C₁₀)aryle, un groupe alkyl(en C₁-C₁₀)amino, un atome d'halogène, un groupe -(CH₂)ₘCONR^{a}R^{b}, un groupe phényle, un groupe benzyle ou un groupe aryle, chacun des groupes phényle, benzyle ou aryle étant substitué, de manière facultative, avec de un à trois groupes, choisis de manière indépendante parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, et un groupe alkyl(en C₁-C₄)cycloalkyle en C₃-C₈, et m étant égal à 0, 1, 2, ou 3;
R₇ est choisi parmi un atome d'hydrogène, un groupe alkyle en C₁-C₁₀, un groupe halogénoalkyle en C₁-C₁₀, un groupe alcényle en C₂-C₁₀, un groupe alcynyle en C₂-C₁₀, un groupe alkyl(en C₁-C₆)aryle, un groupe alkyl(en C₁-C₆)cyclohexyle, un groupe alkyl(en C₁-C₆)cyclopentyle, un groupe alkyl(en C₁-C₆)hétérocyclique ou un groupe aryle ;
ou un de ses sels ou de ses solvates pharmaceutiquement acceptables, à utiliser comme médicament.

15. Utilisation d'un composé dérivé de l'acide dihydro-1H-indazole-5-carboxylique répondant à la formule (I) 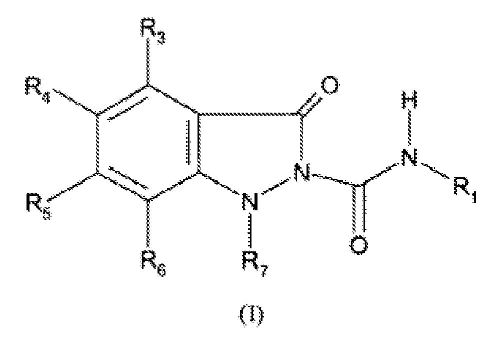 dans laquelle
R₁ est choisi parmi un groupe alkyle en C₅-C₁₃, un groupe halogénoalkyle en C₁-C₁₂, un groupe alcényle en C₄-C₁₂, un groupe alcynyle en C₄-C₁₂, un groupe alkyl(en C₁-C₅)cycloalkyle en C₃-C₈, un groupe cycloalkyle en C₃-C₈, un groupe alkyl(en C₁-C₅)hétérocyclique, un groupe benzyle et un groupe aryle, les substituants du groupe cycloalkyle, du groupe hétérocyclique, du groupe benzyle et du groupe aryle étant substitués de manière facultative avec de un à trois substituants choisis, de manière indépendante, parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, un groupe -(CH₂)ₘNRₐR_{b}, et un groupe alkyl(en C₁-C₄)cycloalkyle en C₃-C₈, R^{a} et R^{b} étant choisis, de manière indépendante, parmi un atome d'hydrogène, un groupe alkyle en C₁-C₈, un groupe alcényle en C₂-C₈, un groupe alcynyle en C₂-C₈, et un groupe alkyl(en C₁-C₅)cycloalkyle en C₃-C₈;
R₃, R₄, R₅, et R₆ sont choisis, de manière indépendante, parmi un atome d'hydrogène, un groupe alkyle en C₁-C₁₂, un groupe halogénoalkyle en C₂-C₁₂, un groupe alcényle en C₂-C₁₂, un groupe alcynyle en C₂-C₁₂, un groupe alkyl(en C₂-C₁₂)aryle, un groupe alkyl(en C₁-C₁₂)cyclohexyle, un groupe alkyl(en C₁-C₁₂)cyclopentyle, un groupe alkyl(en C₁-C₁₂)hétérocyclique, un groupe -(CH₂)ₘCOOH, un groupe -(CH₂)ₘCO-alkyle en C₁-C₁₀, un groupe -(CH₂)ₘCOO-alkyle en C₁-C₁₀, un groupe -(CH₂)ₘCOO-alkyl(en C₁-C₁₀)aryle, un groupe alkyl(en C₁-C₁₀)amino, un atome d'halogène, un groupe -(CH₂)ₘCONR^{a}R^{b}, un groupe phényle, un groupe benzyle ou un groupe aryle, chacun des groupes phényle, benzyle ou aryle étant substitué, de manière facultative, avec de un à trois groupes, choisis de manière indépendante parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, et un groupe alkyl(en C₁-C₄)cycloalkyle en C₃-C₈, et m étant égal à 0, 1, 2, ou 3;
R₇ est choisi parmi un atome d'hydrogène, un groupe alkyle en C₁-C₁₀, un groupe halogénoalkyle en C₁-C₁₀, un groupe alcényle en C₂-C₁₀, un groupe alcynyle en C₂-C₁₀, un groupe alkyl(en C₁-C₆)aryle, un groupe alkyl(en C₁-C₆)cyclohexyle, un groupe alkyl(en C₁-C₆)cydopentyle, un groupe alkyl(en C₁-C₆)hétérocyclique ou un groupe aryle ;
ou d'un de ses sels ou de ses solvates pharmaceutiquement acceptables, pour la préparation d'un médicament destiné au traitement d'un mammifère dans le but de soulager les effets pathologiques d'un faible taux de HDL.

16. Utilisation d'un composé dérivé de l'acide dihydro-1H-indazole-5-carboxylique répondant à la formule (I) 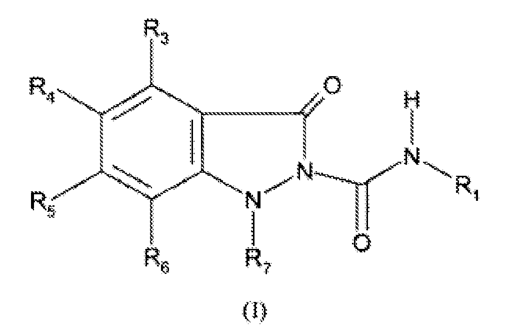 dans laquelle
R₁ est choisi parmi un groupe alkyle en C₅-C₁₃, un groupe halogénoalkyle en C₁-C₁₂, un groupe alcényle en C₄-C₁₂, un groupe alcynyle en C₄-C₁₂, un groupe alkyl(en C₁-C₅)cycloalkyle en C₃-C₈, un groupe cycloalkyle en C₃-C₈, un groupe alkyl(en C₁-C₅)hétérocyclique, un groupe benzyle et un groupe aryle, les substituants du groupe cycloalkyle, du groupe hétérocyclique, du groupe benzyle et du groupe aryle étant substitués de manière facultative avec de un à trois substituants choisis, de manière indépendante, parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, un groupe -(CH₂)ₘNRₐR_{b}, et un groupe alkyl(en C₁-C₄)cycloalkyle en C₃-C₈, R^{a} et R^{b} étant choisis, de manière indépendante, parmi un atome d'hydrogène, un groupe alkyle en C₁-C₈, un groupe alcényle en C₂-C₈, un groupe alcynyle en C₂-C₈, et un groupe alkyl(en C₁-C₅)cycloalkyle en C₃-C₈;
R₃, R₄, R₅, et R₆ sont choisis, de manière indépendante, parmi un atome d'hydrogène, un groupe alkyle en C₁-C₁₂, un groupe halogénoalkyle en C₂-C₁₂, un groupe alcényle en C₂-C₁₂, un groupe alcynyle en C₂-C₁₂, un groupe alkyl(en C₂-C₁₂)aryle, un groupe alkyl(en C₁-C₁₂)cydohexyle, un groupe alkyl(en C₁-C₁₂)cyclopentyle, un groupe alkyl(en C₁-C₁₂)hétérocyclique, un groupe -(CH₂)ₘCOOH, un groupe -(CH₂)ₘCO-alkyle en C₁-C₁₀, un groupe -(CH₂)ₘCOO-alkyle en C₁-C₁₀, un groupe -(CH₂)ₘCOO-alkyl(en C₁-C₁₀)aryle, un groupe alkyl(en C₁-C₁₀)amino, un atome d'halogène, un groupe -(CH₂)ₘCONR^{a}R^{b}, un groupe phényle, un groupe benzyle ou un groupe aryle, chacun des groupes phényle, benzyle ou aryle étant substitué, de manière facultative, avec de un à trois groupes, choisis de manière indépendante parmi un groupe alkyle en C₁-C₈, un groupe halogénoalkyle en C₁-C₈, un groupe alcényle en C₂-C₈, un groupe alcynyle en C₂-C₈, un groupe phényle, un groupe benzyle, un groupe hydroxyle, un groupe alcoxy en C₁-C₅, un groupe -(CH₂)ₘCOOalkyle en C₁-C₅, et un groupe alkyl(en C₁-C₄)cycloalkyle en C₃-C₈, et m étant égal à 0, 1, 2, ou 3;
R₇ est choisi parmi un atome d'hydrogène, un groupe alkyle en C₁-C₁₀, un groupe halogénoalkyle en C₁-C₁₀, un groupe alcényle en C₂-C₁₀, un groupe alcynyle en C₂-C₁₀, un groupe alkyl(en C₁-C₆)aryle, un groupe alkyl(en C₁-C₆)cyclohexyle, un groupe alkyl(en C₁-C₆)cyclopentyle, un groupe alkyl(en C₁-C₆)hétérocyclique, ou un groupe aryle ;
ou d'un de ses sels ou de ses solvates pharmaceutiquement acceptables, pour la préparation d'un médicament destiné au traitement ou à la prévention de la lipase hépatique et/ou d'une maladie dans laquelle la lipase endothéliale intervient comme médiateur.
